(19) Europäisches Patentamt
European Patent Office
Office européen des brevets

(11) EP 4 361 137 A1

(12) EUROPEAN PATENT APPLICATION

(43) Date of publication:
**01.05.2024 Bulletin 2024/18**

(21) Application number: **22204452.1**

(22) Date of filing: **28.10.2022**

(51) International Patent Classification (IPC):
***C07D 401/04*** *(2006.01)* ***A61P 35/00*** *(2006.01)*
***A61K 31/53*** *(2006.01)*

(52) Cooperative Patent Classification (CPC):
**C07D 401/04; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **CEINGE Biotecnologie Avanzate Franco Salvatore s.c. a r.l.**
**80145 Napoli (IT)**

(72) Inventors:
- **ZOLLO, Massimo**
  **80131 Napoli (IT)**
- **ASADZADEH, Fatemeh**
  **80125 Napoli (IT)**
- **FERRUCCI, Veronica**
  **80134 Napoli (IT)**
- **DE ANTONELLIS, Pasqualino**
  **83100 Avellino (IT)**
- **BIBBO', Francesca**
  **82023 CCastelvetere in Val Fortore (BN) (IT)**
- **SORICE, Carmen**
  **83020 Sperone (AV) (IT)**

(74) Representative: **Bianchetti & Minoja with Trevisan & Cuonzo IPS SRL**
**Via Plinio, 63**
**20129 Milano (IT)**

Remarks:
The complete document including Reference Table(s) and the Sequence Listing(s) can be downloaded from the EPO website

(54) **PRUNE1 INHIBITORS AND THERAPEUTIC USE THEREOF**

(57) There are disclosed compounds able to modulate and specifically inhibit the activity of Prune1 protein, pharmaceutical compositions containing them and the use thereof alone or in combination with chemotherapeutic agents in the prevention or treatment of Prune 1-overexpressing tumors and metastasis.

EP 4 361 137 A1

## Description

The present invention provides compounds able to modulate and specifically inhibit the activity of Prune1 protein, pharmaceutical compositions containing them and the use thereof alone or in combination with chemotherapeutic agents in the prevention or treatment of Prune1-overexpressing tumors and metastasis.

## Background of the invention

*Prune1 protein in tumorigenesis*

The human Prune1 protein belongs to the DHH (Asp-His-His) protein superfamily and possesses a nucleotide phosphodiesterase (PDE) (D'angelo et al., 2004) and an exopolyphosphatase (exopolyphosphatase/pyrophosphatase, PPX/PPase) action, with greater affinity for short-chain over long-chain inorganic polyphosphates (polyPs) (Tammenkoski et al., 2008). Regarding its tertiary structure, human Prune1 is a naturally unfolded protein with the ability to interact with several intracellular binding partners, including GSK-3β (Carotenuto et al., 2014) and Nm23-H1/H2 (Diana et al., 2013). Furthermore, Prune1 was also identified as a microtubule-associated protein (MAP) with a role in the enhancement of microtubule (MT) polymerization in the mitotic spindle during cell division (Zollo et al., 2017).

Because of the enzymatic activities and its interaction with several proteins, Prune1 was found able to modulate both intra- and extracellular signaling cascades [including the canonical Wnt (Carotenuto et al., 2014) and TGF-β (Ferrucci et al., 2018) pathways] that regulate cell proliferation, motility, and epithelial-mesenchymal transition (EMT) processes.

Prune1 has been found highly expressed and positively correlated with the grading, EMT, and metastatic status in several metastatic solid tumors: MB groups 3 and 4 (Ferrucci et al., 2018), gastric cancer (GC) (Owe et al., 2007), esophageal squamous cell carcinoma (ESCC) (Noguchi et al., 2009), NSCLC (Carotenuto et al., 2014), thyroid cancer (TC) (Nambu et al., 2016), colorectal cancer (CRC) (Hashimoto et al., 2016), NBL (Carotenuto et al., 2013), BC, and metastatic triple-negative breast cancer (TNBC) (D'angelo et al., 2004; Zollo et al., 2005, Ferrucci et al., 2021). Prune1 overexpression occurs via the amplification and/or gain of chromosome 1q (as identified in tumors of epithelial origin), where PRUNE1 is located (i.e., 1q21.3) (D'angelo et al., 2004). For instance, MB group 3 (γ-subtype), in which Prune1 was found overexpressed, has a trend for gain of chromosome 1q (Cavalli et al., 2017). Moreover, the gain of chromosomal region 1q21 was predominantly reported in BC belonging to the "basal-like" and TNBC subgroups (30%-40%) (Silva et al., 2015) and also in those recurrent (70%) (Goh et al., 2017), in which the levels of Prune1 were found higher (Galasso et al., 2009).

*Prune1 protein in Medulloblastoma*

MB is an embryonal tumor occurring in the cerebellum and represents ~20% of all primary childhood CNS tumors. Recent integrative genomics, messenger RNA (mRNA) expression, and methylation profiling have allowed MB to be stratified into different molecular subgroups: Wingless (WNT), Sonic Hedgehog (SHH), group 3, and group 4 (Cavalli et al., 2017). Heterogeneity within these subgroups has been recognized, and it has been suggested that MB may consist of up 12 subtypes [i.e., WNT (α and β), SHH (α, β, γ, and δ), group 3 (α, β, and γ), and group 4 (α, β, and γ)] (Cavalli et al., 2017). Distinctive transcriptional, mutational, and epigenetic profiles were reported for each MB subtype, with different clinical features (Cavalli et al., 2017). The MB SHH subgroup is characterized by an aberrant activation of the SHH pathway and arises from cerebellar granule neuron progenitors (GNPs) mainly mutated in PTCH1 or SMO receptors, although a non-canonical SHH/GLI activation has also been frequently observed (Cavalli et al., 2017). Although ~60% of MB belongs to groups 3 and 4, future studies are needed to clarify the developmental origins and the biological mechanisms of these latest subgroups (Cavalli et al., 2017). MB group 3 is considered the most aggressive subgroup because of the high metastatic potential and the poor survival rate. Tailored therapies for metastatic MB groups 3 and 4 are currently lacking, and the combination of surgery, craniospinal radiotherapy (except in young children for whom radiotherapy has devastating neurocognitive side effects), and multi-agent chemotherapy is used (Gajjar et al., 2014).

Recently, high expressions of Prune 1 were found in these metastatic MB subgroups (i.e., MB groups 3 and 4), and a new metastatic axis (independent of c-MYC amplification) was dissected in MB group 3 with the poorest prognosis. In this regard, Prune1 protein, due to the binding to Nm23-H1, enhances the canonical TGF-β pathway, thus leading to OTX2 and SNAIL upregulation, PTEN reduction, and EMT activation (Ferrucci et al., 2018). Furthermore, gene expression and gene ontology analyses allowed other genes (i.e., OTX2, CYFIP1, and GLI2) involved in neurogenesis to be correlated to Prune1 (Ferrucci et al., 2018). Interestingly, a cell competitive permeable peptide (cell-penetrating peptide, CPP) that impairs Prunn1/Nn23-H1 complex formation was found to impair both tumorigenesis and metastatic spread in orthotopic models implanted with metastatic MB group 3 cells overexpressing c-MYC and mutated for TP53 (i.e., D425-Med cells), thus representing the most aggressive type of MB with the poorest prognosis (Hill et al., 2015). Furthermore, an anti-Prune1 molecule (pyrimido-pyrimidine derivative, i.e., AA7.1) (Virgilio et al., 2012) was

also found with the ability to bind Prune1 protein, to enhance its intracellular degradation and to increase the PTEN protein level, thus inhibiting the Prune1-mediated metastatic network both in vitro in primary human medullospheres obtained from patients and also in vivo in orthotopic xenograft models of metastatic MB group 3 (Ferrucci et al., 2018).

*Prune1 protein in Breast Cancer and Triple-Negative Breast Cancer (TNBC)*

High expression levels of Prune1 were found associated with metastasis in regional lymph nodes (p = 0.017) and in distant sites (p = 0.029) in a cohort of BC-affected patients (Zollo et al., 2005). These results were also recently confirmed in patients with TNBC through TMA immunohistochemistry technologies (Ferrucci et al., 2021). In detail, the levels of Prune1 protein had a positive correlation with distant metastasis (lung) and infiltrating pro-tumorigenic tumor-associated macrophages (M2-TAMs) with antiinflammatory functions in the tumor microenvironment (TME). Furthermore, a genetically engineered mouse model (GEMM) of metastatic TNBC characterized by the overexpression of both human Prune1 and Wnt1 in breast [through the use of the mouse mammary tumour virus (MMTV) promoter, i.e., MMTV-Prune1/Wnt1] revealed that Prune1 enhances the polarization of TAMs toward the M2 phenotype in the TME via the activation of the canonical (SMAD2-mediated) TGF-β pathway, IL17F secretion, and extracellular vesicle protein content modulation (Ferrucci et al., 2021). Worth noting is that the non-toxic small anti-Prune1 molecule (i.e., AA7.1) was found with the ability to impair the extracellular crosstalk between TNBC cells characterized by Prune1 overexpression and TAMs, thus reducing the metastatic properties of the tumorigenic cells in vivo by using a GEMM of metastatic TNBC (i.e., MMTV-Prune1/Wnt1) (Ferrucci et al., 2021).

**State of the art**

WO2014/045310 discloses pyrimido[5,4-d]pyrimidine and pyrimidine derivatives inhibiting h-prune activity and specifically h-prune cAMP-PDE activity, cell proliferation and cell motility, and their uses in the treatment of cancer overexpressing H-prune, including metastatic cancer. The following metastatic carcinoma are disclosed as possible target of the pyrimido[5,4-d]pyrimidine and pyrimidine derivatives disclosed in WO'310: breast, prostate, colon, pancreas, lung small and non small (NSCL), hepatocarcinoma, gastric and esophageal medulloblastoma, neuroblastoma, glioma, oligodendrioma, astrocytoma, sarcoma, lymphoma, multiple myeloma, leukemia, ovarian, head and neck, melanoma, squamous and basal cell.

One of the compounds disclosed in WO2014/045310, namely (R)-1-(4-((4-methoxybenzyl)amino)pyrimidin-2-yl)pyrrolidin-3-ol (AA7.1) has been reported to decrease Prune1 protein intracellular levels via enhancement of its proteasomal-dependent degradation (Ferrucci et al., 2018). Protein-drug interaction studies (via NMR approaches) showed the amino acid residues of Prune1 that are mainly responsible for the binding to AA7.1 (i.e., L359 and D364) (Ferrucci et al., 2018). This molecule was also found able to decrease Prune1 mRNA and protein levels in different MB group 3 (Ferrucci et al., 2018) and TNBC (Ferrucci et al., 2021) cells. In vitro and in vivo assays also showed the ability of AA7.1 to decrease the cell proliferative and migratory processes. In detail, in MB group 3, AA7.1 impaired the metastatic axis driven by Prune1, thus leading to impairment in TGF-β, decreased levels of OTX2, upregulation of PTEN, inhibition of EMT, reduction in Nestin, and increases in Tuj1 and GFAP differentiation neuronal markers (Ferrucci et al., 2018). These results suggest the potential of AA7.1 to inhibit the neural stem/progenitor cell markers and to increase neuronal differentiation processes, thus inhibiting the role of Prune 1 in these very important biological processes during neurogenesis.

Furthermore, the pharmacological inhibition of Prune1 protein in TNBC (achieved through AA7.1 treatment) decreased the number of metastatic foci in vivo also via inhibiting the switch of TAMs in the TME toward the M2 phenotype (Ferrucci et al., 2021). In TNBC, AA7.1-mediated tumor inhibition occurred through the impairment of the TGF-β pathway, reduction of inflammatory cytokines (i.e., IL-17F) and the modulation of the protein content of extracellular vesicles (i.e., vimentin) (Ferrucci et al., 2021). Importantly, this small molecule is not toxic, as suggested by the lack of acute toxicity measured in naive mice (Balb/C) that were intraperitoneally administered escalating doses (15, 30, and 60 mg/kg) of AA7.1 daily for 1 week (Ferrucci et al., 2021). The results showed no immediate acute toxicity of AA7.1 (in terms of hematological parameters, hepatotoxicity, or nephrotoxicity) in treated mice, as measured via glutamate-pyruvate transaminase 1, glutamic oxaloacetic transaminase, creatinine blood levels, and blood urea nitrogen (Ferrucci et al., 2018). Thus, altogether, these findings suggest that AA7.1 is a non-toxic potential immunomodulatory molecule with the ability to modulate the inflammatory processes in the TME, thus inhibiting the metastatic spread in Prune1-overexpressing tumors.

**Description of the invention**

It has been surprisingly found that modifying certain moieties of AA7.1 molecule, the compound anti-tumor profile can be improved in terms of cell proliferation inhibition, Prune1 degradation at protein and mRNA levels and effective dose able to exert anti-tumor activity.

Accordingly, in a first embodiment, the invention is directed to an anti-tumor compound of formula (I), a salt or enantiomer thereof:

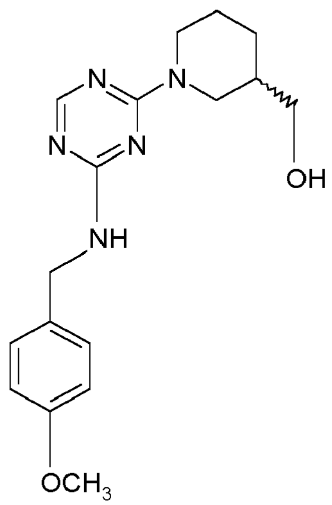

(I)

In a preferred embodiment, the compound of formula (I) is the (S) enantiomer, namely *(S)-(1-(4-((4-methoxybenzyl)amino)-1,3,5-triazin-2-yl)piperidin-3-yl)methanol.*

Any pharmaceutically acceptable salt of the compound (I) can be used according to the invention, the hydrochloride salt being preferred.

The compound (I) can be prepared according to the following scheme:

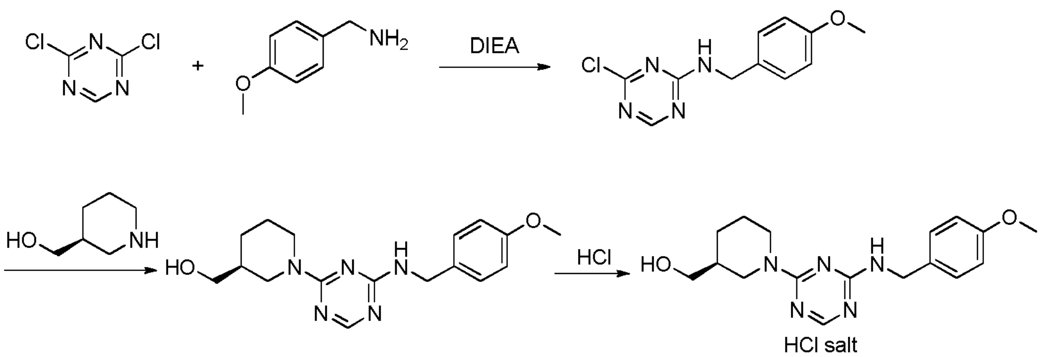


wherein DIEA is N,N-Diisopropylethylamine.

Compound (I) proved able to enhance the degradation of Prune 1, inhibit cell proliferation and migration rate, in *in vitro* and *in vivo* studies. In particular, the inventors performed various in vitro and in vivo experiments to assess the efficacy of compound (I) towards impairing Prune 1, cell migration and proliferation, in particular using highly metastatic models of medulloblastoma and TNBC.

According to the invention, compound (I) can be used in the treatment or prevention of Prune1-overexpressing tumors or tumor metastasis, particularly brain cancer, medulloblastoma, gastric cancer, esophageal squamous cell carcinoma, thyroid cancer, colorectal cancer, neuroblastoma, breast cancer, triple-negative breast cancer.

Compound (I) is administered to a subject in need thereof in a therapeutically effective amount. By "therapeutically effective amount" it is meant an amount of a compound (I) that, when administered alone or in combination with an additional therapeutic agent, is effective to treat or prevent a tumor disease.

In particular, the compound of the present invention can be administered alone or in combination with one or more chemotherapeutic agents. By "administered in combination" or "combination therapy" it is meant that a compound

of the present invention and one or more additional therapeutic agents are administered at the same time or sequentially in any order at different points in time to produce the desired therapeutic effect.

In a preferred embodiment of the invention, the compound (I) is administered in combination with a chemotherapeutic agent which is selected from vincristine, a LSD1 (lysine demethylase 1) inhibitor, preferably the LSD1 inhibitor seclidemstat, carboplatin, cisplatin, cyclophosphamide.

The therapeutic combination of compound (I) with vincristine or seclidemstat is particularly useful for the treatment of brain cancer, medulloblastoma and breast cancer, and as such preferred.

*Vincristine* belongs to a group of drugs known as the vinca alkaloids derived from the periwinkle plant. *Vincristine* acts by binding to tubulin and inhibiting the formation of microtubules thus causing mitosis to arrest at metaphase, through the disruption of mitotic spindle formation. The United States Food and Drug Administration (FDA) approved indications of *vincristine* are acute lymphocytic leukemia, lymphoid blast crisis of chronic myeloid leukemia, and Hodgkin and Non-Hodgkin lymphoma. *Vincristine* also has several off-label uses that include central nervous system (CNS) tumors [including MB], Ewing sarcoma, gestational trophoblastic tumors, multiple myeloma, ovarian cancer, primary CNS lymphoma, small cell lung cancer, and advanced thymoma in adult patients. To date, according to PNET5 protocol, *Vincristine* is used for MB average risk group in the maintenance chemotherapy after radiotherapy at the dose of 1.5 mg/m2. The synergistically action between compound (I) and *Vincristine* allows the dose of the chemotherapeutics to be diminished more than 100 fold. Compound (I) is able to exert a synergistic action also with other chemotherapeutics currently used according to the PNET 5 protocol for the management of (i) standard risk (Carboplatin 35mg/m2), (ii) high risk (Cisplatin 70mg/m2, Cyclophosphamide 1000mg/m2), and (iii) average risk group MB patients during their maintenance therapy (Cisplatin 70mg/m2, CCNU 75mg/m2 and Cyclophosphamide 1000mg/m2).

Seclidemstat [SP-2577] is currently used in clinical trial for several Advanced Solid Tumors. It is an inhibitor of the enzyme lysine demethylase 1 (Kdm1a/Lsd1), whose levels are higher in Medulloblastoma, thus cooperating with Gfilto inhibit genes involved in neuronal commitment and differentiation. The genetic ablation of Lsd1 or its pharmacological inhibition was found to impair Medulloblastoma growth in vivo (Lee et al., 2019).

A further object of the present invention is a pharmaceutical composition comprising compound (I) alone or in combination with chemotherapeutic agents as herein disclosed. Besides the biologically active compounds, the pharmaceutical composition may contain pharmaceutical excipients or carriers suitable for the intended form of administration, such as inert carriers, diluents, binders, lubricants, disintegrating agents, coloring agents. Preferably, the composition is in a form suitable for oral or parenteral administration. Suitable oral administration forms according to the invention include tablets, capsules, powders, granules, suspensions, syrups, while parenteral forms include intravenous, intraperitoneal, subcutaneous or intramuscular forms. The compound may be administered in a single or multiple daily dose. The dosage regimen for the compound of the present invention may vary depending on the mode and route of administration; the patient's age, sex, health, medical condition, weight, symptoms, renal and hepatic function. Generally, the dose can range from about 0.001 to 1000 mg/kg of body weight, preferably from about 0.01 to 100 mg/kg of body weight, and most preferably from 0.1 to 100mg mg/kg/day.

The invention will be further illustrated by the following Figures and examples.

## DESCRIPTION OF THE FIGURES

**Figure 1: LEO-AA7.5(S)T binds Prune1 protein at its the Carboxyl-terminus domain and promotes its degradation in metastatic Medulloblastoma cells belonging to Group 3 (i.e., D425-MED cells) to impair cell proliferation rate.**

**(A)** The chemical synthesis method employed for LEO-AA7.5(S)T is shown. **(B)** The chemical structure of LEO-AA7.5(S)T is shown, together with its IUPAC name, the chemical formula, and the molecular weight (MW). **(C)** Real-time cell proliferation analyses in D425-MED cells (Cell Index; i.e., cell-sensor impedance expressed every 2 min) treated with escalating doses of LEO-AA7.5(S)T (from 10 μM to 100 μM). Water was used as vehicle control cells. IC50 values are calculated through nonlinear regression analysis performed with Graph Pad Prism 9 ([inhibitor] vs. response [three parameters]) for LEO-AA7.5(S)T. Briefly, D425-MED cells were plated (8.000 cells) and treated with the indicated concentrations of LEO-AA7.5(S)T; with vehicle-treated cells (water) as the negative control. Impedance was measured every 2 min over 24 h. The IC50 values were calculated using Graph Pad Prism 9 (71 μM, R2 0.9). Data are means ±SD of three independent experiments. Black line, vehicle; dark grey, 10 μM LEO-AA7.5(S)T; light grey, 100 μM LEO-AA7.5(S)T. **(D)** Quantification of mRNA abundance relative to that in control (Vehicle) cells ($2^{-\Delta\Delta Ct}$) for human PRUNE1 in D425-MED cells treated with 10 μM LEO-AA7.5(S)T for 24 hours. Data are means ±SD. ** P <0.01 (as determined by unpaired two-tailed Student's *t*-tests; n = three independent experiments per group). Black box, vehicle; light grey, 10 μM LEO-AA7.5(S)T. **(E)** Representative immunoblotting analysis of total protein lysate fractions obtained from D425-MED cells, using antibodies against the indicated proteins. β-Actin was used as the loading control. The experiment was performed in triplicate. Right: Densitometry analysis of Prune1 band intensities from blots from three independent experiments. Black box, vehicle; light grey, 10 μM LEO-AA7.5(S)T. Data are means ±SD. * p <0.05 (unpaired two-tailed

student's t-tests; N = 3 independent experiments per group). Vehicle (water)-treated cells were used as control.

**Figure 2: LEO-AA7.5(S)T inhibits tumor growth in vivo in murine xenograft model of metastatic Medulloblastoma in vivo**

**(A)** Representative orthotopic xenograft experiment following injection into the fourth ventricle of nude mice (n=14) of Medulloblastoma Group3 cells (D425-MED) stably expressing Firefly Luciferase gene (i.e., D425-MED-Luc; **Ferrucci et al., 2018)** ($1 \times 10^5$) cells. After 7 days from tumour implantation (i.e., once the tumours were established) the mice (grouped according to their bioluminescence values) were injected intraperitoneally with LEO-AA7.5(S)T at 6 mg/kg/day, or with water as the vehicle for the control group. (B) Representative in vivo bioluminescence (BLI) images of mice orthotopically transplanted using D425-Luc cells implanted (n = 7 control mice, n = 7 treated mice). Mice were imaged every 7 days using an imaging system (IVIS 3D Illumina; Xenogen/ Caliper) to monitor tumour growth from time of implantation (T0) to 21 days after tumour implantation. **(C)** Quantification of photon emission (p/s) from the region of interest (ROI) in mice orthotopically injected with D425-MED-Luc cells and treated in vivo with LEO-AA7.5(S)T (6 mg/kg/day, intraperitoneally) or with water as vehicle. The difference in total flux from the two groups of mice at 21 days from cell orthotopic implantation (T21; after 14 days of treatment) showed impairment of tumour growth in cerebellum in vivo in the LEO-AA7.5(S)T-treated mice; Data are means ± SD, *$P < 0.05$. **(D)** Evaluation of drug toxicity using mice body weight in LEO-AA7.5(S)T -treated and control mice. Data are means ± SD. No significant differences were observed between the two groups, thus suggesting absence of drug-induced hepatotoxicity and renal failure. Black line, vehicle; light grey, 6 mg/Kg/day LEO-AA7.5(S)T. **(E)** Representative immunoblotting analysis of total protein lysate fractions obtained from tumour tissue from orthotopic xenograft experiment. Densitometric values given showed downregulation of Prune1, in n.3 LEO-AA7.5(S)T-treated mice, compared to n.3 vehicle mice. LEO-AA7.5(S)T-treated mice also showed decrease in phospho-Ser467-SMAD2, Phospho-Smad3 (Ser423/425) and OTX2 and upregulation of phospho-S380-PTEN. β-Actin and α-tubulin levels were used as loading controls. The experiment was performed in triplicate. Bottom: Densitometry analysis of targets band intensities from blots from three independent experiments. Data are means ±SD. * $p < 0.05$ (unpaired two-tailed student's t-tests; N = 3 independent experiments per group). Black box, vehicle; light grey, LEO-AA7.5(S)T. N.S., not significant; Vehicle (water)-treated cells were used as control.

**Figure 3: LEO-AA7.5(S)T inhibits tumor growth in vivo in murine xenograft model of metastatic Medulloblastoma in vivo**

**(A)** The plasmid construct used for the generation of the GEMM of Medulloblastoma driven by human PRUNE1 is shown. The plasmid is characterized by human Prune1 cDNA fused to FLAG sequence under the control of MATH1 promoter, in order to overexpress human Prune1 during developing cerebellum (i.e., P0-P4). The same DNA construct also has cloned the Firefly Luciferase (Luc) gene under the control of Cyclin B2 promoter, whose expression is mainly found in proliferating cells during G2/ M transition. **(B)** The resulting GEMM overexpressing Prune1 in the developing cerebellum developed metastatic MB in a background TP53 null (i.e., TP53-/- in FVB strain), as shown by in vivo bioluminescence approach. **(C)** Quantification of mRNA abundance relative to that in control (FVB) cerebellum ($2^{-\Delta\Delta Ct}$) for murine genes belonging to the metastatic axis (Ferrucci et al., 2018) and the molecular MB subgroups, as indicated. The tumors generated by a mouse model of SHH molecular subgroup driven by PTC and TP53 mutations are used as further control. The data show that the tumors generated by MATH1-Prune1/ TP53-/- are characterized by increased expression of 2 out of 4 genes belonging to Group 3 (i.e., NPR3 and GABRA3), 2 out of 4 genes belonging to Group SHH (i.e., SFRP1 and HHIP, mostly due to the overexpression of MATH1) and none of those genes markers of Group 4 MB. These results suggest that the tumors generated by MATH1-Prune1/ TP53-/- are characterized by increased expression of those genes belonging to Group 3 and SHH, probably due to the increased expression of MATH1). Black box, PTCH1-TP53-/-; light grey, MATH1-PRUNE1/TP53-/-; dark grey, cerebellum FVB P60. **(D)** Real-time cell proliferation analyses in murine metastatic Medulloblastoma cells obtained from primary tumors collected from a genetic model of Medulloblastoma Group 3 characterized by the overexpression of human Prune1-FLAG in the developing cerebellum under the control of MATH1 promoter (MATH1-Prune1). The same genetically engineered model (GEMM) also expresses the Firefly luciferase gene only in those proliferating (e.g., tumorigenic) cells under the control of Cyclin B2 promoter (MATH1-Prune1-CyclinB2-Luc). This GEMM was obtained in a genetic background characterized by the absence of both alleles of TP53 gene (MATH1-Prune1/ CyclinB2-Luc/ TP53-/-; i.e., MATH1-Prune1/TP53-/-). The cell proliferation analyses (Cell Index; i.e., cell-sensor impedance expressed every 2 min) were obtained by treating those tumorigenic MATH1-Prune1/TP53-/- cells treated with escalating doses of LEO-AA7.5(S)T (from 6.5 μM to 75 μM). Water was used as vehicle control cells. IC50 values are calculated through nonlinear regression analysis performed with Graph Pad Prism 9 ([inhibitor] vs. response [three parameters]) for LEO-AA7.5(S)T. Vehicle-treated cells (water) was used as the negative control. Impedance was measured every 2 min over 24 h. The IC50 values were calculated using Graph Pad Prism 9 (31.1 μM, R2 0.97). Data are means ±SD of three independent experiments. **(E)** Quantification of mRNA

abundance relative to that in control (Vehicle) cells ($2^{-\Delta\Delta Ct}$) for human and murine PRUNE1 in MATH1-Prune1/TP53-/- cells treated with 10 $\mu$M LEO-AA7.5(S)T for 24 hours. Data are means $\pm$SD. *, P <0.05; **, P <0.01 (as determined by unpaired two-tailed Student's *t*-tests; n = three independent experiments per group). Dark grey box, vehicle; light grey, 10 $\mu$M LEO-AA7.5(S)T. **(F)** Representative immunoblotting analysis of total protein lysate fractions obtained from MATH-Prune1/TP53-/- cells, using antibodies against the indicated proteins. $\beta$-Actin was used as the loading control. The experiment was performed in triplicate. The values from densitometry analysis of target band intensities from blots from three independent experiments are shown. Data are means $\pm$SD. Vehicle (water)-treated cells were used as control.

**Figure 4: LEO-AA7.5(S)T exerts immunomodulatory action by modulating the secretion of inflammatory cytokines from murine Medulloblastoma cells**

**(A)** Quantification of mRNA abundance ($2^{-\Delta\Delta Ct}$) for the indicated cytokines in MATH1-Prune1/TP53-/- cells upon treatment with 10 $\mu$M AAT.5(S)T or water as vehicle control for 24 hours. All data are expressed as the mean $\pm$ standard deviation. **, P<0.01 (as determined by unpaired two-tailed Student's *t*-tests; n = three independent experiments per group). Dark grey box, vehicle; light grey, 10 $\mu$M LEO-AA7.5(S)T. **(B)** Mouse cytokine antibody arrays incubated with pooled conditioned media (CM) from MATH1-Prune1/TP53-/- cells upon treatment with 10 $\mu$M AAT.5(S)T or water as vehicle control for 24 hours. **(C)** Mouse cytokine antibody array was performed to determine expression levels of 61 cytokines in conditioned media (CM) from LEO-AA7.5(S)T-treated MATH1-Prune1/TP53-/- cells. Among those, n.16 cytokines resulted modulated by LEO-AA7.5(S)T treatment. **(D)** The fold-induction of those n.16 cytokines whose expression was statistically significant modulated by LEO-AA7.5(S)T treatment in the conditioned media collected from MATH1-Prune1/TP53-/- cells was shown. Among these, n.6 cytokines resulted upregulated, while the other n.10 was downregulated upon LEO-AA7.5(S)T treatment. All experiments were performed in triplicate. All data are expressed as the mean $\pm$ standard deviation. *, P<0.05; **, P<0.01 (as determined by unpaired two-tailed Student's *t*-tests; n = three independent experiments per group). Dark grey box, vehicle; light grey, 10 $\mu$M LEO-AA7.5(S)T. **(E)** Protein network and related functions generated via Search Tool for Retrieval of Interacting Genes/Proteins (STRING) database using those significantly downregulated cytokines in the conditioned media collected from LEO-AA7.5(S)T-treated MATH1-Prune1/TP53-/- cells is shown. The functions are mostly involved in leucocytes chemotaxis, positive activation of leucocytes migration (including lymphocytes chemotaxis) and positive activation of T cells.

**Figure 5: Immunosuppressive Regulatory T cells (Tregs) in the tumor microenvironment of human MB Group 3 - 4 and in the tumors generated by MATH1-Prune1/TP53-/-**

**(A)** Representative Immunofluorescence (IF) Staining of Primary Tumour Sections from human MB Group 3 - 4 affected patients with the indicated antibodies against CD4, CD8, PD1 and FOXP3 proteins. The percentage of CD4+PD1+, CD4+FOXP3+ and CD8+PD1+, CD8+FOXP3+ cells are shown. More than n.700 cells were counted. Magnification 40x. CD4 (light grey, membrane), CD8 (dark grey, membrane), FOXP3 (dark grey nuclear), and PD1 (white, membrane). **(B)** Representative Immunofluorescence (IF) Staining of Primary Tumour Sections from MATH1-Prune1/TP53-/- model affected patients with the indicated antibodies against CD4 and FOXP3 proteins. Magnification 40x. CD4 (light grey, membrane), FOXP3 (dark grey nuclear). DRAQ5 (light grey nuclear) was used for nuclei staining.

**Figure 6: LEO-AA7.5(S)T impairs the conversion from conventional- to regulatory- T cells (i.e., Tconv to Treg) driven by medulloblastoma Group 3 cells.**

**(A)** Conditioned media (CM) from pooled conditioned media (CM) from MATH1-Prune1/TP53-/- cells upon treatment with 10 $\mu$M AAT.5(S)T or water as vehicle control was collected for 24 hours. CD4+/ CD8+ T cells isolated from the spleen of naive FVB mice were grown in the conditioned media for 48 hours. Untreated T cells were used as negative control of the experiment. **(B)** FACS analysis showing the scatter signals (**left**) and immunofluorescence staining **(right)** of T cells were isolated from single-cell suspension obtained from naive FVB mouse spleen using the Pan T Cell Isolation Kit II, an LS Column. **(C)** Immunofluorescence staining with against murine CD4 (light grey, membrane) and murine FOXP3 (light grey, nuclear) proteins in T cells grown for 48 hours in the conditioned media collected from vehicle- or LEO-AA7.5(S)T-treated MATH1-Prune1/TP53-/- cells. Untreated T cells were used as negative control of the experiments. DAPI (light grey nuclear) was used for nuclei staining. The percentage of CD4+FOXP3+ and CD4+FOXP3- cells is also shown. The SIM2 image was acquired with Elyra 7 and processed with Zeiss ZEN software (blue edition). Magnification, $\times$63. Scale bar, 5 $\mu$m. More than n.50 cells were counted.

**Figure 7: LEO-AA7.5(S)T impairs the medulloblastoma Group 3 tumorigenesis by targeting the immunosuppressive tumor microenvironment.**

**(A)** Representative orthotopic experiment following injection into the fourth ventricle of immunocompetent mice (FVB WT, n=10) with MATH-1-Prune_1 / TP53-/- (1.5 $\times 10^5$) cells. after 10 days from tumour implantation (i.e., once the tumours were established) the mice (grouped according to their bioluminescence values) were injected intraperitoneally with **LEO-AA7.5(S)T** at 6 mg/kg/day, or with water as the vehicle for the control group. **(B)** Representative BLI images of mice orthotopically transplanted using MATH-1-Prune_1 /TP53-/- cells implanted (n = 5 control mice, n = 5 treated mice). Mice were imaged every 7 days using an imaging system (IVIS 3D Illumina; Xenogen/ Caliper) to monitor tumour growth from time of implantation (T0) to 17 days after tumour implantation. **(C)** Quantified photon emission (p/s) from the region of interest of orthotopically mice injected with MATH1-Prune_1 / TP53-/- cells treated in vivo with LEO-AA7.5(S)T (6 mg/kg/day, intraperitoneally) or with water as vehicle. The difference in total flux from the two groups of mice at 17 days from cell orthotopic implantation (T17; after 7 days of **LEO-AA7.5(S)T** showed impairment of tumour growth in cerebellum in vivo in the AA7.5-treated mice; Data are means $\pm$ SD, *P <0.05; **P<0.01; unpaired two-tailed student's t-tests. Dark grey line, vehicle; light grey, LEO-AA7.5(S)T. Ex vivo quantified photon emission (p/s) from the region of interest of metastatic foci in the spinal cord from the orthotopically mice injected with MATH1-Prune_1 / TP53-/- cells treated in vivo with LEO-AA7.5(S)T (6 mg/kg/day, intraperitoneally) or with water as vehicle. The difference in total flux from the two groups of mice at 17 days from cell orthotopic implantation (after 7 days of **LEO-AA7.5(S)T** treatment) showed decrease of metastasis in vivo in the LEO-AA7.5(S)T-treated mice; Data are means $\pm$ SD, *P <0.05; unpaired two-tailed student's t-tests). Dark grey box, vehicle; light grey, LEO-AA7.5(S)T. **(D)** The amount of CCL2 in the sera collected from the vehicle- (n.5) and **LEO-AA7.5(S)T** - (n.5) treated mice was evaluated via enzyme-linked immunosorbent assays (ELISAs). Data are means $\pm$ SD, *P <0.05; **P<0.01; unpaired two-tailed student's t-tests. Dark grey box, vehicle; light grey, LEO-AA7.5(S)T. **(E)** Representative Immunofluorescence (IF) Staining of Primary Tumour Sections from Transplanted Mice Treated with LEO-AA7.5(S)T or with water as a Vehicle. (a) IF multistaining with CD4 (expressed by both T helper and dendritic cells), CD8 (cytotoxic T cells), programmed cell death protein 1 (PD1, marker of immunosuppressive immune cells), FOXP3 (marker of regulatory T cells, Tregs, red) and DAPI was used to stain the nuclei. (b-f) the percentage of positive cells counted in double fluorescence staining (b) T regulatory cells CD4+ FOXP3+ (CD4, FOXP3+, ***p < 0.000 ), (c) immunosuppressive lymphocytes CD4+ PD1+ (CD4, PD1+, ***p < 0.000), (d) T regulatory cells CD8+ FOXP3+ (CD8, FOXP3+ , ***p < 0.000 ) (e) immunosuppressive lymphocytes CD8+PD1+ (CD8, PD1+, ***p < 0.000. DAPI was used to stain the nuclei. Data are means $\pm$ SD. CD4 (light grey, membrane), CD8 (dark grey, membrane), FOXP3 (dark grey nuclear), and PD1 (dark grey, membrane).

**Figure 8: LEO-AA7.5(S)T ameliorates mice survivals in vivo by impairing medulloblastoma Group 3 tumorigenesis**

**(A)** Representative orthotopic experiment following injection into the fourth ventricle of immunocompetent mice (FVB WT, n=20) with MATH1-Prune1/ TP53-/- (1.5 $\times 10^5$)cells. after 10 days from tumour implantation (i.e., once the tumours were established) the mice (grouped according to their body weight) were injected intraperitoneally with AA7.5 at 6 mg/kg/day, or with water as the vehicle for the control group. **(B)** The survival time of two groups was observed for 37 days after tumour implantation. Dark grey box, vehicle; light grey, LEO-AA7.5(S)T. **(C)** Evaluation of drug toxicity using mice body weight in AA7.5 -treated and control mice. Data are means $\pm$ SD, *P <0.05; **P<0.01; ; unpaired two-tailed student's t-tests. Dark grey box, vehicle; light grey, LEO-AA7.5(S)T.

**Figure 9: LEO-AA7.5(S)T acts synergistically with chemotherapeutics (i.e., Vincristine) currently used in clinics for the treatment of Medulloblastoma.**

**(A)** Real-time cell proliferation analyses (Cell Index; i.e., cell-sensor impedance expressed every 2 min) were obtained by treating those tumorigenic MATH1-Prune1/TP53-/- cells treated with escalating doses of Vincristine (from 0.9 $\mu$M to 91 $\mu$M). 0.01% DMSO was used as vehicle control cells. IC50 values are calculated through nonlinear regression analysis performed with Graph Pad Prism 9 ([inhibitor] vs. response [three parameters]) for Vincristine. Impedance was measured every 2 min over 24 h. The IC50 values were calculated using Graph Pad Prism 9 (35 $\mu$M, R2 0.93). Data are means $\pm$SD of three independent experiments. **(B)** Caspase 3 activity measured in MATH1-Prune1/TP53-/- cells ($5\times 10^5$ cells/well in a 24-well plate and incubated overnight) treated with different doses (i.e., 0.3 nM or 0.03 nM) Vincristine alone or in combination with LEO-AA7.5(S)T. Vehicle-treated cells and cells treated with 1 $\mu$M Staurosporine were used as negative and positive controls, respectively. Data are presented as relative fluorescent units (RFUs; excitation: 380 nm; emission: 460 nm) measured after 1 hour of incubation (T1h). Data are means $\pm$ SD. *P < 0.05; N.S., nonsignificant (as determined by unpaired two-tailed Student's t test; n = three independent experiments per group). **(C)** Real-time cell proliferation analyses (Cell Index; i.e., cell-sensor impedance expressed every 2 min) were

obtained by treating those tumorigenic D425-MED cells treated with escalating doses of Vincristine (from 0.9 μM to 91 μM). 0.01% DMSO was used as vehicle control cells. IC50 values are calculated through nonlinear regression analysis performed with Graph Pad Prism 9 ([inhibitor] vs. response [three parameters]) for Vincristine. Impedance was measured every 2 min over 24 h. The IC50 values were calculated using Graph Pad Prism 9 (3.86 μM, R2 0.95). Data are means ±SD of three independent experiments. **(D)** Real-time cell proliferation analyses (Cell Index; i.e., cell-sensor impedance expressed every 2 min) were obtained by treating those tumorigenic D425-MED cells treated with escalating doses of Vincristine (10 nM or 1 nM) alone or in combination with 10 μM LEO-AA7.5(S)T. 0.01% DMSO was used as vehicle control cells. **(E)** Representative orthotopic experiment following injection into the fourth ventricle of immunocompetent mice (FVB WT, n=10) with MATH1-Prune1/TP53-/- ($1.5 \times 10^5$) cells. After 10 days from tumour implantation (i.e., once the tumours were established) the mice (grouped according to their bioluminescence values) were injected intraperitoneally with LEO-AA7.5(S)T at 6 mg/kg/day, or with water as the vehicle for the control group. The mice were imaged every seven days, to monitor tumour growth using an imaging system (IVIS 3D Illumina; Xenogen/ Caliper). **(F)** Tumour growth according to quantified photon emission (p/s) from the region of interest of orthotopically mice injected with MATH1-Prune1/ TP53-/- cells treated in vivo with LEO-AA7.5(S)T (6 mg/kg/day, intraperitoneally) or with water as vehicle. **(G)** The difference in total flux from the two groups of mice after n.17 days from cell orthotopic implantation (T17; after 7 days of AA7.5) showed impairment of tumour growth in cerebellum in vivo in the LEO-AA7.5(S)T-treated mice; Data are means ± SD, *P < 0.05. Black line, vehicle; dark grey, Vincristine; light grey, Vincristine combined to LEO-AA7.5(S)T. **(H)** Representative *in- vivo* and *ex- vivo* tumour growth and angiogenesis analysis. After n.18 days from treatment start (i.e., 25 days from cell injection), the mice were injected with a fluorescent imaging probe (IVISense Integrin Receptor 680 Fluorescent Probe (IntegriSense; Perkin Elmer). Black line, vehicle; dark grey, Vincristine; light grey, Vincristine combined to LEO-AA7.5(S)T.

**Figure 10: (S)-(1-(4-((4-methoxybenzyl)amino)-1,3,5-triazin-2-yl)piperidin-3-yl)methanol HCl salt compound acts synergistically with *Seclidemstat [SP-2577]* to inhibit MB proliferation in vitro.**

**(A-B)** Real-time cell proliferation analyses (Cell Index; i.e., cell-sensor impedance expressed every 2 min) were obtained by treating those tumorigenic MATH1-Prune1/TP53-/- cells (A) or D425-Med cells (B) treated with escalating doses of *Seclidemstat [SP-2577]* or 0.01% DMSO as vehicle control cells. IC50 values are calculated through nonlinear regression analysis performed with Graph Pad Prism 9 ([inhibitor] vs. response [three parameters]). Impedance was measured every 2 min over 24 h. The IC50 values were calculated using Graph Pad Prism 9. Data are means ±SD of three independent experiments. **(C)** Real-time cell proliferation analyses (Cell Index; i.e., cell-sensor impedance expressed every 2 min) were obtained by treating those tumorigenic D425-MED cells treated with *Seclidemstat [SP-2577]* alone or in combination with 10 μM LEO-AA7.5(S)T. 0.01% DMSO was used as vehicle control cells.

**Figure 11: LEO-AA7.5(S)T impairs cell proliferation/ migration rate in vitro in murine metastatic triple negative breast cancer (TNBC) cells overexpressing Prune1 and inhibits their communication with immune cells (i.e., Macrophages).**

**(A)** Quantification of mRNA abundance ($2^{-\Delta Ct}$) for human and murine PRUNE 1 in murine primary cells obtained from a GEMM model of metastatic TNBC overexpressing human Prune1 and Wnt1 in mammary gland, under the control of Mouse Mammary Tumor Virus (MMTV) promoter (i.e., MMTV-Prune1/Wnt1 cells; **Ferrucci et al., 2021**). All data are expressed as the mean ± standard deviation. *, P<0.05; (as determined by unpaired two-tailed Student's *t*-tests; n = three independent experiments per group). Light grey, human Prune1; Dark grey, murine Prune1. **(B)** Real-time cell proliferation analyses (Cell Index; i.e., cell-sensor impedance expressed every 2 min) were obtained by treating those tumorigenic MMTV-Prune1/Wnt1 cells treated with escalating doses of LEO-AA7.5(S)T (10 μM and 100 μM). Water was used as vehicle control cells. Impedance was measured every 2 min over 24 h. Data are means ±SD of three independent experiments. **(C)** Real-time cell migration analyses (Cell Index; i.e., cell-sensor impedance expressed every 2 min) were obtained by treating those tumorigenic MMTV-Prune1/Wnt1 cells treated with escalating doses of LEO-AA7.5(S)T (10 μM and 100 μM). Water was used as vehicle control cells. Impedance was measured every 2 min over 24 h. Data are means ±SD of three independent experiments. **(D)** Quantification of mRNA abundance relative to that in control (Vehicle) cells ($T^{-\Delta\Delta Ct}$) for human and murine PRUNE1 in MMTV-Prune1/Wnt1 cells treated with 10 μM LEO-AA7.5(S)T for 24 hours. Data are means ±SD. *** P <0.001 (as determined by unpaired two-tailed Student's *t*-tests; n = three independent experiments per group). **(E)** Representative immunoblotting analysis of total protein lysate obtained from MMTV-Prune1/Wnt1 cells treated with 10 μM LEO-AA7.5(S)T for 24 hours, by using antibodies against the indicated proteins. β-Actin was used as the loading control. The experiment was performed in triplicate. Densitometry analysis of Prune1 band intensities from blots from three independent experiments. Data are means ±SD. *, p <0.05; **, p<0.01 (unpaired two-tailed student's t-tests; N = 3 independent experiments per group). Vehicle (water)-treated cells were used as control. **(F)** J774A.1 macrophages were grown for 48 h in conditioned media collected (after 24 h) from MMTV-Prune-1/Wnt1 treated with vehicle (water) or 10 μM LEO-AA7.5(S)T. **(G)** Real-time PCR analysis of some M2-associated genes, including IL-10, Arg-1, MMP-9, and IL-1β, in J774A.1 macrophages grown for 48 h in conditioned media from MMTV-Wnt1 cells treated with vehicle (water) or 10 μM LEO-AA7.5(S)T. Untreated J774A.1 macrophages were used as the negative controls (UNT, black). *, $P < 0.05$; **, $P<0.01$; ***, $P<0.001$ in unpaired two-tailed student's t-tests compared with untreated macrophages. The experiment was performed in triplicate. Black box, untreated; strong dark grey, vehicle; dark gray, 50 μM LEO-AA7.5(S)T; Light grey, 10 μM LEO-AA7.5(S)T.

**Figure 12: LEO-AA7.5(S)T is not toxic in vivo.**

**(A)** FVB mice (4-week-old) grouped according to body weight were intraperitoneally administered with escalating doses of LEO-AA7.5(S)T (60mg or 120mg/kg/day) or vehicle (water) for 12 days. At sacrifice, the blood and organs were collected and evaluated. Haematological and biochemical markers of hepatic and renal functions were evaluated. Data are expressed as means from four mice within each treatment group. **(B-D) LEO-AA7.5(S)T** does not show any signs of toxicity in the treated mice, as demonstrated by no decrease in liver **(B),** spleen **(C)** and kidney **(D)** weight in the treated mice compared to controls. **(E)** The absence of drug-induced hepatotoxicity and renal failure was shown by glutamate-pyruvate transaminase 1, glutamic oxaloacetic transaminase and creatinine blood levels, and blood urea nitrogen from the treated-mice. Black box, vehicle; dark grey, 60mg/kg/day μM LEO-AA7.5(S)T; light grey, 120 mg/kg/day LEO-AA7.5(S)T;

**Figure 13: LEO-AA7.5(S)T outperforms the compound AA7.1 in terms of target degradation and dose in vitro in MB and TNBC cells**

**(A)** Real-time cell proliferation analyses in D425-MED cells (Cell Index; i.e., cell-sensor impedance expressed every 2 min) treated with escalating doses of LEO-AA7.5(S)T (from 10 μM to 100 μM) and AA7.1 (100 μM). Water was used as vehicle control cells. Briefly, D425-MED cells were plated (8.000 cells) and treated with the indicated concentrations of LEO-AA7.5(S)T or AA7.1; with vehicle-treated cells (water) as the negative control. Impedance was measured every 2 min over 24 h. **(B)** Representative immunoblotting analysis of total protein lysate fractions obtained from D425-MED cells, using antibodies against the indicated proteins. β-Actin was used as the loading control. The experiment was performed in triplicate. Bottom: Densitometry analysis of Prune1 band intensities from blots from three independent experiments. Data are means ±SD. * $p <0.05$; ** $P<0.01$ (unpaired two-tailed student's t-tests; N = 3 independent experiments per group). Vehicle (water)-treated cells were used as control. **(C)** Quantification of mRNA abundance relative to that in control (Vehicle) cells ($2^{-\Delta\Delta Ct}$) for human PRUNE1 in D425-MED cells treated with 10 μM LEO-AA7.5(S)T or 100 μM AA7.1 for 24 hours. Data are means ±SD. ** $P <0.01$ (as determined by unpaired two-tailed Student's *t*-tests; n = three independent experiments per group). Black box, vehicle; dark grey, AA7.1; light grey, LEO-AA7.5(S)T; **(D)** Representative immunoblotting analysis of total protein lysate fractions obtained from MMTV-Prune1/Wnt1 cells, using antibodies against the indicated proteins. β-Actin was used as the loading control. The experiment was performed in triplicate. Bottom: Densitometry analysis of Prune1 band intensities from blots from three independent experiments. Data are means ±SD. * $p <0.05$ (unpaired two-tailed student's t-tests; N = 3 independent experiments per group). Vehicle (water)-treated cells were used as control. **(E)** Quantification of mRNA abundance relative to that in control (Vehicle) cells ($2^{-\Delta\Delta Ct}$) for human (light grey) and murine (dark grey) PRUNE1 in MMTV-Prune1/Wnt1 cells treated with 50/ 10 μM LEO-AA7.5(S)T or 100 μM AA7.1 for 24 hours. Data are means ±SD. *** $P <0.001$ (as determined by unpaired two-tailed Student's *t*-tests; n = three independent experiments per group).

**Figure 14: LEO-AA7.5(S)T outperforms the compound AA7.1 in terms of T cells conversion from Treg to Tconv in vivo and ex vivo**

**(A)** Conditioned media (CM) from pooled conditioned media (CM) from MATH1-Prune1/TP53-/- cells upon treatment with 10 μM LEO-AA7.5(S)T, 100 μM AA7.1 or water as vehicle control was collected for 24 hours. CD4+/CD8+ T cells isolated from the spleen of naive FVB mice were grown in the conditioned media for 48 hours. Untreated T cells were used as negative control of the experiment. Immunofluorescence staining with against murine CD4 (light grey, membrane) and murine FOXP3 (light grey, nuclear) proteins in T cells grown for 48 hours in the conditioned media collected from vehicle- or LEO-AA7.5(S)T-treated MATH1-Prune1/TP53-/- cells. Untreated T cells were used as negative control of the experiments. The percentage of CD4+FOXP3+ and CD4+FOXP3- cells is shown. The SIM2 image was acquired with Elyra 7 and processed with Zeiss ZEN software (blue edition). Magnification, ×63. Scale bar, 5 μm. More than n.50 cells were counted. **(B)** Representative orthotopic experiment following injection into the fourth ventricle of immunocompetent mice (FVB WT, n=13) with MATH1-Prune1/ TP53-/- ($3 \times 10^5$) cells. After 10 days from tumour implantation (i.e., once the tumours were established) the mice (grouped according to their bioluminescence values) were injected intraperitoneally with AA7.1 at 30 mg/kg/day or 60 mg/kg/day, or with water as the vehicle for the control group.

**(C)** Representative BLI images of mice orthotopically transplanted using MATH1-Prune1/TP53-/- cells implanted. Mice were imaged using an imaging system (IVIS 3D Illumina; Xenogen/ Caliper) to monitor tumour growth from time of implantation. **(D)** Quantified photon emission (p/s) from the region of interest of orthotopically mice injected with MATH1-Prune1/ TP53-/- cells treated in vivo with AA7.1 or with water as vehicle. The difference in total flux from the two groups of mice showed impairment of tumour growth in cerebellum in vivo in the AA7.1-treated mice; Data are means ± SD, *P <0.05; unpaired two-tailed student's t-tests. **(E)** Representative Immunofluorescence (IF) Staining of Primary Tumour Sections from Transplanted Mice Treated with AA7.1 or with water as a Vehicle for the antibody as indicated. DAPI was used to stain the nuclei. Data are means ± SD. CD4: light grey membrane; FOXP3: dark grey nuclear; PD1: dark grey membrane; DAPI (nuclei): dark grey.

## Example 1

**Synthesis of (S)-(1-(4-((4-methoxybenzyl)amino)-1,3,5-triazin-2-yl)piperidin-3-yl)methanol HCl salt (LEO-AA7.5(S)T)**

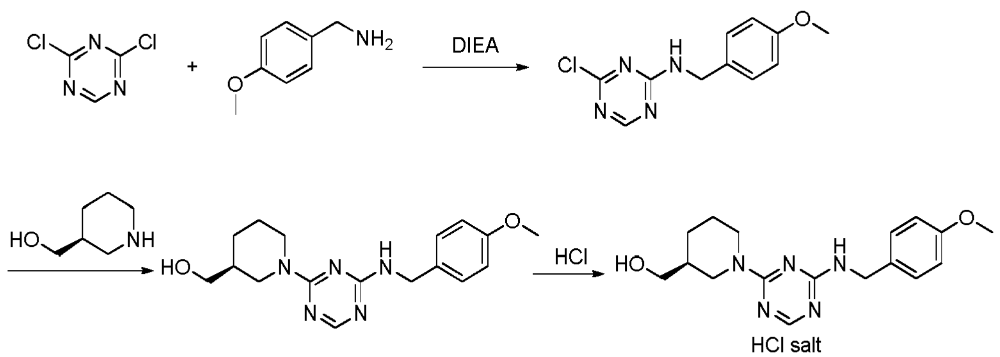


**Step 1**

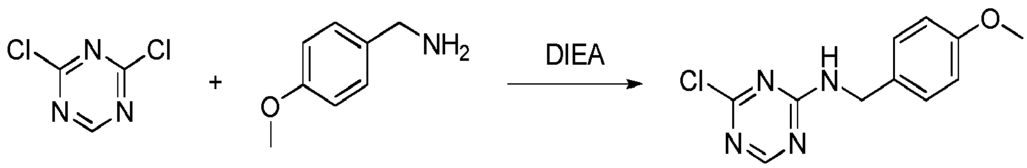


| compounds | M.W | Amount | MMol | Eq | supplier companies |
|---|---|---|---|---|---|
| 1 | 149.9 | 0.5g | 3.3mmol | 1.0 | energy chemical |
| PMBNH2 | 137 | 0.457g | 3.3mmol | 1.0 | energy chemical |
| DIEA | 139 | 0.517g | 4.0mmol | 1.2 | Sinopharm chemical reagent co., Ltd |
| DCM | | 20mL | | | Shanghai titan scientific co.,Ltd |

2,4-dichloro-1,3,5-triazine 0.5 g and DIEA 0.52 g was dissolved in 20 mL DCM at 0°C under $N_2$, (4-methoxyphenyl)methanamine 0.45 g in 2 mL DCM was added dropwise and the mixture was stirred at room temperature overnight. The reaction mixture was used in next step.

**Step 2**

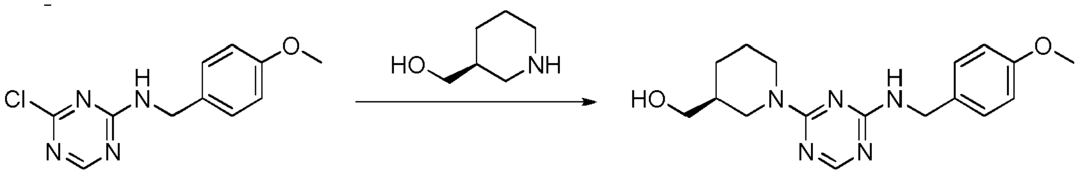

| compounds | M.W | Amount | Mol | Eq | supplier companies |
|---|---|---|---|---|---|
| A | 250.68 | 0.82g | 3.3mmol | 1.0 | |
| (S)-piperidin-3-ylmethanol | 115.17 | 0.38g | 3.3mmol | 1.0 | energy chemical |
| DIEA | 129 | 0.5 g | 4.0mmol | 1.2 | Sinopharm chemical reagent co., Ltd |
| DCM | | 20 ml | | | Shanghai titan scientific co..Ltd |

The reaction mixture of step 1 was cooled to 0°C, DIEA was added, then (S)-piperidin-3-ylmethanol in 2 mL DCM was added dropwise and the mixture was stirred at rt overnight, quenched the reaction with water and the product was extracted with DCM and washed with water, the product was purified by SG(DCM/MeOH=20/1) to get product 0.56 g.

**Step 3**

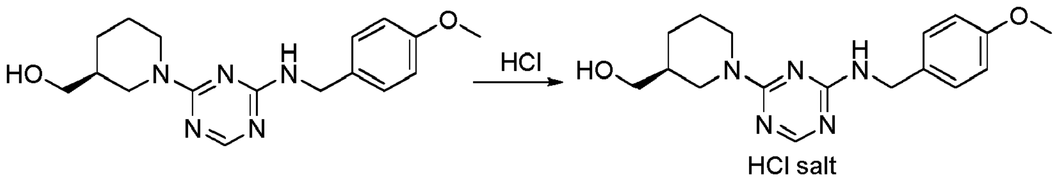


| compounds | M.W | Amount | Mol | Eq | supplier companies |
|---|---|---|---|---|---|
| A | 329 | 0.56 g | 1.7mmol | 1.0 | |
| 1N HCl | | 4.25mL | 4.25mmol | 2.5 | Sinopharm chemical reagent co., Ltd |
| H2O | | 20 ml | | | |

(S)-(1-(4-(4-methoxybenzylamino)-1,3,5-triazin-2-yl)piperidin-3-yl)methanol 0.56 g was suspended in 20 ml water, cooled to 0°C, 4.25 mL 1N HCl aq., was added and the suspension cleaned. The product was get by freeze-drying.

$^1$H NMR δ 1.28 (1H, $CH_2$C*H2*CH), 1.44 (1H, $CH_2$C*H2*CH2), 1.60 (1H, $CH_2$C*H*CH2), 1.73 (1H, $CH_2$C*H2*CH), 1.75 (1H, $CH_2$C*H2*CH2), 2.90 (1H, NC*H*$_2$), 3.06 (1H, NC*H*$_2$), 3.20 (1H, NC*H*$_2$), 3.28 (1H, NC*H*$_2$), 3.35 (1H, C*H2*OH), 3.41 (1H, C*H2*OH), 3.75 (3H, ArOC*H*$_3$), 4.49 (1H, OH), 4.60 (1H, C*H*$_2$Ar), 4.67 (1H, C*H*$_2$Ar), 6.93 (2H, 2 x CHC*H*COC$H_3$), 7.37 (2H, 2 x C*H*CHCOC$H_3$), 8.42 (1H, NH), 9.23 (1H, Triazine NC*H*N); MS ESI (m/z): $C_{17}H_{24}N_5O_2$ 330 $(M+H)^+$.

**Example 2**

**(S)-(1-(4-((4-methoxybenzyl)amino)-1,3,5-triazin-2-yl)piperidin-3-yl)methanol HCl salt compound is able to enhance the degradation of Prune1 protein and reduces tumorigenesis of metastatic Medulloblastoma belonging to Group 3 (i.e., D425-MED)**

The half maximal inhibitory concentration (IC50) of *(S)-(1-(4-((4-methoxybenzyl)amino)-1,3,5-triazin-2-yl)piperidin-3-yl)methanol HCl salt compound* for the inhibition of cell proliferation index was measured for a human MB Group3 cell model that carried TP53 mutation and c-MYC amplification (i.e., D425-Med cells), as 71 μM (R = 0.9; **Figure 1C**). This value is lower than the IC50 reported for AA7.1 in the same D425-Med cells (i.e., 129.99 +/- 52.05 μM (R = 0.97; Ferrucci et al., 2018). D425-Med cells were then treated with 10 μM *(S)-(1-(4-((4-methoxybenzyl)amino)-1,3,5-triazin-*

*2-yl)piperidin-3-yl)methanol HCl salt compound* for 24 hours, which resulted in a statistically significant decrease of endogenous Prune1 mRNA (0.3 fold, **Figure 1D)** and protein levels (0.6 fold; **Figure 1E**). As expected, this new molecule resulted efficacy on Prune1 target inhibition at a dose 10 fold lower than AA7.1 (10 μM vs 100 μM; Ferrucci et al., 2018). Further studies will dissect out the mechanism of actions that are responsible for Prune1 protein and mRNA degradation in the treated cells.

The modulation of MB growth and metastases by *(S)-(1-(4-((4-methoxybenzyl)amino)-1,3, 5-triazin-2-yl)piperidin-3-yl)methanol HCl salt compound* was then investigated in vivo using orthotopic xenograft mouse models of MB Group3 and D425-Luc cells (Asadzadeh et al., 2019). The dose of *(S)-(1-(4-((4-methoxybenzyl)amino)-1,3,5-triazin-2-yl)piperidin-3-yl)methanol HCl salt compound* was 6 mg/kg/day intraperitoneally.

In this in vivo preclinical trial, Medulloblastoma Group 3 cells (D425-MED) stably expressing Firefly Luciferase gene (i.e., D425-MED-Luc; Ferrucci et al., 2018) (1 $\times 10^5$ cells) were implanted into the fourth ventricle of nude mice (n=14). After 7 days from tumour implantation (i.e., once the tumours were established) the mice (grouped according to their bioluminescence values) were injected intraperitoneally with *(S)-(1-(4-((4-methoxybenzyl)amino)-1,3,5-triazin-2-yl)piperidin-3-yl)methanol HCl salt compound* at 6 mg/kg/day, or with water as the vehicle for the control group (**Figures 2A**). These data showed significantly decreased of intracranial tumor growth after 2 weeks of treatment (**Figures 2B, C**). Furthermore, an antimetastatic action in the treated mice is also shown (**Figures 2B**). In this regard, all the control-mice (7 out of 7) showed metastases in the spinal cord, while two out of seven treated-mice showed the presence of metastases (**Figures 2B**).

This anti-metastatic action is mostly due to the immunomodulatory action of the molecule, as shown by the cytotoxicity of the innate immune system through the activation of Natural Killer (NK) cells in the treated mice as compared to the control group (see the activation marker N-CAM1[CD56] in Figure 2E).

Here, *(S)-(1-(4-((4-methoxybenzyl)amino)-1,3,5-triazin-2-yl)piperidin-3-yl)methanolHCl salt compound* impaired MB Group 3 cancer progression at a lower dose when compared to AA7.1 (i.e., 6 mg/kg/day vs 60 mg/kg/every two days; Ferrucci et al., 2018).

Of note, *(S)-(1-(4-((4-methoxybenzyl)amino)-1,3,5-triazin-2-yl)piperidin-3-yl)methanol HCl salt compound* did not show any signs of toxicity in these treated mice, as demonstrated by no decrease in total body weight in the treated mice compared to controls (**Figure 2D**).

Immunoblotting analyses of the cerebellar tumours in these mice treated with (*S)-(1-(4-((4-methoxybenzyl)amino)-1,3,5-triazin-2-yl)piperidin-3-yl)methanol HCl salt compound* in vivo showed reduced PRUNE1 levels, inhibition of nuclear phosphorylated SMAD2/3 and OTX2, and overexpression of activated PTEN. In the same tissues, the treated mice also showed inhibition of EMT with N-cadherin loss and increased E-cadherin (**Figure 2E).**

**Example 3**

***(S)-(1-(4-((4-methoxybenzyl)amino)-1,3,5-triazin-2-yl)piperidin-3-yl)methanol HCl salt compound* is able to inhibit Prune1-driven metastatic axis in a metastatic Medulloblastoma Group 3 genetically engineered mouse model (GEMM).**

We tested the ability of *(S)-(1-(4-((4-methoxybenzyl)amino)-1,3,5-triazin-2-yl)piperidin-3-yl)methanol HCl salt compound* to inhibit tumorigenesis in a GEMM of metastatic Medulloblastoma Group 3. To this aim, we have recently generated a GEMM of Medulloblastoma driven by human PRUNE1 by using a construct plasmid characterized by human Prune 1 cDNA fused to FLAG sequence under the control of MATH1 promoter, in order to overexpress human Prune1 during developing cerebellum (i.e., P0-P4; Lumpkin et al., 2003). The same DNA construct also has cloned the Firefly Luciferase (Luc) gene under the control of Cyclin B2 promoter (**Figure 3A**), whose expression is mainly found in proliferating cells during G2/ M transition (Wu et al., 2010). The resulting GEMM overexpressing Prune1 in the developing cerebellum (MATH1-Prune1) developed MB and metastasis in the spinal cord in a background TP53 null (i.e., MATH1-Prune1/ TP53-/- ; in FVB strain) (**Figure 3B**).

This GEMM was characterized in terms of metastatic axis driven by Prune1 (Ferrucci et al., 2018) and MB molecular subgroups. To this aim, qPCR analyses were performed on primary tumors generated by MATH1-Prune1/ TP53-/- to determine the expression levels of the main genes markers of molecular subgroup of metastatic MB (i.e., SHH, Group 3 and Group 4; as described by Northcott et al., 2012; Taylor et al., 2012; Cavalli et al., 2017). Our data show increased levels of Prune1, OTX2 and reduced PTEN mRNA in those tumors generated by the GEMM as compared to normal cerebellum belonging to FVB and a mouse model of SHH molecular subgroup driven by PTC and TP53 mutations (**Figure 3C**). This, thus, is suggesting that Prune1 is able to drive the metastatic axis (Ferrucci et al., 2018) also in this mouse model (**Figure 3C**). Furthermore, the qPCR analyses performed on those genes whose expression were found altered in the different molecular subgroups of MB, show increased expression of 2 out of 4 genes belonging to Group 3 (i.e., NPR3 and GABRA3), 2 out of 4 genes belonging to Group SHH (i.e., SFRP1 and HHIP, mostly due to the overexpression of MATH1) and none of the genes marker of Group 4 (**Figure 3C**). These results suggest that the

tumors generated by MATH1-Prune1/ TP53-/- are characterized by increased expression of those genes belonging to Group 3 and SHH, probably due to the increased expression of MATH1).

Furthermore, the half maximal inhibitory concentration (IC50) of *(S)-(1-(4-((4-methoxybenzyl)amino)-1,3,5-triazin-2-yl)piperidin-3-yl)methanol HCl salt compound* for the inhibition of cell proliferation index was measured in primary cells obtained from the tumors generated by MATH1-Prune1/ TP53-/- GEMM, as 31.1 μM (R = 0.97; **Figure 3D**).

The same cells were then treated with 10 μM *(S)-(1-(4-((4-methoxybenzyl)amino)-1,3,5-triazin-2-yl)piperidin-3-yl)methanol HCl salt compound* for 24 hours, which resulted in a statistically significant decrease of human and murine endogenous Prune1 mRNA (0.15 and 0.4 fold, respectively **Figure 1E**). Immunoblotting analyses of MATH1-Prune1/ TP53-/- cells treated with 10 μM (*S)-(1-(4-((4-methoxybenzyl)amino)-1,3,5-triazin-2-yl)piperidin-3-yl)methanol HCl salt compound* for 24 hours showed reduced human and murine Prune1 levels, inhibition of nuclear phosphorylated SMAD2 and OTX2. This thus indicate that *(S)-(1-(4-((4-methoxybenzyl)amino)-1,3,5-triazin-2-yl)piperidin-3-yl)methanol HCl salt compound* can inhibit the metastatic axis also in this GEMM of metastatic MB driven by Prune1.

**Example 4**

***(S)-(1-(4-((4-methoxybenzyl)amino)-1,3,5-triazin-2-yl)piperidin-3-yl)methanol HCl salt* exerts immunomodulatory actions in vitro**

Since Prune1 has been recently reported to be involved in the communication between tumorigenic and immune cells in the tumor microenvironment of TNBC, and its pharmacological inhibition (via AA7.1) could impair this crosstalk, we investigated the ability of (*S)-(1-(4-((4-methoxybenzyl)amino)-1,3,5-triazin-2-yl)piperidin-3-yl)methanol HCl salt compound* in inhibiting the expression of those cytokines already reported to be targeted by immunomodulatory drug (i.e. IMID, e.g., Pomalidomide currently tested in phase 2 trial for recurrent MB; Fangusaro J et al., 2021). To this aim, primary cells obtained from the tumors generated by MATH1-Prune1/ TP53-/- GEMM were treated with 10 μM (*S)-(1-(4-((4-methoxybenzyl)amino)-1,3,5-triazin-2-yl)piperidin-3-yl)methanol HCl salt compound* for 24 hours. qPCR analyses showed decreased levels of those inflammatory cytokines targeted by IMIDs drugs (**Figure 4A**), thus suggesting that this new molecule has immunomodulatory action in vitro. Of interest, among this genes we found MCP1 (or CCL2) chemokine whose levels were found correlated to leptomeningeal metastases in models of metastatic MB (Garzia et al., 2018), thus suggesting a potential for (*S)-(1-(4-((4-methoxybenzyl)amino)-1,3,5-triazin-2-yl)piperidin-3-yl)methanol HCl salt compound* to inhibit metastatic dissemination of MB.

To gain insight into the immunomodulatory action of the new molecule, we performed a cytokine array to measure the secretion level of n.61 cytokines/ chemokines in the conditioned media collected from MATH1-Prune1/ TP53-/- cells treated for 24 hours with 10 μM *(S)-(1-(4-((4-methoxybenzyl)amino)-1,3,5-triazin-2-yl)piperidin-3-yl)methanol HCl salt compound* or water as vehicle control (**Figure 4B**). Among these soluble molecules, 10 cytokines/ chemokines were found statistically significantly downregulated in the conditioned media upon treatment with (*S)-(1-(4-((4-methoxybenzyl)amino)-1,3,5-triazin-2-yl)piperidin-3-yl)methanol HCl salt compound,* while n.6 cytokines/ chemokines were found upregulated (**Figure 4C-D**). Of importance, those significantly downregulated cytokines were found to take part into a protein-protein interaction network (generated via Search Tool for Retrieval of Interacting Genes/Proteins (STRING) database) whose function are mostly involved in leucocytes chemotaxis, positive activation of leucocytes migration (including lymphocytes chemotaxis) and positive activation of T cells (**Figure 4E**).

**Example 5**

***(S)-(1-(4-((4-methoxybenzyl)amino)-1,3,5-triazin-2-yl)piperidin-3-yl)methanol HCl salt* can lower the conversion of Conventional toward Regulatory T cells (i.e., Tconv→ Treg) whose presence are found in the tumor microenvironment of MB Group 3 -4 affected patients**

Since *(S)-(1-(4-((4-methoxybenzyl)amino)-1,3,5-triazin-2-yl)piperidin-3-yl)methanol HCl salt compound* has been found with the ability to inhibit inflammatory cytokines/ chemokines whose functions is mainly involved in positive activation and recruitment of T cells, we investigated the presence of immunosuppressive T lymphocytes (i.e., PD1+FOXP3+CD4+ and PD1+FOXP3+CD8+) in the tumor microenvironment by using primary paraffin embedded tumor sections obtained from n.14 children affected by MB Group 3 - 4. Our immunofluorescence analyses, show a low percentage of immunosuppressive T cells (i.e., PD1+FOXP3+CD4+ and PD1+FOXP3+CD8+) **(Figure** 5A). Furthermore, the presence of immunosuppressive regulatory T cells (Tregs; CD4+FOXP3+) was also found in the tumor microenvironment of MATH1-Prune1/TP53-/- model (**Figure 5B**).

Altogether, these data show the presence of immunosuppressive lymphocytes in the tumor microenvironment of MB Group 3 - 4 of human or murine origin.

Because of the action of *(S)-(1-(4-((4-methoxybenzyl)amino)-1,3,5-triazin-2-yl)piperidin-3-yl)methanol HCl salt*

*compound* against cytokines/ chemokines mainly involved in chemotaxis and activation of T cells, we evaluated its potential to inhibit the conversion between conventional and regulatory T cells. To this aim, we isolated CD4+CD8+ T cells from the spleen of naive FVB mouse and cultured them for 48 hours in the conditioned media collected from MATH1-Prune1/TP53-/- cells previously treated with 10 μM *(S)-(1-(4-((4-methoxybenzyl)amino)-1,3,5-triazin-2-yl)piperidin-3-yl)methanol HCl salt compound* for 24 hours (**Figures 6A-B**) or water as vehicle control. Untreated T cells were used as further control. High resolution immunofluorescence analyses were performed with antibodies against CD4 and nuclear FOXP3 protein. Our data show increased percentage of CD4+FOXP3+ (i.e., Tregs) among those lymphocytes grown in the conditioned media collected from MATH1-Prune1/TP53-/- cells, compared to untreated lymphocytes, thus demonstrating the ability of MB cells to promote the conversion from Tconv to Tregs **(Figures 6C**). In contrast, a decrease in the percentage of Tregs were shown among those T cells grown in the conditioned media collected from MATH1-Prune1/TP53-/- cells treated with 10 μM (*S)-(1-(4-((4-methoxybenzyljamino)-1,3,5-triazin-2-yl)piperidin-3-yl)methanol HCl salt compound* for 24 hours.

Altogether, these results demonstrate the ability of (*S)-(1-(4-((4-methoxybenzyl)amino)-1,3,5-triazin-2-yl)piperidin-3-yl)methanol HCl salt compound* to impair the release of those cytokines in MB cells that are involved in the conversion of T cells from Tconv to immunosuppressive Tregs in vitro.

**Example 6**

***(S)-(1-(4-((4-methoxybenzyl)amino)-1,3,5-triazin-2-yl)piperidin-3-yl)methanol HCl salt* inhibits MB progression in vivo by targeting tumor microenvironment**

The modulation of MB growth and metastases by *(S)-(1-(4-((4-methoxybenzyl)amino)-1,3, 5-triazin-2-yl)piperidin-3-yl)methanol HCl salt compound* was then investigated in vivo using orthotopic syngenic immunocompetent mouse models of MB Group3.

In this in vivo preclinical trial, MATH1-Prune1/TP53-/- cells ($1.5 \times 10^5$ cells) were implanted into the fourth ventricle of nude mice (n=10). After 10 days from tumour implantation (i.e., once the tumours were established) the mice (grouped according to their bioluminescence values) were injected intraperitoneally with (*S)-(1-(4-((4-methoxybenzyl)amino)-1,3,5-triazin-2-yl)piperidin-3-yl)methanol HCl salt compound* at 6 mg/kg/day, or with water as the vehicle for the control group for further 7 days (**Figures 7A, B**). These data showed significantly decreased of intracranial tumor growth after 2 weeks of treatment (**Figure 7C**). Furthermore, a decreased intensity of BLI signal from the spinal cord of the treated mice was observed as compared to the control group, thus confirming the antimetastatic action of (*S)-(1-(4-((4-methoxybenzyljamino)-1,3,5-triazin-2-yl)piperidin-3-yl)methanol HCl salt compound* (**Figures 7B, C**).

Of interest, the mice treated with (*S)-(1-(4-((4-methoxybenzyl)amino)-1,3,5-triazin-2-yl)piperidin-3-yl)methanol HCl salt compound* also showed decreased levels of sera circulating CCL2, thus also suggesting an anti-metastatic action (**Figures 7D).**

Finally, quantitative immunofluorescence analyses of the cerebellar tumor sections in these mice treated with (*S)-(1-(4-((4-methoxybenzyl)amino)-1,3,5-triazin-2-yl)piperidin-3-yl)methanol HCl salt compound* in vivo showed reduced immunosuppressive T cells (CD4+PD1+FOXP3+ and CD8+PD1+FOXP3+) in the tumor microenvironment (**Figure 7E**).

Altogether, these data show the ability of (*S)-(1-(4-((4-methoxybenzy)jamino)-1,3,5-triazin-2-yl)piperidin-3-yl)methanol HCl salt compound* to inhibit MB progression, to reduce the metastatic dissemination by targeting the tumor microenvironment via CCL2 inhibition and immunosuppressive T cells reduction.

Finally, the ability of (*S)-(1-(4-((4-methoxybenzyl)amino)-1,3,5-triazin-2-yl)piperidin-3-yl)methanol HCl salt compound* to improve survival in MB-bearing immunocompetent mice generated by orthotopically implantation of MATH1-Prune1/TP53-/- cells ($1.5 \times 10^5$ cells). After 10 days from tumour implantation (i.e., once the tumours were established) the mice were injected intraperitoneally with (*S)-(1-(4-((4-methoxybenzyl)amino)-1,3,5-triazin-2-yl)piperidin-3-yl)methanol salt compound* at 6 mg/kg/day for n.38 days (**Figure 8A**). The treated mice showed improved overall survival (**Figure 8B**) with no toxic effects (**Figure 8C**) as compared to control-mice.

**Example 7**

***(S)-(1-(4-((4-methoxybenzyl)amino)-1,3,5-triazin-2-yl)piperidin-3-yl)methanol HCl salt* acts synergistically with *Vincristine* to inhibit MB progression in vitro and in vivo.**

The potential of (*S)-(1-(4-((4-methoxybenzyl)amino)-1,3,5-triazin-2-yl)piperidin-3-yl)methanol HCl salt compound* to act in combinatorial manner with the chemotherapeutics currently used in clinics in the treatment of MB was investigates. We used *Vincristine* because of its use at the dose of 1.5 $mg/m^2$ in the maintenance regimens of MB average risk group after radiotherapy (when applicable), as described by PNET5 protocol.

The half maximal inhibitory concentration (IC50) of *Vincristine* in murine MATH1-Prune1/TP53-/- cells is 35 μM (R=0.9) (**Figure 9A**), thus comparable to *(S)-(1-(4-((4-methoxybenzyl)amino)-1,3,5-triazin-2-yl)piperidin-3-yl)methanol HCl salt compound* (**Figure 3D**). Because of the proapoptotic action of *Vincristine* (that causes mitotic arrest at metaphase), we tested the enzymatic activation of Caspase 3 in MATH1-Prune1/TP53-/- cells upon treatment with a lower dose of *Vincristine* alone or in combination with *(S)-(1-(4-((4-methoxybenzyl)amino)-1,3,5-triazin-2-yl)piperidin-3-yl)methanol HCl salt compound.* Vehicle-treated and Staurosporine-treated cells were used as negative and positive controls, respectively. Our data show activation of Caspase 3 upon treatment with *Vincristine* (0.3 nM and 0.03 nM) (**Figure 9B**). In contrast, the treatment with *(S)-(1-(4-((4-methoxybenzyl)amino)-1,3,5-triazin-2-yl)piperidin-3-yl)methanol HCl salt compound* alone does not induce apoptotic action, while the combination with the lower dose of *Vincristine* (i.e., 0.03 nM) promotes Caspase 3 activation (**Figure 9B**). This thus suggested a potential synergistic behavior by combining the pro-apoptotic effect of *Vincristine* and the anti-proliferative/immunomodulatory action of *(S)-(1-(4-((4-methoxybenzyl)amino)-1,3,5-triazin-2-yl)piperidin-3-yl)methanol HCl salt compound.* We tested this hypothesis in the human MB Group 3 cells, D425-Med. The half maximal inhibitory concentration (IC50) of *Vincristine* in D425-Med cells is 3.86 μM (R = 0.95; **Figure 9C**).

Of importance, the addition of 10 μM *(S)-(1-(4-((4-methoxybenzyl)amino)-1,3,5-triazin-2-yl)piperidin-3-yl)methanol HCl salt compound* to 10 nM or 1 nM *Vincristine* reduces the cell proliferation rate in D425-Med cells (**Figure 9D**), thus indicating a synergistic action.

We then measured the combinatorial treatment with *(S)-(1-(4-((4-methoxybenzyl)amino)-1,3,5-triazin-2-yl)piperidin-3-yl)methanol HCl salt compound* and *Vincristine* in vivo. To this end, D425-Med cells (1 $\times 10^5$ cells) were implanted into the fourth ventricle of nude mice (n=18). After 7 days from tumour implantation (i.e., once the tumours were established) the mice (grouped according to their bioluminescence values) were injected intraperitoneally with 0.3 mg/kg/day Vincristine alone or in combination with 6 mg/kg/day (*S)-(1-(4-((4-methoxybenzyl)amino)-1,3,5-triazin-2-yl)piperidin-3-yl)methanol HCl salt compound* for 18 days (**Figures 9E**). These data showed significantly decreased of intracranial tumor growth in those mice receiving the combinatorial treatment (**Figures 9F, G**).

Of interest, the mice treated with (*S)-(1-(4-((4-methoxybenzyl)amino)-1,3,5-triazin-2-yl)piperidin-3-yl)methanol HCl salt compound* also showed decreased levels of angiogenesis as detected via fluorescent imaging probe (IVISense Integrin Receptor 680 Fluorescent Probe; Perkin Elmer) (**Figure 9H**).

### Example 8

### (S)-(1-(4-((4-methoxybenzyl)amino)-1,3,5-triazin-2-yl)piperidin-3-yl)methanol HCl salt compound acts synergistically with *Seclidemstat [SP-2577]* to inhibit MB proliferation in vitro.

*(S)-(1-(4-((4-methoxybenzyl)amino)-1,3,5-triazin-2-yl)piperidin-3-yl)methanol HCl salt compound* was found to act synergistically with *Seclidemstat [SP-2577],* an epigenetic drug targeting Lsd1 (Lee et al., 2019), that is currently used in clinical trial for several Advanced Solid Tumors (NCT03895684). In this regard, the half maximal inhibitory concentration (IC50) of *Vincristine* in murine MATH1-Prune1/TP53-/- cells is 19,6 μM (R=0.95) (**Figure 10A**) and D425-Med (50 μM R=0.95, **Figure 10B**). Its combination (at the dose of 22.5 μM) together with 10 μM of *(S)-(1-(4-((4-methoxybenzyl)amino)-1,3,5-triazin-2-yl)piperidin-3-yl)methanol HCl salt compound,* enhance the inhibition of cell proliferation in vitro in D425-Med cells (**Figure 10C**). This, thus, suggest new therapeutic regimens for the treatment of MB group 3 based on the combination between *(S)-(1 -(4-((4-methoxybenzyl)amino)-1,3,5-triazin-2-yl)piperidin-3-yl)methanol HCl salt compound* and epigenetics drugs targeting Lsd1.

### Example 9

### *(S)-(1-(4-((4-methoxybenzyl)amino)-1,3,5-triazin-2-yl)piperidin-3-yl)methanol HCl salt* inhibits cell proliferation, cell migrations in TNBC cells and their communication with macrophages

Since the ability of AA7.1 to inhibit TNBC progression by targeting the communication between tumorigenic- and immune- cells has been recently reported (Ferrucci et al., 2021), here we tested the potential of (*S)-(1-(4-((4-methoxybenzyl)amino)-1,3,5-triazin-2-yl)piperidin-3-yl)methanol HCl salt compound* in a cell model of murine metastatic TNBC. In this regard, we used murine primary cells obtained from a GEMM model of metastatic TNBC characterized by the overexpression of both human Prune 1 and Wnt1 in mammary gland under the control of Mouse Mammary Tumor Virus (MMTV; i.e., MMTV-Prune1-Wnt1 cells; Ferrucci et al., 2021). These cells were characterized by higher expression levels of human Prune1 transgene (**Figure 11A**). Our in vitro assays show reduction of the proliferation (**Figure 11B**) and migration (**Figure 11C**) rate in MMTV-Prune1-Wnt1 cells treated with *(S)-(1-(4-((4-methoxybenzyl)amino)-1,3,5-triazin-2-yl)piperidin-3-yl)methanol HCl salt compound* in a dose dependent manner. The ability of the molecule to enhance the degradation of human and murine Prune transcript (**Figure 11D**) and protein (**Figure 11E**) in MMTV-Prune1-Wnt1 cells at the dose of 10 mM was also verified.

Finally, the immunomodulatory action of *(S)-(1-(4-((4-methoxybenzyl)amino)-1,3,5-triazin-2-yl)piperidin-3-yl)methanol HCl salt compound* against the communication between tumorigenic and immune cells was also tested. To this purpose, murine J774.A1 macrophages were grown (after starvation) in the conditioned media collected from MMTV-Prune1-Wnt1 treated with *(S)-(1-(4-((4-methoxybenzyl)amino)-1,3,5-triazin-2-yl)piperidin-3-yl)methanol HCl salt compound* or water as vehicle control (**Figure 11F**). Untreated macrophages were used as further negative control of the assay (**Figure 11F**). Our qPCR analyses showed decreased levels of M2-polarized marker genes in those macrophages grown in the conditioned media collected from MMTV-Prune1-Wnt1 cells previously treated with 10 μM *(S)-(1-(4-((4-methoxybenzyl)amino)-1,3,5-triazin-2-yl)piperidin-3-yl)methanol HCl salt compound* (**Figure 11G**).

These data are indicating that this new anti-Prune1 molecule has the ability to impair TNBC growth and migration and to inhibit the polarization of M2-macrophages in vitro.

**Example 10**

***(S)-(1-(4-((4-methoxybenzyl)amino)-1,3,5-triazin-2-yl)piperidin-3-yl)methanol HCl salt* is not toxic in vivo**

Toxicity studies were carried out with escalating-dose of *(S)-(1-(4-((4-methoxybenzyl)amino)-1,3,5-triazin-2-yl)piperidin-3-yl)methanol HCl salt compound* in vivo in naive FVB mice (4 weeks old; grouped according to weight), as 60 and 120 mg/kg intraperitoneally, administered daily for 12 days (**Figure 12A**). These showed no immediate toxicity effects of *(S)-(1-(4-((4-methoxybenzyl)amino)-1,3,5-triazin-2-yl)piperidin-3-yl)methanol HCl salt compound.* Blood was collected and haematological and biochemical analyses were carried out, along with morphological evaluation of their organs (i.e. spleen, liver, kidneys) (**Figure 12B-D).** There were no significant variations in the haematological parameters (e.g. white blood cell count), and no signs of hepatotoxicity or nephrotoxicity across these three groups of mice, according to glutamate-pyruvate transaminase 1, creatinine blood levels, and blood urea nitrogen (**Figure 12E**).

**Example 11**

**Comparison between *(S)-(1-(4-((4-methoxybenzyl)amino)-1,3,5-triazin-2-yl)piperidin-3-yl)methanol HCl salt* with the compound AA7.1 in terms of target degradation and dose.**

Comparison studies showed similar effect of 100 μM *AA7.1* and 10 μM *(S)-(1-(4-((4-methoxybenzyl)amino)-1,3,5-triazin-2-yl)piperidin-3-yl)methanol salt* in terms of cell proliferation inhibition of MB cells (**Figure 13A**), Prune1 degradation at protein **(Figure 13B)** and mRNA **(Figure 13C)** levels in D425-Med cells. Similar action was shown for the degradation of protein and mRNA levels of the target (Prune1) in murine TNBC cells (i.e., MMTV-Prune1-Wnt1 cells, **Figure 13D, E**).

Furthermore, the ex vivo action of 10 μM *(S)-(1-(4-((4-methoxybenzyl)amino)-1,3,5-triazin-2-yl)piperidin-3-yl)methanol HCl salt* on the inhibition of the conversion from Tconv to Treg cells due to the presence of tumorigenic cells is comparable to those exerted by 100 μM AA7.1 (**Figure 14A**).

Altogether, these in vitro studies, demonstrate that the new compound *(S)-(1-(4-((4-methoxybenzyl)amino)-1,3,5-triazin-2-yl)piperidin-3-yl)methanol HCl salt* is effective against Prune1 degradation and tumor cell proliferation in vitro at a dose 10 fold lower than AA7.1.

Finally, AA7.1 is able to inhibit MB progression in vivo (**Figure 14B-D**) by targeting the immunosuppressive tumor microenvironment (**Figure 14E**) by treating mice at 60 mg/kg/day. In contrast, the new compound *(S)-(1-(4-((4-methoxybenzyl)amino)-1,3,5-triazin-2-yl)piperidin-3-yl)methanol HCl salt* exert anti-tumorigenic action in vivo in the same model at 6 mg/kg/day (**Figure 7A-E**).

**Example 12** - **Materials and Methods**

**1) c-Prune1 (354-453) expression and purification**

$^{15}$N labeled c-Prune1 expression and purification protocols have been performed as previously reported with slight modifications **[Carotenuto et al., 2013].** The purity (95%) of the recombinant c-Prune1 was confirmed by SDS-PAGE and the protein concentration (150 μM for NMR study) was calculated by nanodrop 2000c spectrophotometer.

**2) NMR spectroscopy**

NMR spectra were recorded at 298 K on a Varian Inova 600-MHz spectrometer, with a 5-mm inverse-detection cryoprobe equipped with z-gradient, located at IBB-CNR, Napoli.

For interaction studies, samples (total volume, 600 μL) were prepared in 5-mm NMR tubes, using 1mM of AA7.5 and 10μM of C-prune1 (AA7.5/C-prune1 molar ratio of 100:1) in $H_2O/^2H_2O$ mixture at pH 6.8. STD spectra were acquired with 512 scans with onresonance irradiation at -0.5 ppm for selective saturation of protein resonances and off-resonance irradiation at 30 ppm for reference spectra. A train of 40 Gaussian-shaped pulses of 50 milliseconds with 1-millisecond delay between pulses were used, for a total saturation time of 2 seconds. Subtraction of FID values with on- and off-resonance protein saturation was achieved by phase cycling with a spectral width of 7191.66 Hz, relaxation delay 1.0 s and 8k data points for acquisition and 16k for transformation.

1H and STD spectra of AA7.5 alone were acquired as reference. 1D 1H and STD spectra were processed by means of the ChemAxon software (http://www.chemaxon.com). Relative STD values were calculated by dividing STD signal intensities by the intensities of the corresponding signals in a 1D 1H NMR reference spectrum of the same sample recorded with 512 scans and similarly processed. To map the binding site of AA7.5 on Prune1 surface, $^{15}N$ uniformly labeled c-prune1 (80 μM in 50 mM Tris-HCl, 150 mM NaCl, pH 7) was titrated with increasing amounts of molecule (20, 40, 60, 80, 160, 240 μM). 2D [$^1H$, $^{15}N$] HSQC spectra were acquired with 32 transients per t1 value. Pre-saturation of water was employed during a recycle delay of 1.5 s. 1K complex points were acquired in t2, with an acquisition time of 102.5 ms, while 128 complex points were acquired in t1 with an acquisition time of 64 ms. Backbone $^{15}N$ and HN resonance assignments were obtained on the basis of previously reported assignments **[Carotenuto et al., 2013].** To determine the per residue chemical shift perturbation upon binding and account for differences in spectral widths between $^{15}N$ and $^1H$ resonances, weighted average chemical shift differences, ΔδHN , were calculated for the amide $^{15}N$ and $^1H$ resonances, using equation:

$$\Delta\delta HN= [(\Delta HN)^2 + (0.17x\Delta^{15}N)^2]^{1/2}$$

where ΔH and ΔN are the differences between free and bound chemical shifts. The weighted average chemical shift differences were mapped to the Prune1 structure **[Carotenuto et al., 2013].** using PyMOL graphics program. CARA software was used to generate overlays of the 2D spectra.

**3) Microscale thermophoresis (MST)**

The Microscale thermophoresis (MST) is now a widely used technique to determine the KD or EC50 of protein-ligand interactions. The method exploits the tendency of macromolecules to migrate along a thermal gradient (i.e., thermophoresis). Differences in thermophoresis as a function of a macromolecule's binding state can be measured and assembled into a binding curve that can be analyzed to obtain the Kd. MST was used to evaluate the interaction between c-Prune1 (354-453) and A71.5. MST experiments were performed on a Monolith NT 115 system (Nano Temper Technologies, München, Germany) at 25 °C. 20 μM of c-Prune1 (354-453) protein was labelled with 60 μM of NT-647-NHS reactive dye, the reaction was performed using labelling buffer for 1 h at RT. The binding was performed in binding buffer, 20 mM Tris/HCl, 150 mM NaCl, DTT 1 mM, 0,05 % Tween20, preparing 16-point serial dilution (1:2) of the molecule A71.5 at the final concentration ranged from 250 μM to 0.00763 μM. A71.5 was prepared by first dissolving it in 100% DMSO and then diluting the molecule in binding buffer to obtain a final DMSO concentration of 5%. The samples were filled into Standard capillaries (Nano Temper Technologies) and the measurements were conducted using 20% LED power. An equation implemented by the software MO-S002 MO Affinity Analysis, provided by the manufacturer, was used for fitting normalized fluorescence values at different concentrations of ligands **[Smaldone et al., 2018].** Data points are averages from two independent experiments.

**4) Cell culture**

The triple-negative murine 4T1 Dual LN P3 (referred to as 4T1; ATCC: #CRL-2539) breast-cancer cell line was grown in RPMI 1640 medium (Euroclone) supplemented with 10% (v/v) fetal bovine serum (FBS; Invitrogen), 2 mM L-glutamine (Invitrogen), and 1% (v/v) antibiotics (10000 U/mL penicillin, 10 mg/mL streptomycin [Invitrogen]) (Pen/Strep).

J774A.1 murine macrophages (referred to as J774 cells; ATCC: #TIB-67), MMTV-Prune1-Wnt1 cells were grown in high-glucose Dulbecco's modified Eagle's medium (DMEM; Euroclone) supplemented with 20% (v/v) FBS, 2 mM L-glutamine, and 1% (v/v) antibiotics (Pen/Strep). D425-MED, D425-MED-Luc and MATH-Prune1/TP52-/- cells were grown in high-glucose Dulbecco's modified Eagle's medium (DMEM; Euroclone) and Iscove's Modified Dulbecco's Medium (IMDM; Sigma) at 1:1 (v/v) dilution supplemented with 20% (v/v) FBS, 2 mM L-glutamine, 1% non-essential amino acid solution (M7145; Sigma-Aldrich/Merck), and 1% (v/v) antibiotics (Pen/Strep). The cells were grown at 37 °C in a humidified atmosphere of 95% air, 5% CO2 (v/v).

Primary murine TNBC cells from tumors generated by MMTV-Prune1/Wnt1 mice **[Ferrucci et al., 2021]** and Primary murine Medulloblastoma Group 3 cells from tumors generated MATH-Prune1/TP53-/- mice were obtained (2

months from tumor onset).

**5) LEO-AA7.5(S)T in vitro treatment**

D425.MED and MMTV-Prune1/Wnt1 cells were plated and treated with increasing concentrations of LEO-AA7.5(S)T for 24 hours. Water-treated cells were used as negative control for the experiments.

**6) LEO-AA7.5(S)T in-vivo treatment**

Starting after seven days from tumour implantation (i.e., once the tumours were established) the mice (grouped according to their bioluminescence values) were injected intraperitoneally with LEO-AA7.5(S)T at 6 mg/kg/day, or with water as the vehicle for the control group. After n.28 days from tumour implantation (i.e., after three weeks of AA7.5 treatment), the mice were sacrificed, and the organs were collected.

**7) T cells isolation and culture**

The isolation of untouched mouse CD4+/ CD8+ T cells from single-cell suspensions from spleen of n.2 naive FVB mice were performed by using Pan T Cell Isolation Kit II, mouse (130-095-130, Miltenyi biotec), according to the manufacturer instruction. Once isolated, T cells were grown for 48 hours in the conditioned media previously collected for 24 hours from pooled vehicle- or 10 μM LEO-AA7.5(S)T-treated MATH1-Prune1/TP53-/- cells.

**8) Coculture experiments with macrophages**

The effects of MMTV-Prune-1/Wnt1 cells upon treatment with LEO-AA7.5(S)T were analyzed for M2-polarization of J774A. 1 murine macrophages. Briefly 1 $\times 10^6$ MMTV-Prune-1/Wnt1 cells were resuspended in 10 mL complete DMEM medium, plated into 10-cm-diameter plates, treated with 10 μM LEO-AA7.5(S)T or water as vehicle control, and grown at 37 °C for 24 h. Then the conditioned media were collected. One day before culture in the conditioned media, J774A.1 macrophages were plated into 10-cm-diameter plates. At the time of the culturing in conditioned media, macrophages were at 50% confluence, and after 6 h of starvation in serum-free medium, the macrophages were grown in conditioned media for 48 h. Each experimental point was carried out in triplicate. After the culturing in the conditioned media, the macrophages were washed with PBS, collected, and used for RNA extraction.

**9) Cell proliferation assays (i.e., cell index)**

Cellular proliferation rates were determined with the xCELLigence system (Roche). D425-MED, MATH-Prune1/TP53-/- and MMTV-Prune1/Wnt1 cells ($8 \times 10^3$) were treated with increasing concentrations of LEO-AA7.5(S)T, harvested, and then added to a single well of an xCELLigence E-plate 16. The proliferation rates were determined at 2-min intervals by measuring the impedance changes across the electrodes at the bottoms of the wells. These cell index values were calculated by the xCELLigence RTCA software. with vehicle-treated cells were the negative control. Impedance was measured every 2 min over 24 hours. The IC50 values were calculated through nonlinear regression analysis performed with Graph Pad Prism 9 ([inhibitor] vs. response (three parameters). Data are means ± SD of three independent experiments.

**10) Immunoblotting analysis**

Cells for immunoblotting analysis were washed in cold PBS and lysed in cell lysis buffer [20 mM sodium phosphate (pH 7.4), 150 mM NaCl, 10% (v/v) glycerol, 1% (w/v) sodium deoxycholate, 1% (v/v) Triton X-100] supplemented with protease inhibitors (Roche). The resulting cell lysates were cleared by centrifugation at 16,200g for 10 min at room temperature, and the supernatants were re- moved and assayed for protein concentrations with the Protein Assay Dye Reagent (Bio-Rad). The cell lysates (30 μg) were resolved by SDS-polyacrylamide gel electrophoresis (SDS-PAGE) gels of different percentages, which depended on the molecular weights of the proteins of interest. The proteins were then electrophoretically transferred to polyvinylidene difluoride (PVDF) membranes (Millipore). After 1 hour in blocking solution with 5% (w/v) dry milk fat in PBS or 5% (w/v) BSA (Sigma-Aldrich) in tris-buffered saline [both of which contained 0.02% (v/v) Tween 20] (TBS-Tween), the PVDF mem- branes were incubated with the required primary antibody overnight at 4°C: anti-PRUNE1 (1:250; **Ferrucci et al., 2018),** or anti-β-actin (1:10,000; A5441, Sigma-Aldrich). The membranes were then incubated with the required secondary antibodies for 1 hour at room temperature: secondary mouse or rabbit HRP-conjugated antibodies (NC 15 27606, ImmunoReagents Inc.), diluted in 5% (w/v) BSA in TBS-Tween or in 5% (w/v) milk fat in PBS-Tween, according to the manufacturer’s instructions. The protein bands were

visualized by chemiluminescence detection (Pierce, Thermo Fisher Scientific, IL, USA). Densitometry analysis was performed with ImageJ software. The peak areas of the bands were measured on the densitometry plots, and the relative proportions (percentages) were calculated. Then, the density areas of the peaks were normalized with those of the loading controls, and the ratios for the corresponding controls are presented as fold changes. Immunoblotting was performed in triplicate. Densitometry analyses shown were derived from three independent experiments.

**11) Cytokine antibody array**

The conditioned media collected from three different vehicle- or 10 μM LEO-AA7.5(S)T-treated MATH1-Prune1/TP53-/- cells after 24 hours were pooled. The relative levels of cytokines in the pooled conditioned media and murine sera were measured (RayBio C-Series Mouse Cytokine Antibody Array C3 [AAM-CYT-3-2]), according to the manufacturer protocol. Densitometric analysis was carried out using the Quantity One software (BioRad). Expression levels were normalized to the levels of the positive control spots (contained within the membrane). For each cytokine, the mean ±standard error was determined.

**12) High resolution immunofluorescence**

T cells grown for 48 hours in conditioned media collected from vehicle- or LEO-AA7.5(S)T-treated MATH1-Prune1/TP53-/- cells were fixed in 4% paraformaldehyde in phosphate-buffered saline (PBS) for 30 min, washed three times with PBS, and permeabilized for 15 min with 0.1% Triton X-100 (215680010; Acros Organics) diluted in PBS. The cells were then blocked with 3% bovine serum albumin (A9418; Sigma) in PBS for 1 h at room temperature. The samples were incubated with the appropriate primary antibodies overnight at 4 °C: anti-FOXP3 (1:2000; ab54501; Abcam); anti-CD4 (1:100; sc-19641, SantaCruz). After washing with PBS, the samples were incubated with the secondary antibody at room temperature for 1 h, as anti-mouse Alexa Fluor 488 (1:200; ab 150113; Abcam) or anti-rabbit Alexa Fluor 546 (1:200; Abcam). DNA was stained with DAPI (1:1000; #62254, Thermo Fisher). The slides were washed and mounted with cover slips with 50% glycerol (G5150; Sigma-Aldrich). Microscopy images were obtained using the Elyra 7 platform (Zeiss) with the optical Lattice SIM2 technology (with the ZEN software, Zeiss, blue edition), using the 63× oil immersion objective.

**13) RNA extraction and qPCR assays**

RNA samples were extracted using TRIzol RNA Isolation Reagent (15596026, Invitrogen), according to the manufacturer instructions. Reverse transcription was performed with 5× All-In-One RT MasterMix (g592; ABM), according to the manufacturer instructions. The reverse transcription products (cDNA) were amplified by qRT-PCR using an RT-PCR system (7900; Applied Biosystems, Foster City, CA, USA). The cDNA preparation was through the cycling method, by incubating the complete reaction mix as follows: cDNA reactions: (37 °C for 10 min and 60 °C for 15 min); Heat-inactivation: 95 °C for 3 min; Hold stage: 4 °C. The targets were detected with the SYBR green approach, using BrightGreen BlasTaq 2× PCR MasterMix (G895; ABM). Human ACTB was used as the housekeeping gene to normalize the quantification cycle (Cq) values of the other genes. These runs were performed on a PCR machine (Quantstudio5, Lifetechnologies) with the following thermal protocol:
Hold stage: 50 °C for 2 min; Denaturation Step: 95 °C for 10 min; Denaturation and annealing (×45 cycles): 95 °C for 15 s and 60 °C for 60 s; Melt curve stage: 95 °C for 15 s, 60 °C for 1 min, and 95 °C for 15 s. Relative expression of the target genes was determined using the $2^{-\Delta\Delta Cq}$ method, as the fold increase compared with the controls. The data are presented as means ±SD of the $2^{-\Delta\Delta Cq}$ values (normalized to human ACTB) of three replicates.

**14) Primers sequences for qPCR analyses**

The primers used in the SYBR green assays were:

| | |
|---|---|
| ACTB Forward: GACCCAGATCATGTTTGAGACCTT | (SEQ ID NO: 1) |
| ACTB Reverse: CCAGAGGCGTACAGGGATAGC | (SEQ ID NO:2) |
| IL-1β Forward: ATGATGGCTTATTACAGTGGCAA | (SEQ ID NO:3) |
| IL-1β Reverse: GTCGGAGATTCGTAGCTGGA | (SEQ ID NO:4) |
| m-IL-1β forward, TCAGGCAGGCAGTATCACTCA | (SEQ ID NO:5) |
| m-IL-1β reverse, AGGTCCACGGGAAAGACACA | (SEQ ID NO:6) |
| mTNFα forward, CAGTTCTATGGCCCAGACCC | (SEQ ID NO:7) |

(continued)

| | |
|---|---|
| mTNFα reverse, ACGTGGGCTACAGGCTTGTC | (SEQ ID NO:8) |
| m-IL-12 forward, ATCACACGGGACCAAACCAG | (SEQ ID NO:9) |
| m-IL-12 reverse, CCAGGCAACTCTCGTTCTTGT | (SEQ ID NO:10) |
| m-IL-6 forward, CTTCCAGAGATACAAAGAAATGATGG | (SEQ ID NO:11) |
| m-IL-6 reverse, CTCCAGAAGACCAGAGGAAATTTT | (SEQ ID NO:12) |
| m-IL-10 forward, CCAGTACAGCCGGGAAGACA | (SEQ ID NO:13) |
| m-IL-10 reverse, CGCAGCTCTAGGAGCATGTG | (SEQ ID NO:14) |
| m-β-actin forward, AGGCCAACCGTGAAAAGATG | (SEQ ID NO:15) |
| m-β-actin reverse, GCCTGGATGGCTACGTACATG | (SEQ ID NO:16) |
| m-CCL2 forward AGCATCCACGTGTTGGCTC | (SEQ ID NO:17) |
| m-CCL2 reverse TTGGGATCATCTTGCTGGTG | (SEQ ID NO:18) |
| m.MMP9 forward, TACCCGCTGTATAGCTACCTCGA | (SEQ ID NO:19) |
| m-MMP9 reverse, GCCACGACCATACAGATACTGGA | (SEQ ID NO:20) |
| mIL17F forward: TGAAGTGCACCCGTGAAACA | (SEQ ID NO:21) |
| mIL17F reverse: TCTTCCGAGCTGCTACCTC | (SEQ ID NO:22) |
| mIL28a forward: ACCTCCAACCTGTTTCGCCT | (SEQ ID NO:23) |
| mIL28a reverse: AGGGTTCCAGGTCAGACACACT | (SEQ ID NO:24) |
| m-prune forward, CTGCAGCGGACAGACCTCTT | (SEQ ID NO:25) |
| m-prune reverse, GCAACCAGGGCATCATAGCT | (SEQ ID NO:26) |
| h-prune forward, CTGGCCAACTCACCCTCATC | (SEQ ID NO:27) |
| h-prune reverse, TGCCTCCTCTAGGGCTGTGT | (SEQ ID NO:28) |
| m-CCL2 forward, CAGCAGCAGGTGTCCCAAA | (SEQ ID NO:29) |
| m-CCL2 reverse, TGTCTGGACCCATTCCTTCTTG | (SEQ ID NO:30) |
| m-ARG1 forward CAGAAGAATGGAAGAGTCAG | (SEQ ID NO:31) |
| m-ARG1 Reverse -CAGATATGCAGGGAGTCACC | (SEQ ID NO:32) |
| m-PTEN forward GAGCCATTTCCATCCTGCAG | (SEQ ID NO:33) |
| m- PTEN Reverse - GCCTGTGGCTGAAGAAAAAGG | (SEQ ID NO:34) |
| m-OTX forward TGCGGTATGGACTTGCTGC | (SEQ ID NO:35) |
| m-OTX Reverse - GAAGATGTCTGGGTACCGGGT | (SEQ ID NO:36) |
| mSFRP1 forward, TACCAGCCAAGCCTCTAAGCC | (SEQ ID NO:37) |
| mSFRP1 reverse, TCAATGATGGGCCTCTGACTTCA | (SEQ ID NO:38) |
| mEYA forward, TGGTGTGGAAGAAGAGCAAGG | (SEQ ID NO:39) |
| mEYA reverse, AGTGCCATGAGGTCCGAGTG | (SEQ ID NO:40) |
| mMATH1forword, CGGACCCAGAATACACGCAC | (SEQ ID NO:41) |
| mMATH1reverse, CAAAAGTTGCTCTGCATTGGC | (SEQ ID NO:42) |
| mHHIP forward, TATGACCCAGACTCACAATGGA | (SEQ ID NO:43) |
| mHHIP reverse, GTGACTCTGCATTCCTCAGGC | (SEQ ID NO:44) |
| mNPR3 forward, ACAAGGGCTCAATGAGGAGAT | (SEQ ID NO:45) |
| mNPR3 reverse, AAAGCCAGAACGTAGAGGAGGA | (SEQ ID NO:46) |
| mGABRA5 forward, AGGATGATGGCACACACTTCTCTA | (SEQ ID NO:47) |
| mGABRA5 reverse, CCATTGGAAAGTCCTCAACCT | (SEQ ID NO:48) |
| mIMPG2 forward, GAGGAGGTGGCTGCTTAG | (SEQ ID NO:49) |
| mIMPG2 reverse, TCTGCAACGGTTTCA | (SEQ ID NO:50) |
| mKCNA1 forward, CCCAAAAAATACCCAAGAG | (SEQ ID NO:51) |
| mKCNA1 reverse CCTTTACGCAATTTGGAAACC | (SEQ ID NO:52) |
| mRBM24 forward, TGGTAGGAAGGCCAATGTGAA | (SEQ ID NO:53) |
| mRBM24 reverse, TGAACGCCAAAGGCAAAC | (SEQ ID NO:54) |
| mUNC5D forward, GATCGTCGTGCATTTGC | (SEQ ID NO:55) |
| mUNC5D reverse, GAGTCGATGGGCTCCTCATTT | (SEQ ID NO:56) |
| mKHDRBMS2 forward, TCCTGTGAAACAGTATGGAAG | (SEQ ID NO:57) |

(continued)

| | |
|---|---|
| mKHDRBMS2 reverse, TCTTGTAGCCTCTTCAAGGAG | (SEQ ID NO:58) |
| mOAS1 forward, TGGAGTTTGATTGCTGCCA | (SEQ ID NO:59) |
| mOAS1 reverse, TGGCATAGATTCTTGGATCAGG | (SEQ ID NO:60) |

**15) Caspase assay**

MATH1-Prune1/TP53-/- cells ($3\times10^4$) were plated in 96-well plate and treated with different doses of Vincristine (i.e., 0.3 nM or 0.03 nM) alone or combined to 10 μM LEO-AA7.5(S)T. Vehicle- (0.01% DMSO) treated cells were used as negative controls. Staurosporine-treated cells (1 μM) were used as positive controls of the assays. The assay was performed by using Caspase-3 Activity Assay Kit (#5723, Cell Signaling), by following the manufacture's instruction. Briefly, after 24 hours from the treatment started, cell medium was removed and 30 ul of cell lysis buffer (PI-7018, Cell Signaling) were added. The plate was then incubated for 5 minutes on ice. Later, 25 ul of cell lysate were mixed with 200 ul substrate solution B (#11734S, Cell Signaling) in a black 96 well plate. Fluorescence was detected by using a multimode plate reader (Enspire, 2300, PerkinElmer). The positive control AMC (25 μl, #11735S, Cell Signaling) mixed with 200 ul 1X assay buffer A (#11736S, Cell Signaling) was used as positive control of fluorescence.

**16) Cerebellum orthotopic implantation of D425-MED-Luc in xenograft nude mice.**

D425-MED-luc [**Ferrucci et al., 2018**] were implanted into the right cerebellar hemisphere of female (n.14) athymic nude mice (5 weeks old), as previously described [**Asadzadeh et al., 2018**], to establish orthotopic intracerebellar D425-MED-Luc xenograft models. Nude mice were anaesthetised with Ketamine/ Xylazine (87.5 mg/kg, 12.5 mg/kg, respectively). The mice were then placed in a stereotactic frame by hooking their incisors onto the frame hold. A small skin incision (length, 1 mm) and a burr hole (diameter, 0.7 mm) were created using a microsurgical drill (Fine Science Tools, Foster City, CA, USA). D425-MED-Luc cells ($1\times10^5$)were resuspended in 10 μL PBS and injected slowly through the burr hole into the right cerebellar hemisphere (stereotactic coordinates from Bregma: anteroposterior, 5.5 mm; right lateral, 2.1 mm; dorsoventral 5.0 mm), with a steady force over 30 s, using a 10-AL, 26-gauge Hamilton Gastight model 1701RN syringe needle (SigmaAldrich) that was inserted perpendicular to the cranial surface. The incision was sutured using Safil polyglycolic acid synthetic absorbable suture (6/0). The mice were kept on a warming blanket after the surgery, to help them to maintain their body temperature. Their mobility and respiratory patterns were observed continuously, and once the mice had recovered from the anaesthesia; they were place in a sterile housing cage. The mice were monitored daily for development of potential neurological deficits. Seven days after the implantation of the cells, the mice were imaged, and tumour growth was evaluated by bioluminescence acquisition using an imaging system (IVIS 3D Illumina; Xenogen/ Caliper). For the acquisitions, the mice were anaesthetised by inhalational of isoflurane, and the D-luciferin (122799; PerkinElmer) (15 mg/mL stock) was injected intraperitoneally (100 μL per 10 g body weight). At 5 min from the luciferin injection, the mice were imaged for 3 min. To quantify the bioluminescence, the integrated fluxes of photons (p/s) within each area of interest were determined using the Living Images Software Package 3.2 (Xenogen-Perkin Elmer). For LEO-AA7.5(S)T in-vivo treatment, starting after seven days from tumour implantation (i.e., once the tumours were established) the mice (grouped according to their bioluminescence values) were injected intraperitoneally with LEO-AA7.5(S)T at 6 mg/kg/day, or with water as the vehicle for the control group. After 28 days from tumour implantation (i.e., after three weeks of AA7.5 treatment), the mice were sacrificed, and the organs were collected.

**17) Cerebellum implantation of MATH1-PRUNE1-CICLYN-B2-LUC P53-/-in immunocompetent syngeneic mice model for tumor growth evaluation.**

MATH1-Prune1/ TP53-/- were implanted into the right cerebellar hemisphere of female (n.10) immunocompetent syngeneic mice model (5 weeks old, strain: FVB WT) to establish intracerebellar MATH1-Prune1/ TP53-/- models. Mice were anaesthetised with Ketamine/ Xylazine (87.5 mg/kg, 12.5 mg/kg, respectively). The mice were then placed in a stereotactic frame by hooking their incisors onto the frame hold. A small skin incision (length, 1 mm) and a burr hole (diameter, 0.7 mm) were created using a microsurgical drill (Fine Science Tools, Foster City, CA, USA of MATH1-Prune1/ TP53-/- ($1.5\times10^5$) were resuspended in 10 μL PBS and injected slowly through the burr hole into the right cerebellar hemisphere (stereotactic coordinates from Bregma: anteroposterior, 5.5 mm; right lateral, 2.1 mm; dorsoventral 5.0 mm), with a steady force over 30 s, using a 10-AL, 26-gauge Hamilton Gastight model 1701RN syringe needle (SigmaAldrich) that was inserted perpendicular to the cranial surface. The incision was sutured using Safil polyglycolic acid synthetic absorbable suture (6/0). The mice were kept on a warming blanket after the surgery, to help them to maintain their body temperature. Their mobility and respiratory patterns were observed continuously, and once the mice had recovered from

the anaesthesia; they were place in a sterile housing cage. The mice were monitored daily for development of potential neurological deficits. 10 days after the implantation of the cells, the mice were imaged, and tumour growth was evaluated by bioluminescence acquisition using an imaging system (IVIS 3D Illumina; Xenogen/ Caliper). For the acquisitions, the mice were anaesthetised by inhalational of isoflurane, and the D-luciferin (122799; PerkinElmer) (15 mg/mL stock) was injected intraperitoneally (100 μL per 10 g body weight). At 5 min from the luciferin injection, the mice were imaged for 3 min. To quantify the bioluminescence, the integrated fluxes of photons (p/s) within each area of interest were determined using the Living Images Software Package 3.2 (Xenogen-Perkin Elmer). For LEO-AA7.5(S)T in-vivo treatment, starting after 10 days from tumour implantation (i.e., once the tumours were established) the mice (grouped according to their bioluminescence values) were injected intraperitoneally with LEO-AA7.5(S)T at 6 mg/kg/day, or with water as the vehicle for the control group. After 17 days from tumour implantation (i.e., after one weeks of LEO-AA7.5(S)T treatment), the mice were sacrificed, and the organs were collected for immunohistochemistry and immunofluorescence analyses.

**18) ELISA assay**

ELISA assay (CODICE) was performed by following the manufacturer instruction, to measure the levels of CCL2 cytokine in the sera from immunocompetent syngeneic mice orthotopically implanted with MATH1-Prune1/ TP53-/- cells and treated intraperitoneally for seven days with LEO-AA7.5(S)T (n.5) at 6 mg/kg/day, or with water (n.5) as the vehicle control group.

**19) Cerebellum implantation of MATH1-PRUNE1-CICLYN-B2-LUC P53-/-in immunocompetent syngeneic mice model for survival analysis**

MATH1-Prune1/ TP53-/- were implanted into the right cerebellar hemisphere of female (n.24) immunocompetent syngeneic mice model (5 weeks old, strain: FVB WT). to establish intracerebellar MATH1-Prune1/ TP53-/- models, mice were anaesthetised with Ketamine/ Xylazine (87.5 mg/kg, 12.5 mg/kg, respectively). The mice were then placed in a stereotactic frame by hooking their incisors onto the frame hold. A small skin incision (length, 1 mm) and a burr hole (diameter, 0.7 mm) were created using a microsurgical drill (Fine Science Tools, Foster City, CA, USA of MATH1-Prune1/ TP53-/- (1 × $10^5$) were resuspended in 10 μL PBS and injected slowly through the burr hole into the right cerebellar hemisphere (stereotactic coordinates from Bregma: anteroposterior, 5.5 mm; right lateral, 2.1 mm; dorsoventral 5.0 mm), with a steady force over 30 s, using a 10-AL, 26-gauge Hamilton Gastight model 1701RN syringe needle (SigmaAldrich) that was inserted perpendicular to the cranial surface. The incision was sutured using Safil polyglycolic acid synthetic absorbable suture (6/0). The mice were kept on a warming blanket after the surgery, to help them to maintain their body temperature. Their mobility and respiratory patterns were observed continuously, and once the mice had recovered from the anaesthesia; they were place in a sterile housing cage. The mice were monitored daily for development of potential neurological deficits. For LEO-AA7.5(S)T in-vivo treatment, starting after 10 days from tumour implantation (i.e., once the tumours were established) the mice (grouped according to body weight values) were injected intraperitoneally with LEO-AA7.5(S)T at 6 mg/kg/day, or with water as the vehicle for the control group. mouse body weights were measured at the indicated time points. Mouse survival was closely monitored during the entire experimental period.

**20) In vivo implantation of MMTV-Prune1/Wnt1 cells in immunocompetent syngeneic mice model**

Five-week-old female immunocompetent FVB mice were anesthetized with ketamine/ xylazine (87.5 mg/kg, 12.5 mg/kg, respectively). They were then implanted in the VIII right-side mammary gland with 2.5 × $10^5$ MMTV-Prune1/Wnt1 cells. The tumor growth was evaluated by caliper weekly. For treatment with LEO-AA7.5(S)T in-vivo, starting 14 days from tumor implantation (i.e., once tumors were established), the mice were grouped according to their bioluminescence values and injected intraperitoneally with LEO-AA7.5(S)T 10 mg/kg daily, or with water as the vehicle control. Tumor growth was monitored every 7 days by caliper for each experimental point. At the end of the experiments, the primary tumors were dissected out for protein extraction and/ or embedded in paraffin for immunohistochemistry (IHC) analysis and the sera was collected for CCL2 evaluation via ELISA.

**21) LEO-AA7.5(S)T acute toxicity in vivo**

The toxicity of LEO-AA7.5(S)T was determined with 4-week-old FVB mice grouped according to body weight. Escalating doses of LEO-AA7.5(S)T (60mg or 120mg/kg/day) or vehicle (water) were administered intraperitoneally, daily for 12 days. At sacrifice, the blood and organs were collected and evaluated. Haematological and biochemical markers of hepatic and renal functions were evaluated. Data are expressed as means from four mice within each treatment group.

### 22) Immunofluorescence of paraffin-embedded tissue sections (Mantra Quantitative Pathology Workstation)

Paraffin sections of the tumor specimens (thickness, 3 μm) were deparaffinised and rehydrated by immersing the slides in Xylene Substitute (A5597; Sigma), as three washes for 5 min each, then serially in 100%, 95%, 70%, 50%, and 30% ethanol (two washes for 10 min, for each), and deionised water (two washes, for 5 min each). The washes were then followed by PBS, PBS containing 0.02% Triton-X 100 (215680010; Acros Organics), and PBS (two washes for 5 min, for each). For antigen retrieval, the slides were immersed in boiling 10 mM sodium citrate buffer (pH 6.0) using a microwave oven, and then maintained at sub-boiling temperature for 10 min. The slides were left to cool at room temperature for 30 min. The sections were then washed by immersion in distilled water for 5 min. To block endogenous peroxidase activity, the tissue sections were placed in a solution with 3.0% hydrogen peroxide in methanol for 15 min. Then, to decrease the nonspecific background fluorescence, the tissues were digested by treating them with a solution containing 0.2% trypsin (T2600000; Sigma-Aldrich) and 0.001% CaCl2 for 10 min at 37 °C, in a humidified chamber. The slides were then washed in PBS, PBS containing 0.02% Triton-X 100 (215680010; Acros Organics), and PBS (two washes for 5 min, for each). The tissue sections were blocked with 6% bovine serum albumin (A9418; Sigma), 5% fetal bovine serum (ECS0180L; Euroclone), and 20 mM MgCl2 in PBS containing 0.02% Triton-X 100, for 1 h at room temperature, and incubated overnight with the primary antibodies at 4 °C in a humidified chamber. Tissue sections were washed in PBS and PBS containing 0.02% Triton-X100, and incubated with anti-mouse Alexa Fluor 488 (ab 150113; Abcam), antirabbit Alexa Fluor 488 (150077; Abcam) and antirabbit Alexa Fluor 546 (#A10040; ThermoFisher), as the secondary antibodies. DNA was stained with DRAQ5 (#62254; ThermoFisher). The slides were then washed, dehydrated with 70%, 90%, and 100% ethanol, and mounted with cover slips using 50% glycerol (G5150; Sigma-Aldrich). Confocal microscopy was carried out using Mantra Quantitative Pathology Workstation, using the 40× objective or a laser scanning confocal microscope Leica TCS SP5, using the 63× oil immersion objective. The quantification was performed by using inForm image analysis software.

### References

Bibbò F, Sorice C, Ferrucci V, Zollo M. Functional Genomics of PRUNE1 in Neurodevelopmental Disorders (NDDs) Tied to Medulloblastoma (MB) and Other Tumors. Front Oncol. 2021 Oct 22;11:758146. doi: 10.3389/fonc.2021.758146. PMID: 34745995; PMCID: PMC8569853.

D'Angelo A, Garzia L, Andre A, Carotenuto P, Aglio V, Guardiola O, et al. Prune cAMP Phosphodiesterase Binds Nm23-H1 and Promotes Cancer Metastasis. Cancer Cell (2004) 5(2):137-49. doi: 10.1016/81535-6108(04)00021-2

Tammenkoski M, Koivula K, Cusanelli E, Zollo M, Steegborn C, Baykov AA, et al. Human Metastasis Regulator Protein H-Prune Is a Short-Chain Exopolyphosphatase. Biochemistry (2008) 47(36):9707-13. doi: 10.1021/bi8010847

Carotenuto M, De Antonellis P, Liguori L, Benvenuto G, Magliulo D, Alonzi A, et al. H-Prune Through GSK-3beta Interaction Sustains Canonical WNT/beta-Catenin Signaling Enhancing Cancer Progression in NSCLC. Oncotarget(2014) 5(14):5736-49. doi: 10.18632/oncotarget.2169

Diana D, Smaldone G, De Antonellis P, Pirone L, Carotenuto M, Alonzi A, et al. Mapping Functional Interaction Sites of Human Prune C-Terminal Domain by NMR Spectroscopy in Human Cell Lysates. Chemistry (2013) 19(37):12217-20. doi: 10.1002/chem. 201302168

Zollo M, Ahmed M, Ferrucci V, Salpietro V, Asadzadeh F, Carotenuto M, et al. PRUNE Is Crucial for Normal Brain Development and Mutated in Microcephaly With Neurodevelopmental Impairment. Brain (2017) 140(4):940-52. doi: 10.1093/brain/awx014

Ferrucci V, de Antonellis P, Pennino FP, Asadzadeh F, Virgilio A, Montanaro D, et al. Metastatic Group 3 Medulloblastoma Is Driven by PRUNE1 Targeting NME1-TGF-Beta-0TX2-SNAIL via PTEN Inhibition. Brain (2018) 141(5):1300-19. doi: 10.1093/brain/awy039

Carotenuto, M., Pedone, E., Diana, D. et al. Neuroblastoma tumorigenesis is regulated through the Nm23-H1/h-Prune C-terminal interaction. Sci Rep 3, 1351 (2013). https://doi.org/10.1038/srep01351

Smaldone G, Pirone L, Capolupo A, Vitagliano L, Monti MC, Di Gaetano S, Pedone E. The essential player in adipogenesis GRP78 is a novel KCTD15 interactor. Int J Biol Macromol. 2018 Aug;115:469-475. doi: 10.1016/j.ijbiomac.2018.04.078. Epub 2018 Apr 14. PMID: 29665387.

Ferrucci V, Asadzadeh F, Collina F, Siciliano R, Boccia A, Marrone L, Spano D, Carotenuto M, Chiarolla CM, De Martino D, De Vita G, Macrì A, Dassi L, Vandenbussche J, Marino N, Cantile M, Paolella G, D'Andrea F, di Bonito M, Gevaert K, Zollo M. Prune-1 drives polarization of tumor-associated macrophages (TAMs) within the lung metastatic niche in triple-negative breast cancer. iScience. 2020 Dec 13;24(1):101938. doi: 10.1016/j.isci.2020.101938. PMID: 33426510; PMCID: PMC7779777.

Asadzadeh F, Ferrucci V, DE Antonellis P, Zollo M. In vivo bioluminescence imaging using orthotopic xenografts towards patient's derived-xenograft Medulloblastoma models. Q J Nucl Med Mol Imaging. 2017 Mar;61(1):95-101. doi:

10.23736/S1824-4785.16.02959-9. Epub 2016 Dec 16. PMID: 27982543.

Oue N, Yoshida K, Noguchi T, Sentani K, Kikuchi A, Yasui W. Increased Expression of H-Prune Is Associated With Tumor Progression and Poor Survival in Gastric Cancer. Cancer Sci (2007) 98(8):1198-205. doi: 10.1111/j.1349-7006.2007.00515.x

Noguchi T, Oue N, Wada S, Sentani K, Sakamoto N, Kikuchi A, et al. H-Prune Is an Independent Prognostic Marker for Survival in Esophageal Squamous Cell Carcinoma. Ann Surg Oncol (2009) 16(5):1390-6. doi: 10.1245/s10434-007-9585-3

Nambu J, Kobayashi T, Hashimoto M, Tashiro H, Sugino K, Shimamoto F, et al. H-Prune Affects Anaplastic Thyroid Cancer Invasion and Metastasis. Oncol Rep (2016) 35(6):3445-52. doi: 10.3892/or.2016.4759

Hashimoto M, Kobayashi T, Tashiro H, Arihiro K, Kikuchi A, Ohdan H. H-Prune Is Associated With Poor Prognosis and Epithelial-Mesenchymal Transition in Patients With Colorectal Liver Metastases. Int J Cancer (2016) 139(4):812-23. doi: 10.1002/ijc.30118

D'Angelo A, Garzia L, Andre A, Carotenuto P, Aglio V, Guardiola O, et al. Prune cAMP Phosphodiesterase Binds Nm23-H1 and Promotes Cancer Metastasis. Cancer Cell (2004) 5(2):137-49. doi: 10.1016/S1535-6108(04)00021-2

Zollo M, Andre A, Cossu A, Sini MC, D Angelo A, Marino N, et al. Overexpression of H-Prune in Breast Cancer Is Correlated With Advanced Disease Status. Clin Cancer Res (2005) 11(1):199-205.

Cavalli FMG, Remke M, Rampasek L, Peacock J, Shih DJH, Luu B, et al. Intertumoral Heterogeneity Within Medulloblastoma Subgroups. Cancer Cell (2017) 31(6):737-754 e6. doi: 10.1016/j.ccell.2017.05.005

Goh JY, Feng M, Wang W, Oguz G, Yatim S, Lee PL, et al. Chromosome 1q21.3 Amplification Is a Trackable Biomarker and Actionable Target for Breast Cancer Recurrence. NatMed (2017) 23(11):1319-30. doi: 10.1038/nm.4405

Silva GO, He X, Parker JS, Gatza ML, Carey LA, Hou JP, et al. Cross-Species DNA Copy Number Analyses Identifies Multiple 1q21-Q23 Subtype-Specific Driver Genes for Breast Cancer. Breast Cancer Res Treat (2015) 152(2):347-56. doi: 10.1007/s10549-015-3476-2

Galasso A, Zollo M. The Nm23-H1-H-Prune Complex in Cellular Physiology: A 'Tip of the Iceberg' Protein Network Perspective. Mol Cell Biochem (2009) 329(1-2):149-59. doi: 10.1007/s11010-009-0115-4

Gajjar AJ, Robinson GW. Medulloblastoma-Translating Discoveries From the Bench to the Bedside. Nat Rev Clin Oncol (2014) 11(12):714-22. doi: 10.1038/nrclinonc.2014.181

Hill RM, Kuijper S, Lindsey JC, Petrie K, Schwalbe EC, Barker K, et al. Combined MYC and P53 Defects Emerge at Medulloblastoma Relapse and Define Rapidly Progressive, Therapeutically Targetable Disease. Cancer Cell (2015)

Virgilio A, Spano D, Esposito V, Di Dato V, Citarella G, Marino N, et al. Novel Pyrimidopyrimidine Derivatives for Inhibition of Cellular Proliferation and Motility Induced by H-Prune in Breast Cancer. Eur J Med Chem (2012) 57:41-50. doi: 10.1016/j.ejmech. 2012.08.020

Below J, M Das J. Vincristine. [Updated 2022 Jul 11]. In: StatPearls [Internet]. Treasure Island (FL): StatPearls Publishing; 2022 Jan-. Available from: ht(ps:llwww.ncbi.nlm.nih.govlbooksINBK537122

Lumpkin EA, Collisson T, Parab P, Omer-Abdalla A, Haeberle H, Chen P, Doetzlhofer A, White P, Groves A, Segil N, Johnson JE. Math1-driven GFP expression in the developing nervous system of transgenic mice. Gene Expr Patterns. 2003 Aug;3(4):389-95. doi: 10.1016/s1567-133x(03)00089-9. PMID: 12915300.

Ting Wu‡§, Xiuli Zhang§, Xiaohua Huang§, Yuqing Yang¶, and Xianxin Hua; Regulation of Cyclin B2 Expression and Cell Cycle G2/M Transition by Menin; THEJOURNALOFBIOLOGICALCHEMISTRY VOL.285,NO.24,pp.18291-18300

Northcott PA, Dubuc AM, Pfister S, Taylor MD. Molecular subgroups of medulloblastoma. Expert Rev Neurother. 2012 Jul;12(7):871-84. doi: 10.1586/ern. 12.66 PMID: 22853794; PMCID: PMC4334443

Taylor MD, Northcott PA, Korshunov A, Remke M, Cho YJ, Clifford SC, Eberhart CG, Parsons DW, Rutkowski S, Gajjar A, Ellison DW, Lichter P, Gilbertson RJ, Pomeroy SL, Kool M, Pfister SM. Molecular subgroups of medulloblastoma: the current consensus. Acta Neuropathol. 2012 Apr;123(4):465-72. doi: 10.1007/s00401-011-0922-z. Epub 2011 Dec 2. PMID: 22134537; PMCID: PMC3306779

Fangusaro J, Cefalo MG, Garré ML, Marshall LV, Massimino M, Benettaib B, Biserna N, Poon J, Quan J, Conlin E, Lewandowski J, Simcock M, Jeste N, Hargrave DR, Doz F, Warren KE. Phase 2 Study of Pomalidomide (CC-4047) Monotherapy for Children and Young Adults With Recurrent or Progressive Primary Brain Tumors. Front Oncol. 2021 Jun 8;11:660892. doi: 10.3389/fonc.2021.660892. PMID: 34168987; PMCID: PMC8218626.

Garzia L, Kijima N, Morrissy AS, De Antonellis P, Guerreiro-Stucklin A, Holgado BL, Wu X, Wang X, Parsons M, Zayne K, Manno A, Kuzan-Fischer C, Nor C, Donovan LK, Liu J, Qin L, Garancher A, Liu KW, Mansouri S, Luu B, Thompson YY, Ramaswamy V, Peacock J, Farooq H, Skowron P, Shih DJH, Li A, Ensan S, Robbins CS, Cybulsky M, Mitra S, Ma Y, Moore R, Mungall A, Cho YJ, Weiss WA, Chan JA, Hawkins CE, Massimino M, Jabado N, Zapotocky M, Sumerauer D, Bouffet E, Dirks P, Tabori U, Sorensen PHB, Brastianos PK, Aldape K, Jones SJM, Marra MA, Woodgett JR, Wechsler-Reya RJ, Fults DW, Taylor MD. A Hematogenous Route for Medulloblastoma Leptomeningeal Metastases.

Cell. 2018 Feb 22;172(5):1050-1062.e14. doi: 10.1016/j.cell,2018.01.038. *Erratum in:* Cell. 2018 May 31;173(6):1549. PMID: 29474906; PMCID: PMC6346737

Lee, C., Rudneva, V.A., Erkek, S. et al. Lsdl as a therapeutic target in Gfil-activated medulloblastoma. Nat Commun 10, 332 (2019). https://doi.org/10.1038/s41467-018-08269-5

## Claims

**1.** A compound of formula (I), a salt or enantiomer thereof:

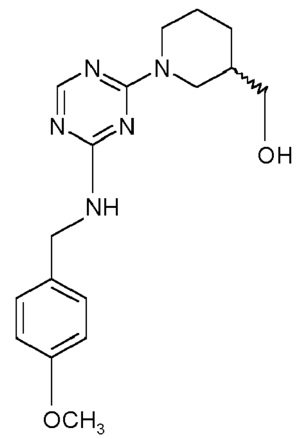

**2.** The compound of formula (I), which is the (S) enantiomer.

**3.** The compound of claims 1-2, which is in the form of hydrochloride salt.

**4.** A pharmaceutical composition comprising the compound of claims 1 and 2, together with pharmaceutically acceptable excipients.

**5.** A pharmaceutical composition according to claim 4, further comprising an anti-tumor compound which is selected from vincristine and seclidemstat.

**6.** A combined preparation containing the compound of formula 1 and an anti-tumor compound selected from vincristine and seclidemstat, for simultaneous or separate administration.

**7.** The compound of claims 1-3, the pharmaceutical composition of claims 4-5 or the combined preparation of claim 6, for use as a medicament.

**8.** The compound of claims 1-3, the pharmaceutical composition of claims 4-5 or the combined preparation of claim 6, for use in the treatment or prevention of Prune1-overexpressing tumor or tumor metastasis.

**9.** The compound of claims 1-3, the pharmaceutical composition of claims 4-5 or the combined preparation of claim 6, for use according to claim 8, wherein said tumor and tumor metastasis are selected from: medulloblastoma, brain cancer, gastric cancer, esophageal squamous cell carcinoma, thyroid cancer, colorectal cancer, neuroblastoma, breast cancer, triple-negative breast cancer.

**10.** The compound of claims 1-3, the pharmaceutical composition of claims 4-5 or the combined preparation of claim 6, for use according to claim 9, wherein said tumor or tumor metastasis is medulloblastoma or breast cancer.

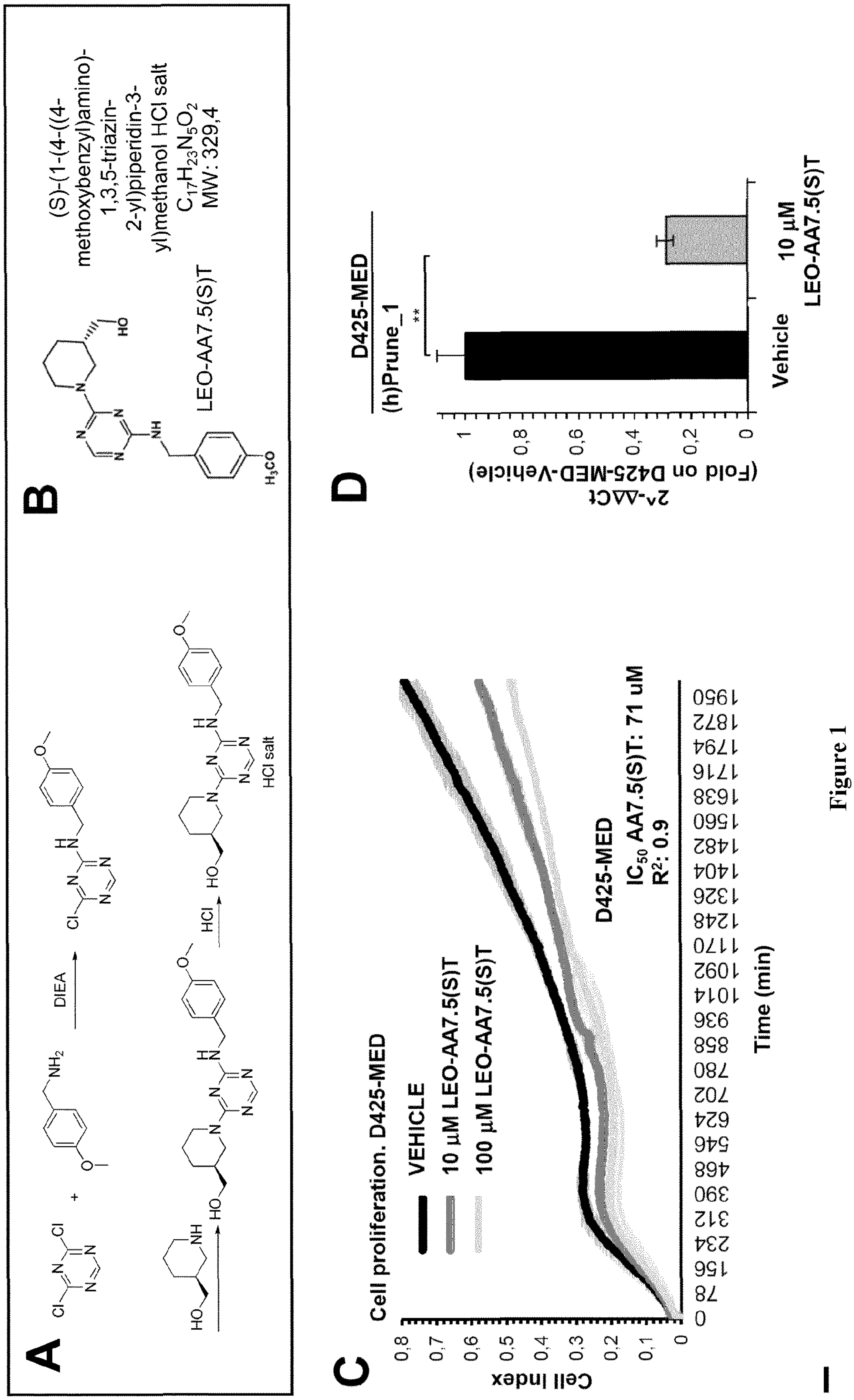
A
DIEA
HCl
HCl salt
B
LEO-AA7.5(S)T
(S)-(1-(4-((4-methoxybenzyl)amino)-1,3,5-triazin-2-yl)piperidin-3-yl)methanol HCl salt
$C_{17}H_{23}N_5O_2$
MW: 329,4
C
Cell proliferation. D425-MED
VEHICLE
10 μM LEO-AA7.5(S)T
100 μM LEO-AA7.5(S)T
D425-MED
$IC_{50}$ AA7.5(S)T: 71 uM
$R^2$: 0.9
Cell Index
Time (min)
D
(h)Prune_1
D425-MED
**
2^-ΔΔCt (Fold on D425-MED-Vehicle)
Vehicle
10 μM LEO-AA7.5(S)T

Figure 1

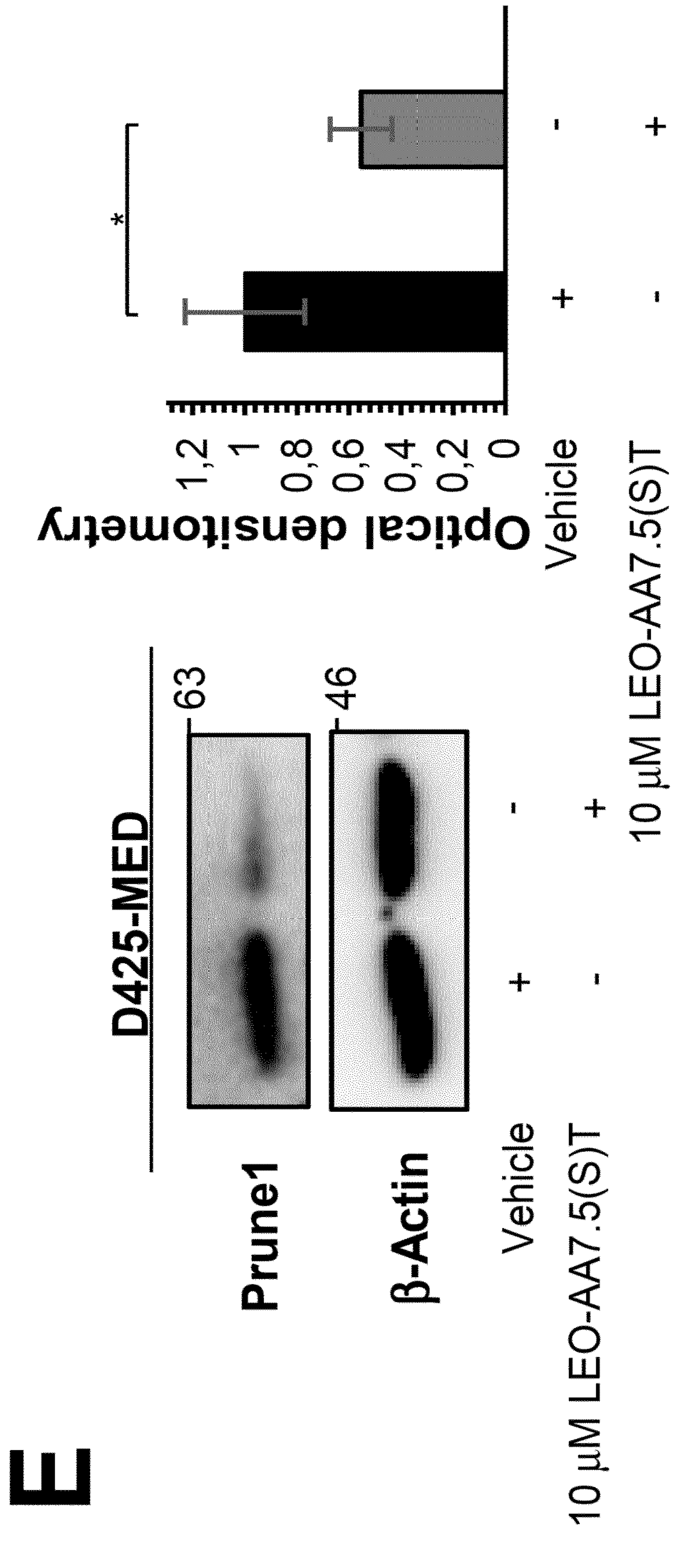


Figure 1 (continued)

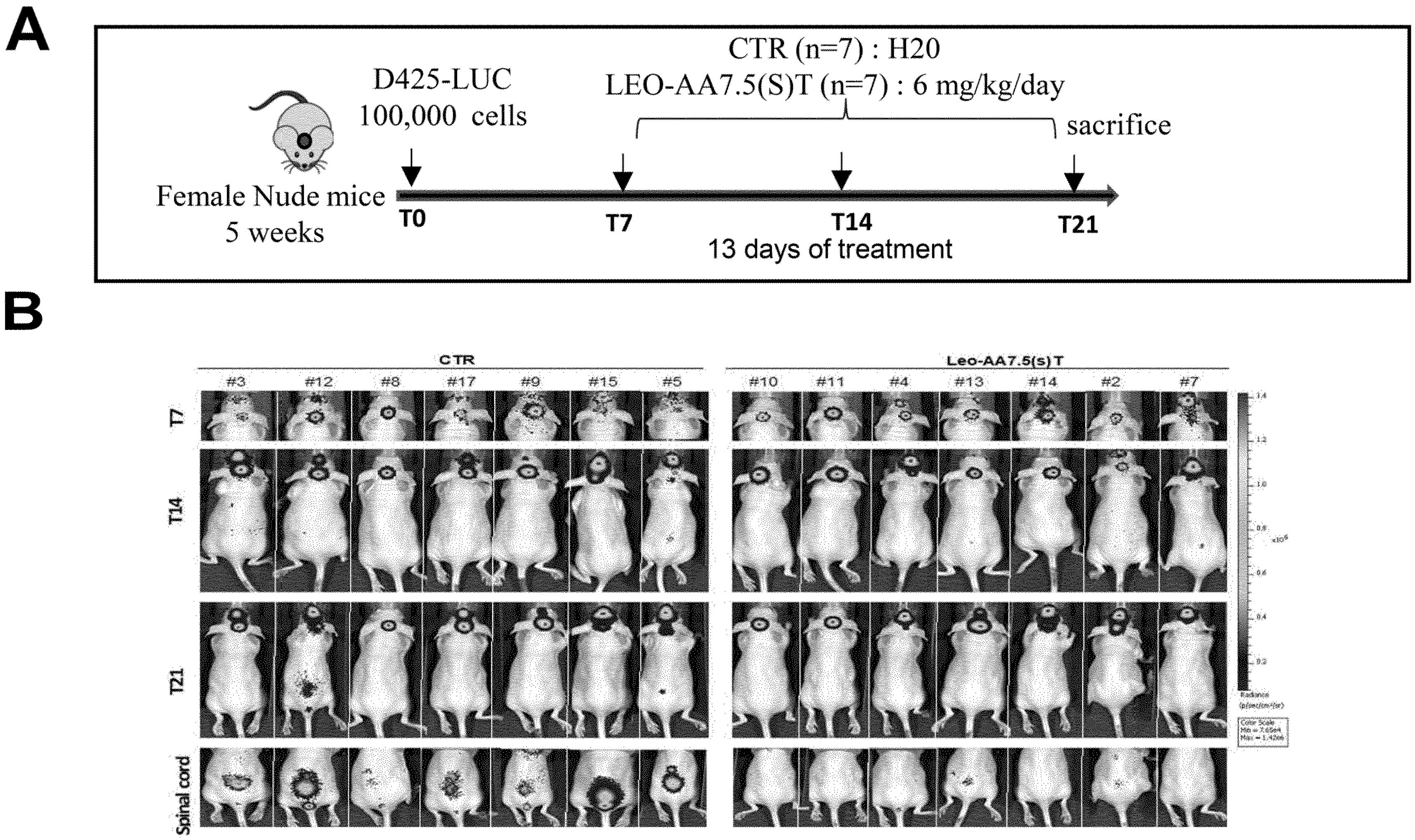
A
D425-LUC
100,000 cells
Female Nude mice
5 weeks
CTR (n=7) : H20
LEO-AA7.5(S)T (n=7) : 6 mg/kg/day
sacrifice
T0
T7
T14
T21
13 days of treatment
B
CTR
Leo-AA7.5(s)T
#3
#12
#8
#17
#9
#15
#5
#10
#11
#4
#13
#14
#2
#7
T7
T14
T21
Spinal cord

Figure 2

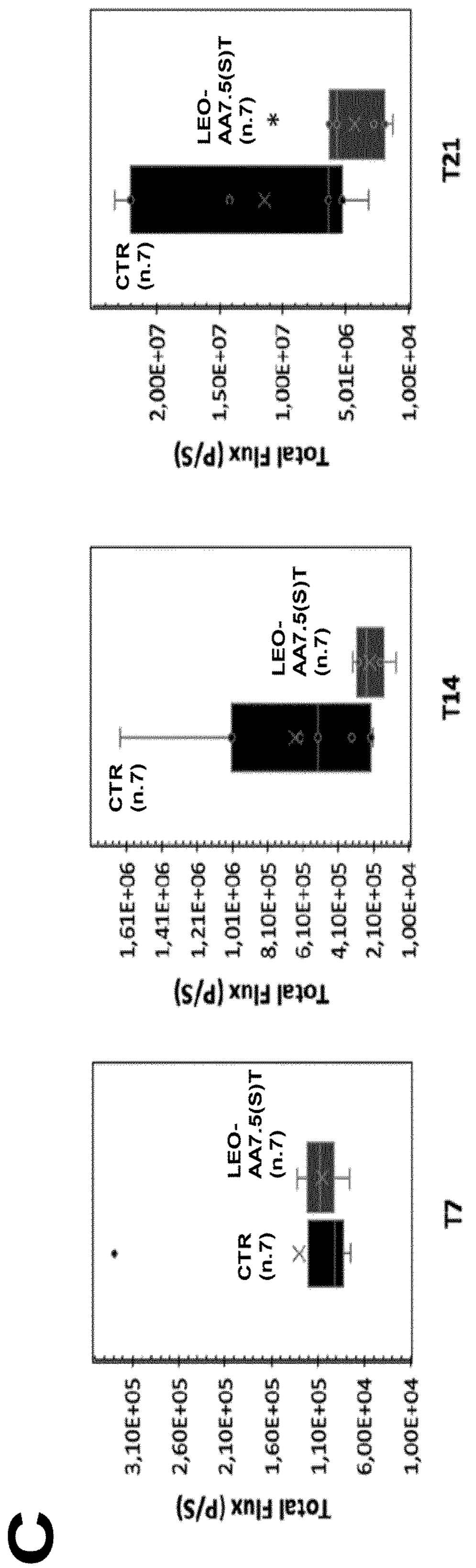


Figure 2 contined

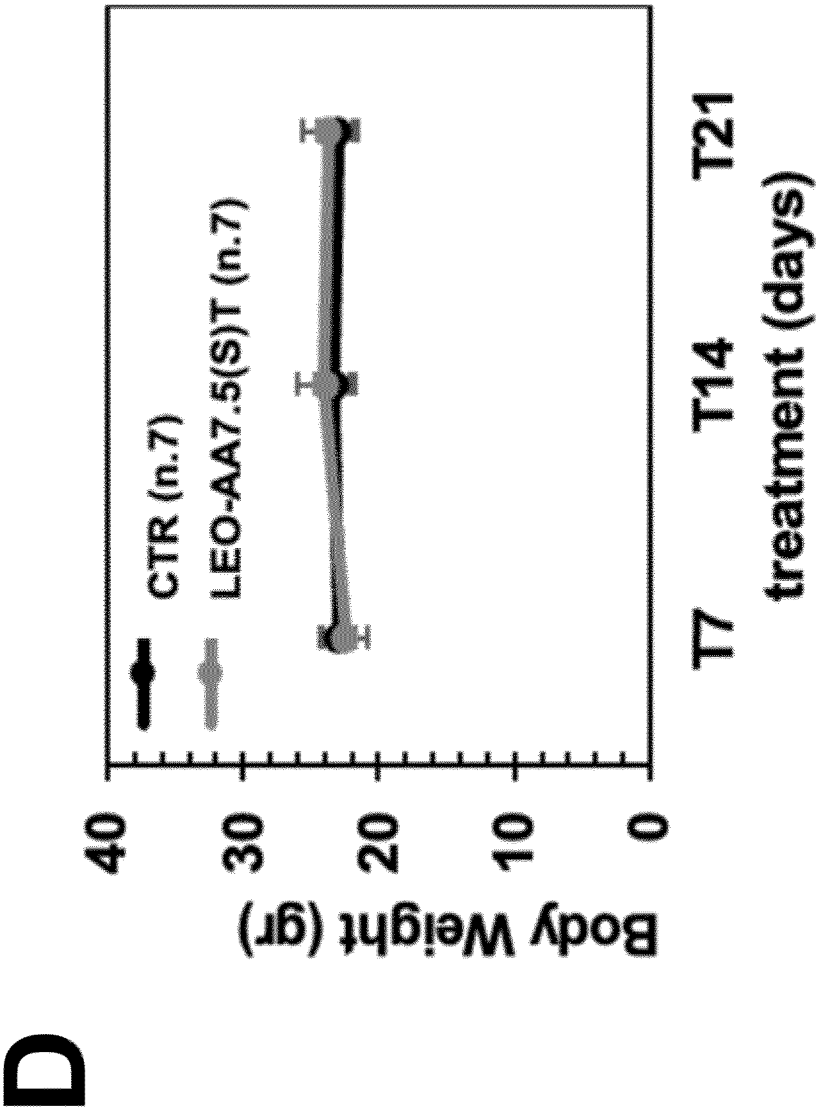


Figure 2 contined

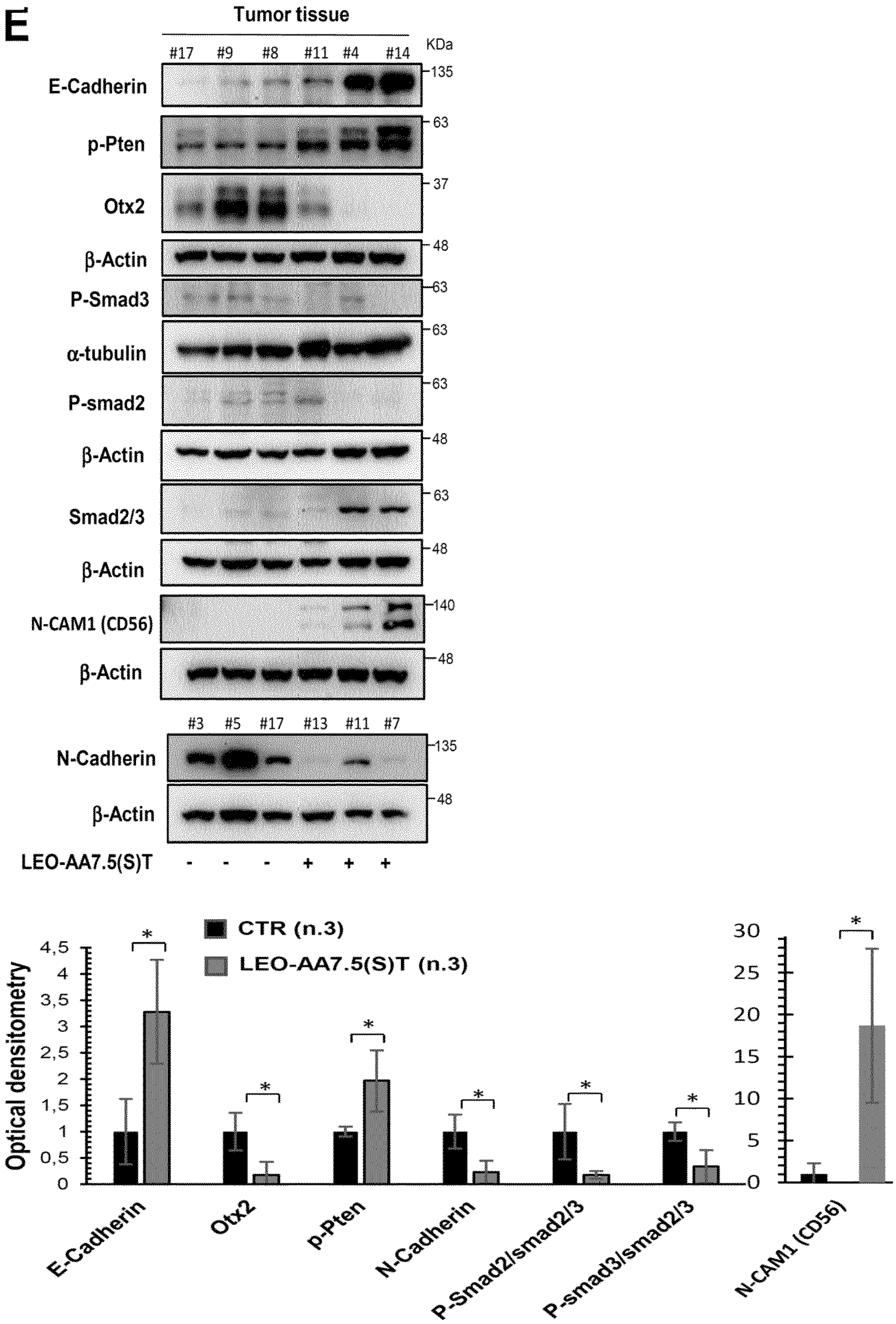


Figure 2 (continued)

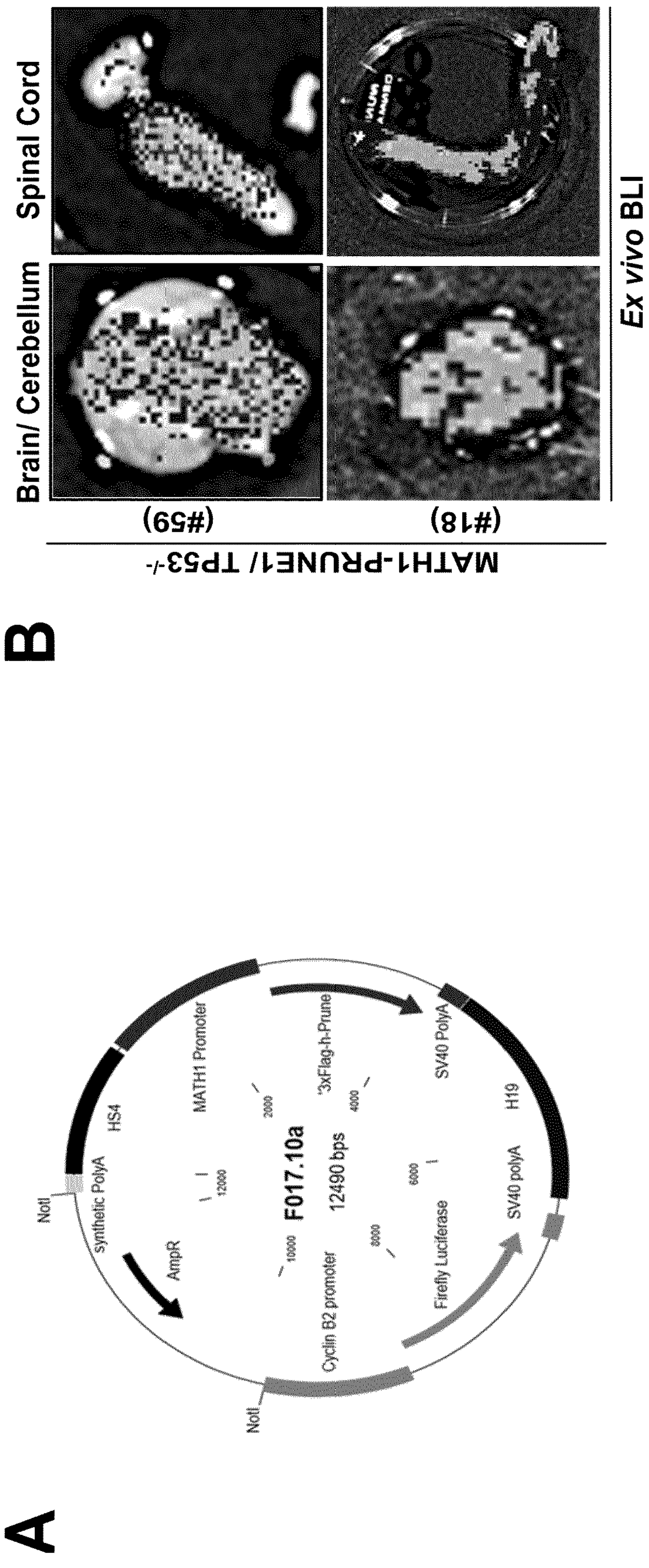
A
NotI
synthetic PolyA
HS4
MATH1 Promoter
'3xFlag-h-Prune
SV40 PolyA
H19
SV40 polyA
Firefly Luciferase
Cyclin B2 promoter
AmpR
NotI
F017.10a
12490 bps
2000
4000
6000
8000
10000
12000
B
Brain/ Cerebellum
Spinal Cord
MATH1-PRUNE1/ TP53-/-
(#59)
(#18)
Ex vivo BLI

Figure 3

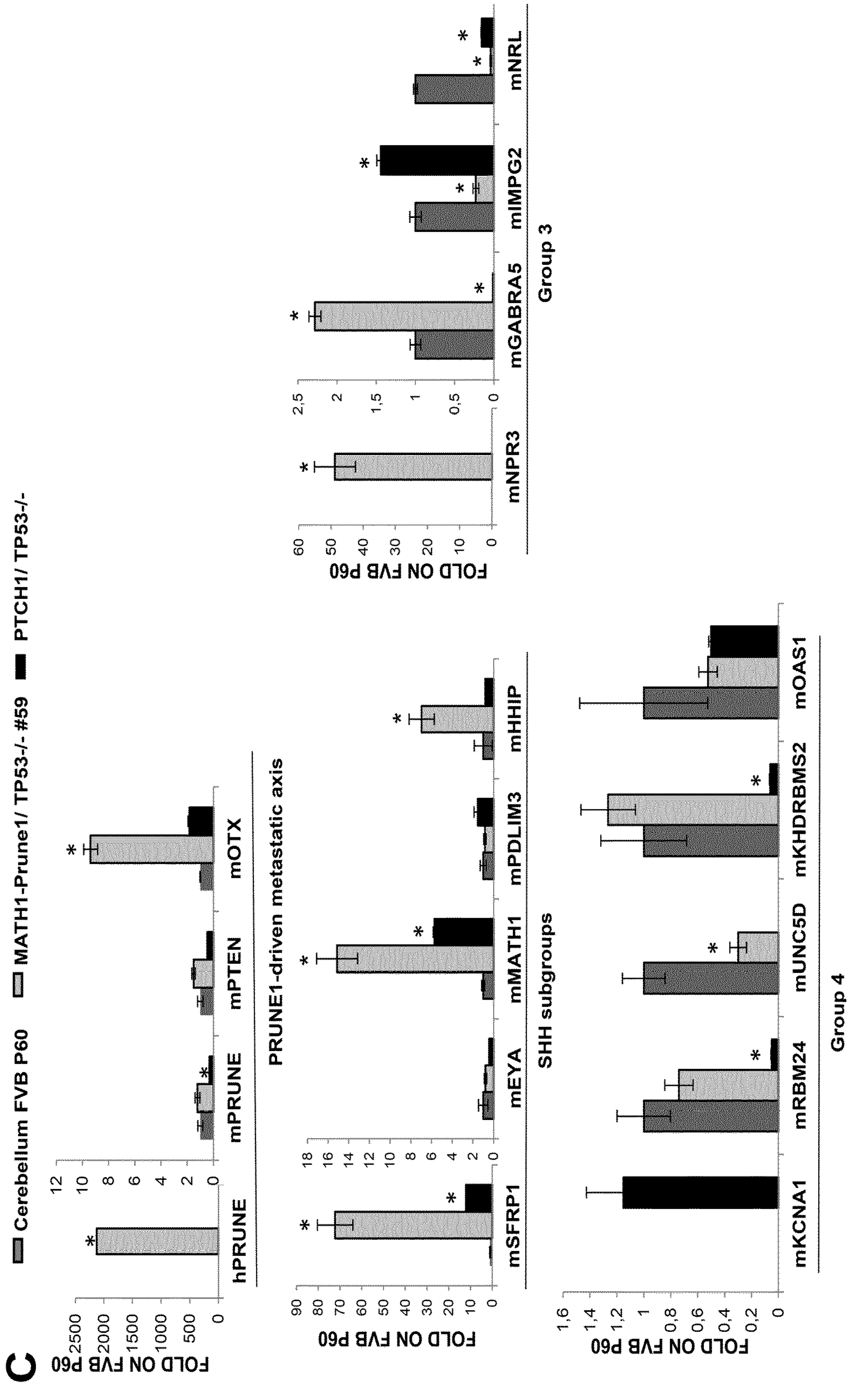


Figure 3 (continued)

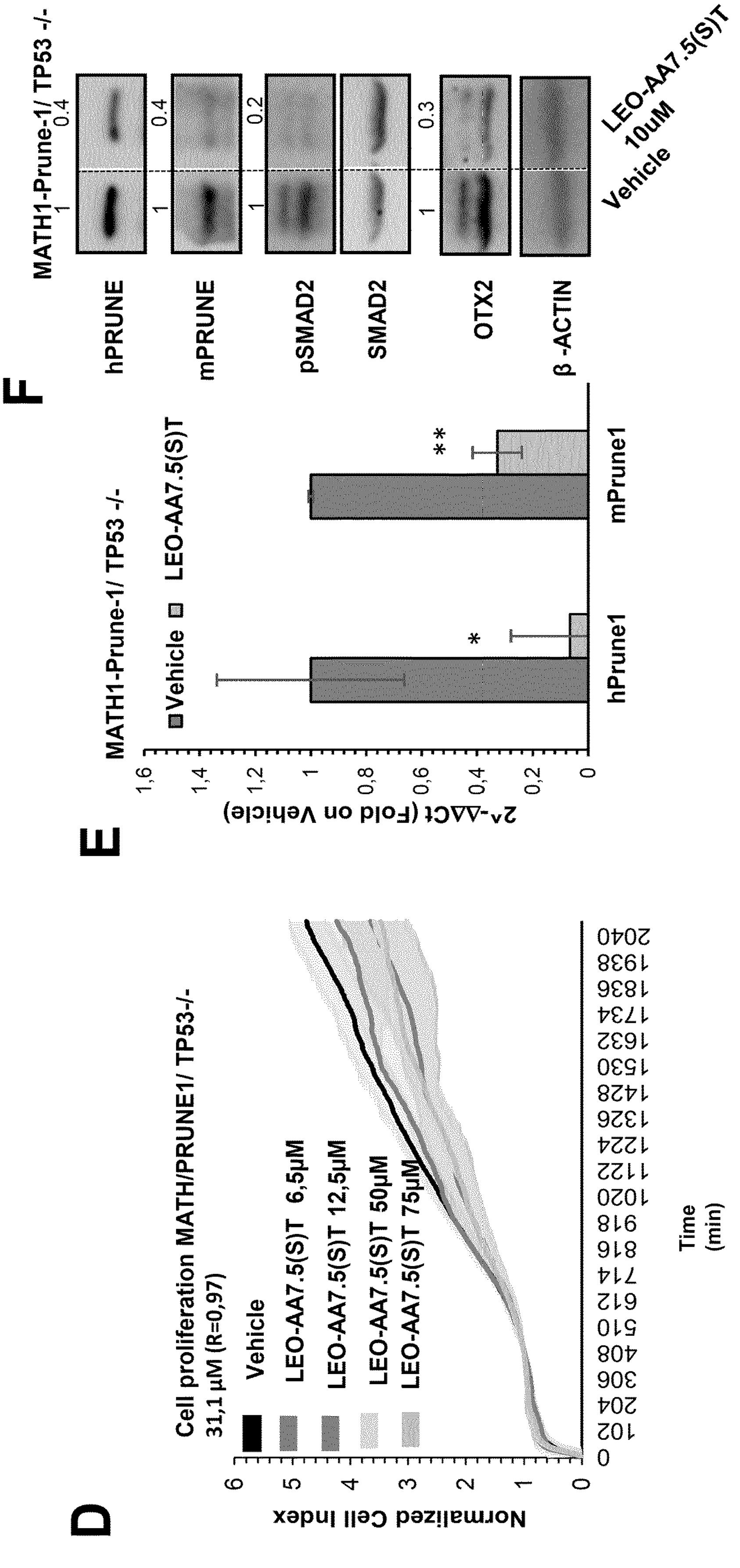


Figure 3 (continued)

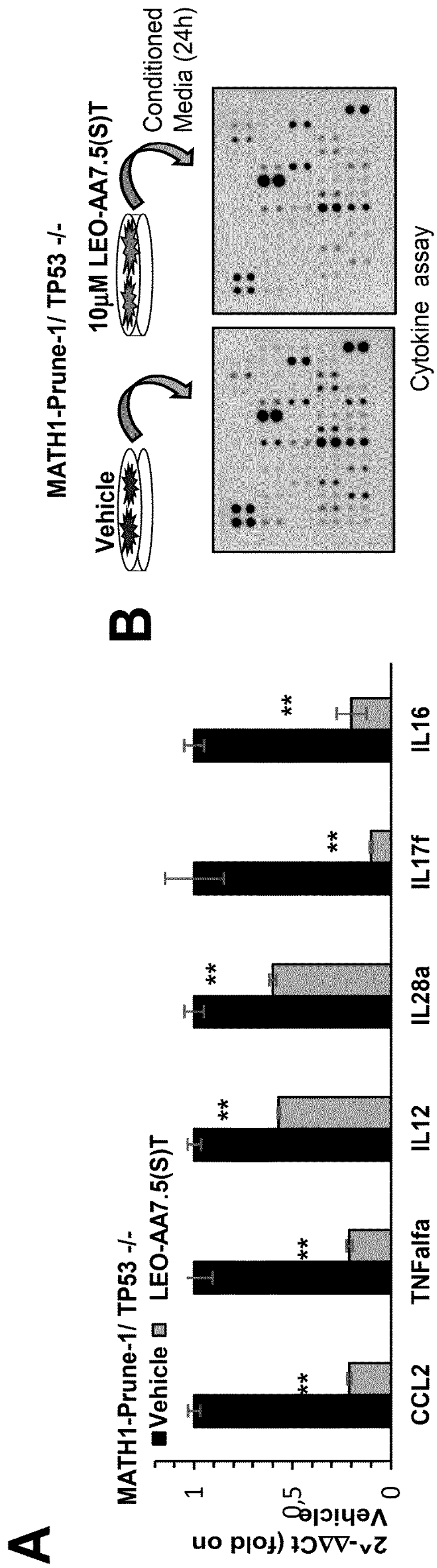
A
MATH1-Prune-1/ TP53 -/-
Vehicle
LEO-AA7.5(S)T
2^-ΔΔCt (fold on Vehicle)
0
0,5
1
**
CCL2
TNFalfa
IL12
IL28a
IL17f
IL16
B
MATH1-Prune-1/ TP53 -/-
Vehicle
10μM LEO-AA7.5(S)T
Conditioned Media (24h)
Cytokine assay

Figure 4

C

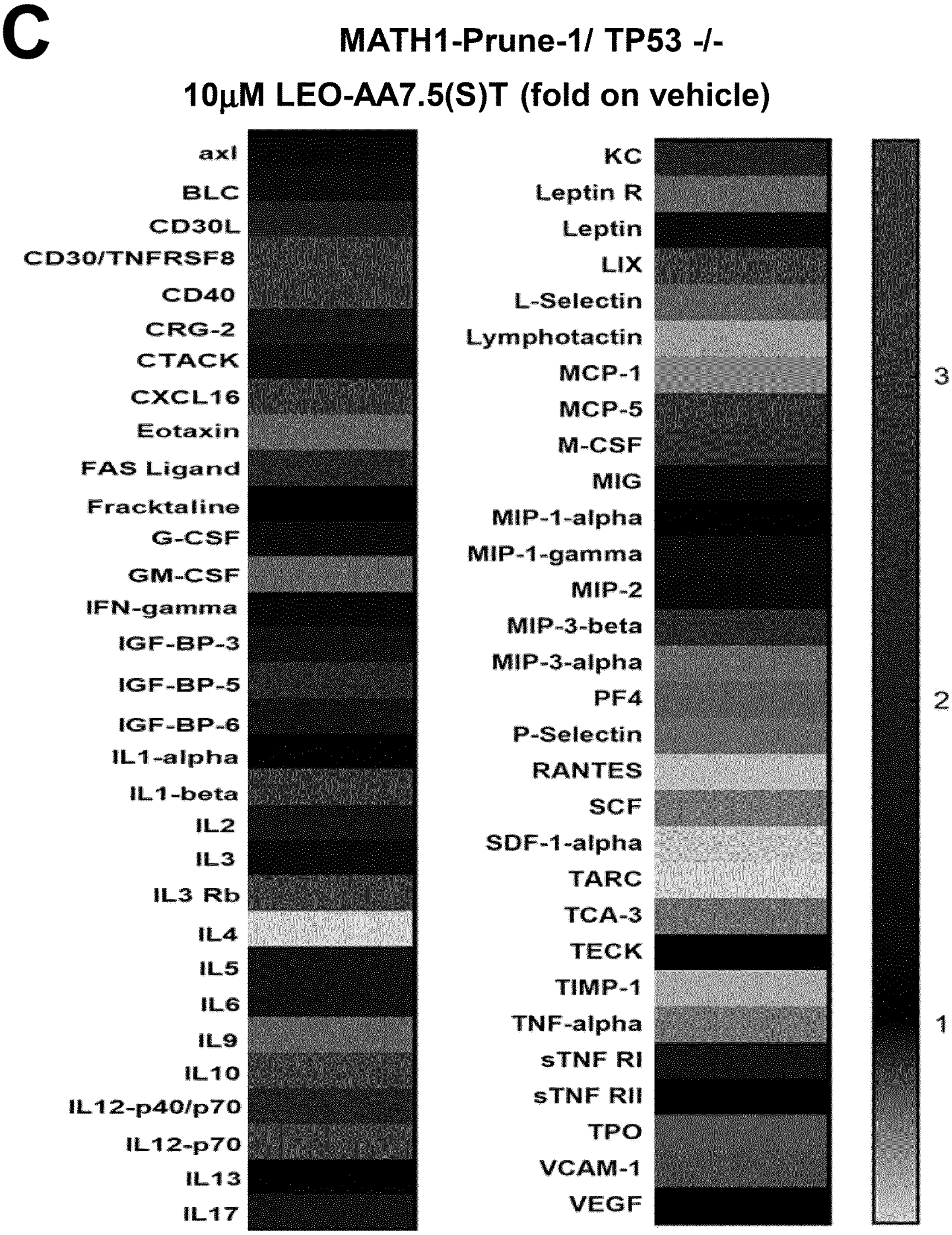


Figure 4 (continued)

E

## Downregulated cytokines

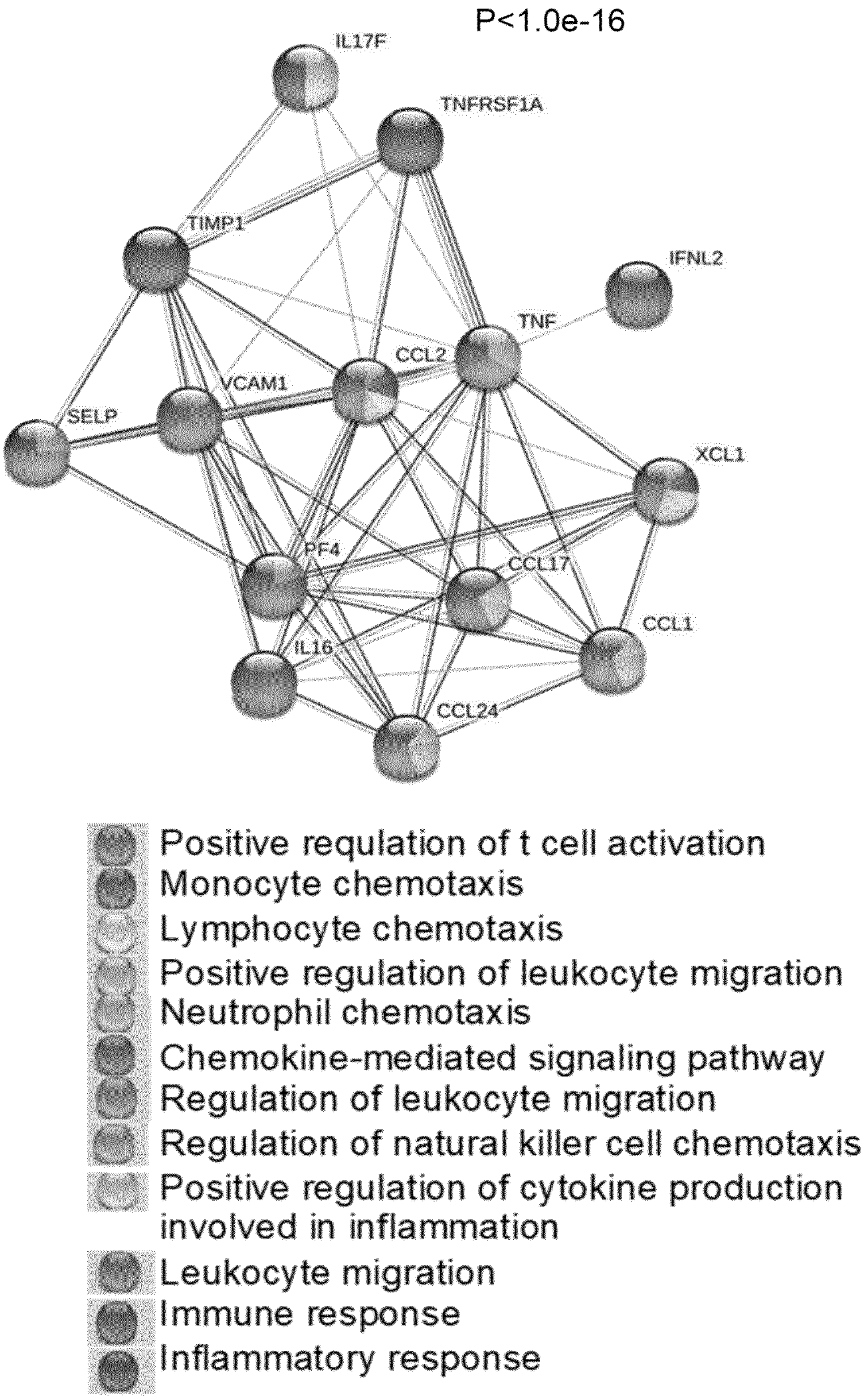


**Figure 4 (continued)**

D

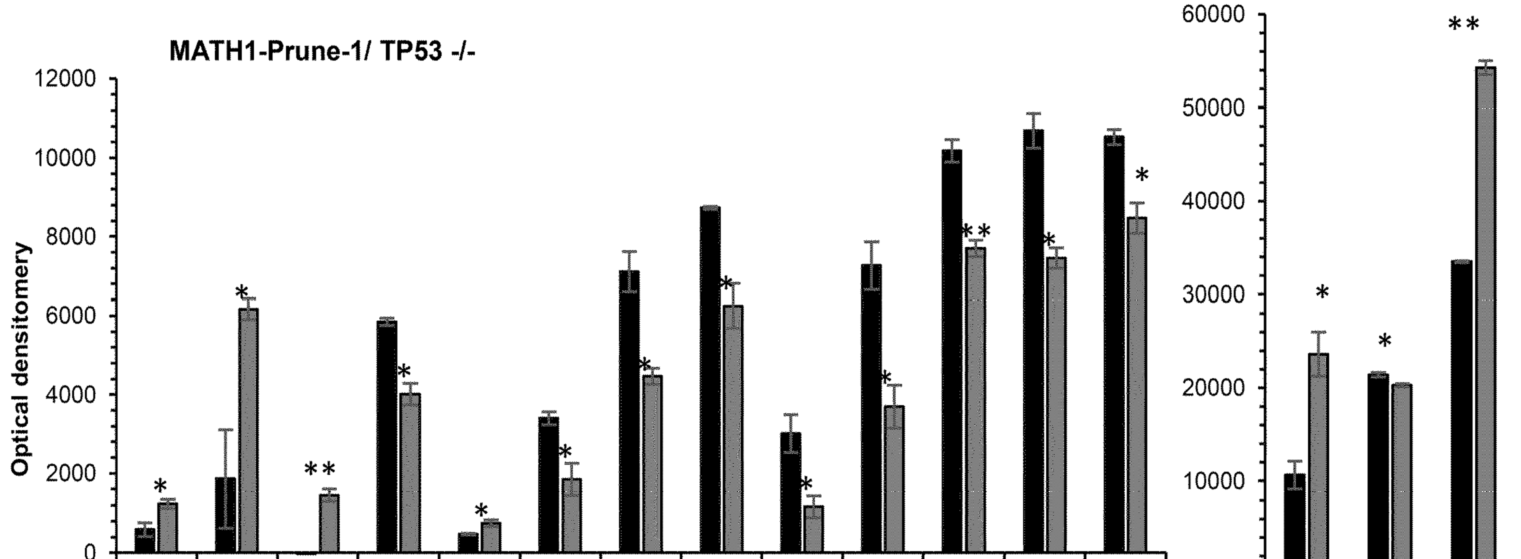


| | CD30L | CXCL16 | Eotaxin | Eotaxin-2 | IL6 | Lymphotactin | MCP-1 | P-Selectin | TARC | TIMP-1 | PF4 | TCA-3 | VCAM-1 |
|---|---|---|---|---|---|---|---|---|---|---|---|---|---|
| **CTR** | 590,75 | 1869,8 | -228,8 | 5847,8 | 474,25 | 3400,8 | 7108,8 | 8731,3 | 3013,8 | 7264,3 | 10179 | 10686 | 10527 |
| **LEO-AA7.5(S)T** | 1243 | 6166,2 | 1461,1 | 4015,9 | 751,23 | 1854,4 | 4462,8 | 6246,1 | 1169 | 3697,3 | 7700,3 | 7453,8 | 8467,8 |

| | KC | sTNF RI | IGF-BP-6 |
|---|---|---|---|
| **CTR** | 10651,8 | 21406,8 | 33501,8 |
| **LEO-AA7.5(S)T** | 23621,2 | 20284,5 | 54246,1 |

**Figure 4 (continued)**

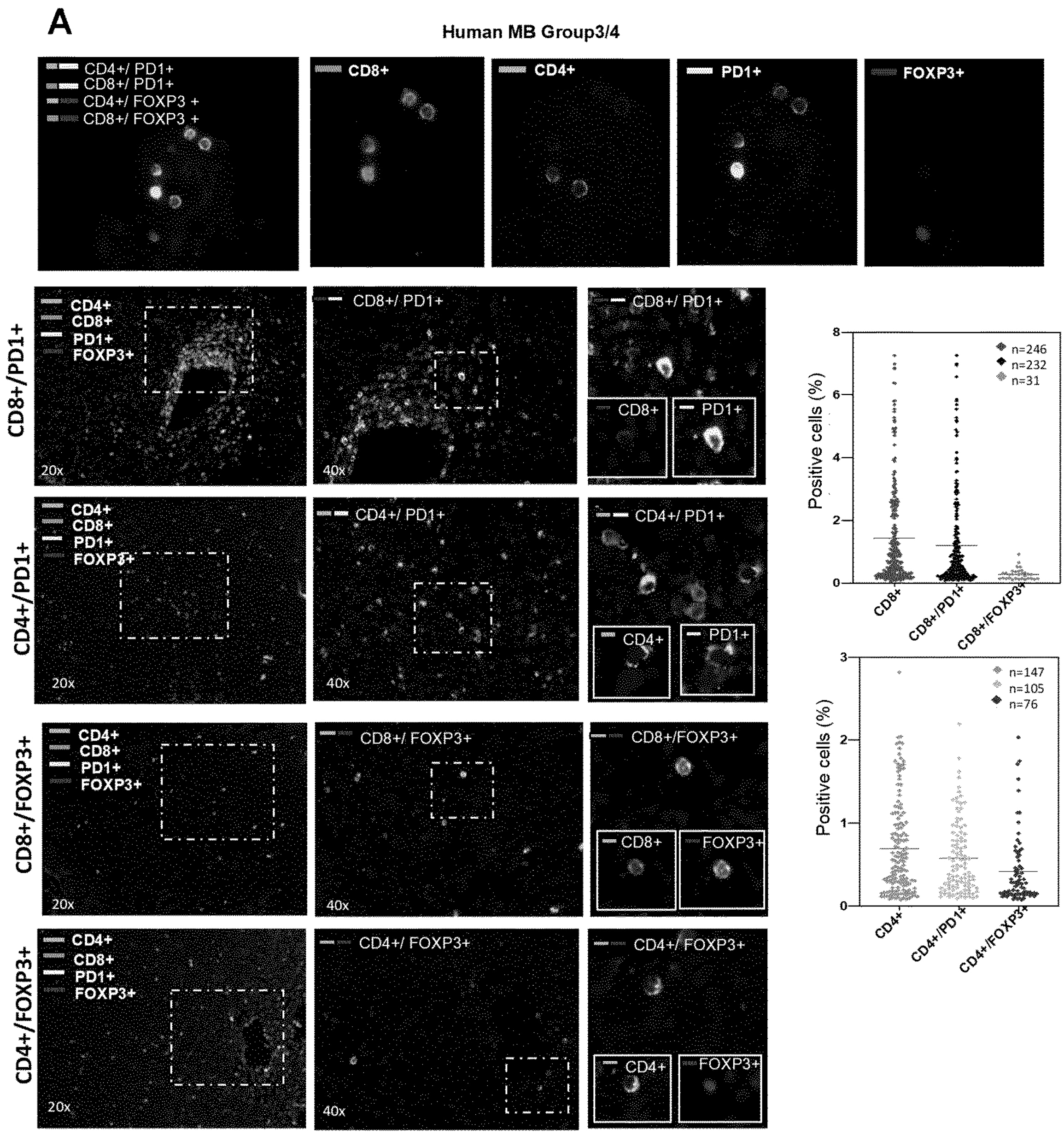
A
Human MB Group3/4
CD4+/ PD1+
CD8+/ PD1+
CD4+/ FOXP3 +
CD8+/ FOXP3 +
CD8+
CD4+
PD1+
FOXP3+
CD8+/PD1+
CD4+/PD1+
CD8+/FOXP3+
CD4+/FOXP3+
CD4+
CD8+
PD1+
FOXP3+
20x
40x
Positive cells (%)
n=246
n=232
n=31
CD8+
CD8+/PD1+
CD8+/FOXP3+
n=147
n=105
n=76
CD4+
CD4+/PD1+
CD4+/FOXP3+

Figure 5

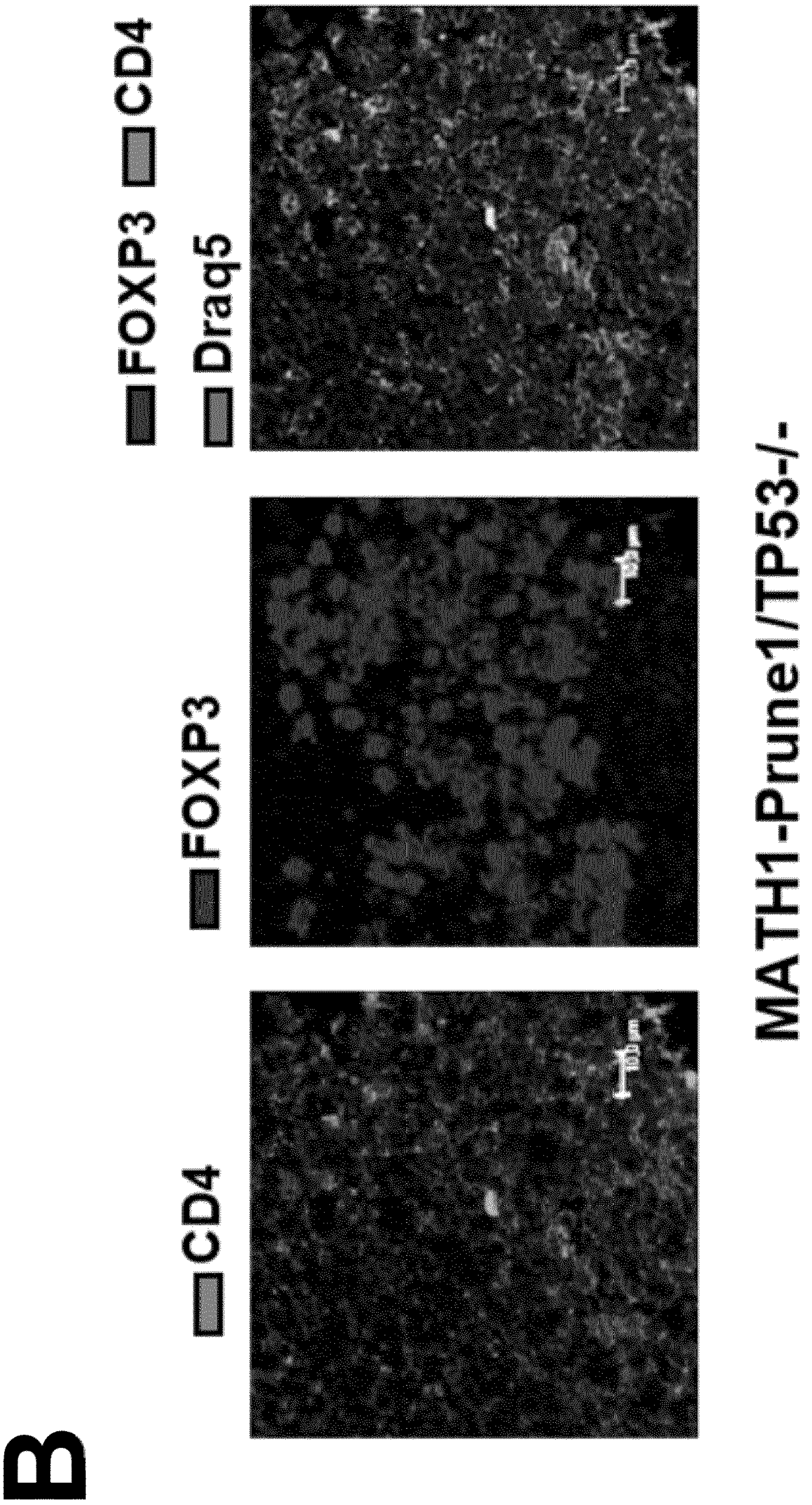


Figure 5 (continued)

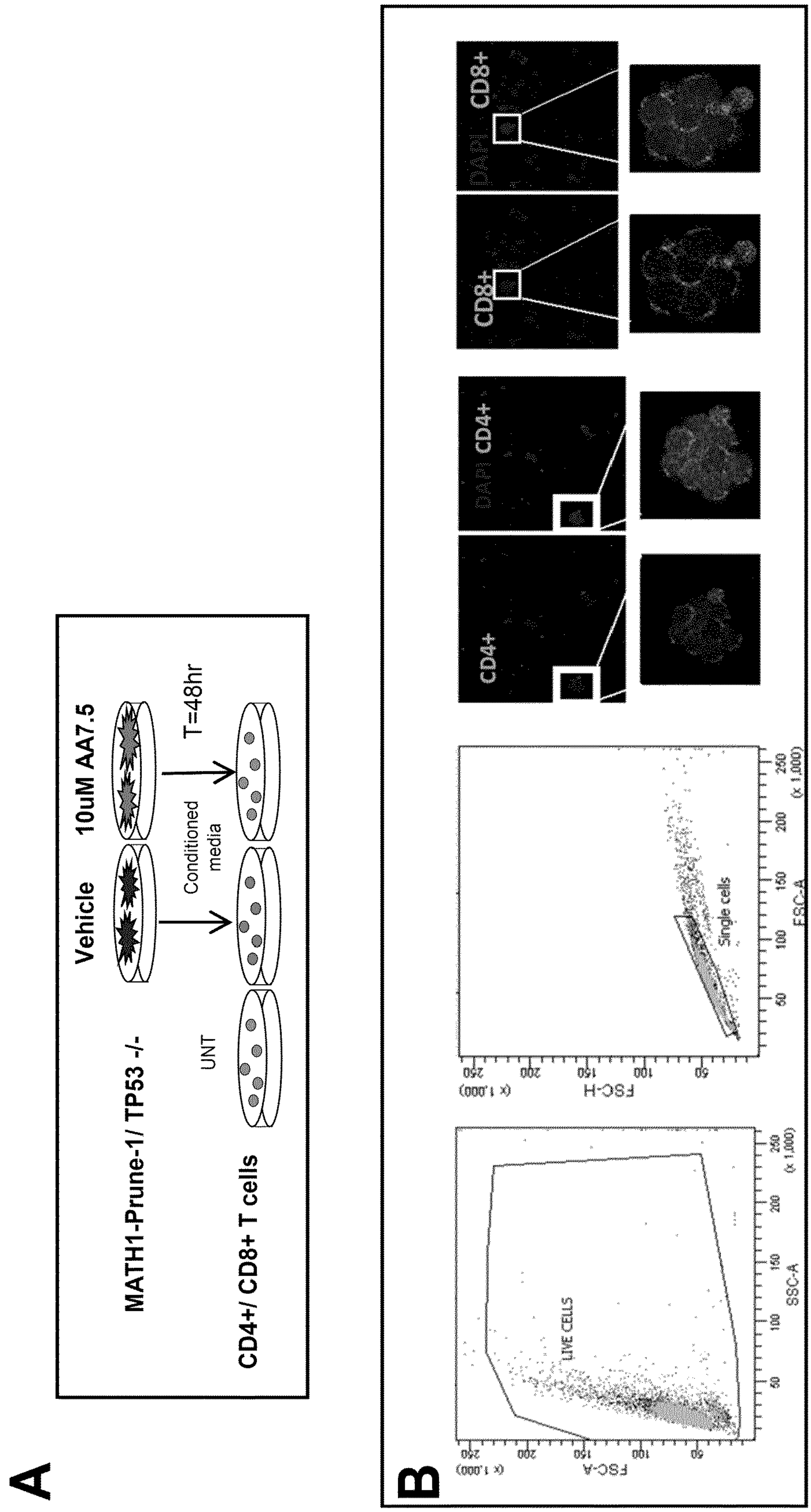
A
MATH1-Prune-1/ TP53 -/-
Vehicle
10uM AA7.5
Conditioned media
T=48hr
UNT
CD4+/ CD8+ T cells
B
LIVE CELLS
FSC-A
SSC-A
FSC-H
Single cells
CD4+
DAPI CD4+
CD8+
DAPI CD8+

Figure 6

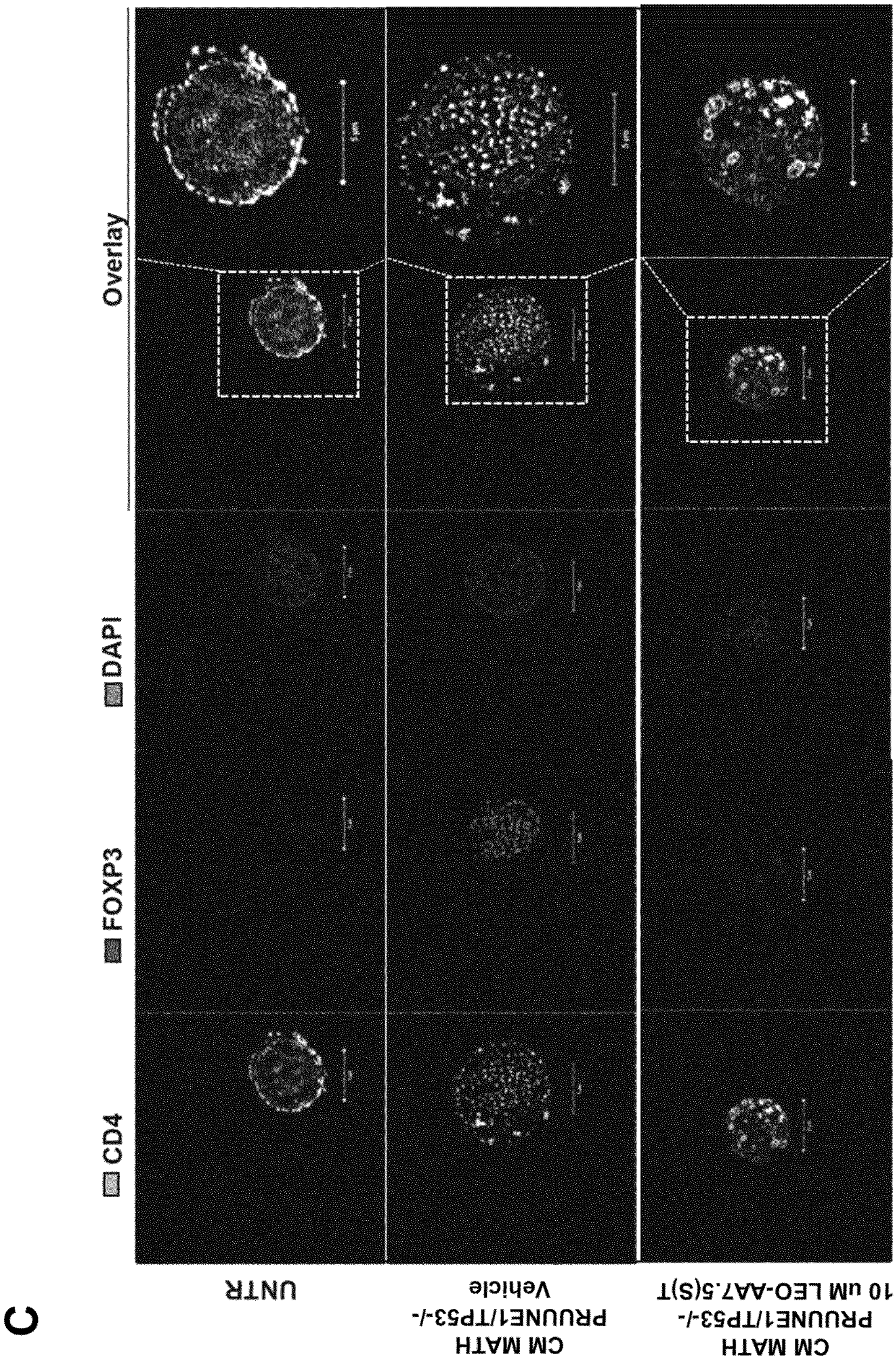


Figure 6 (continued)

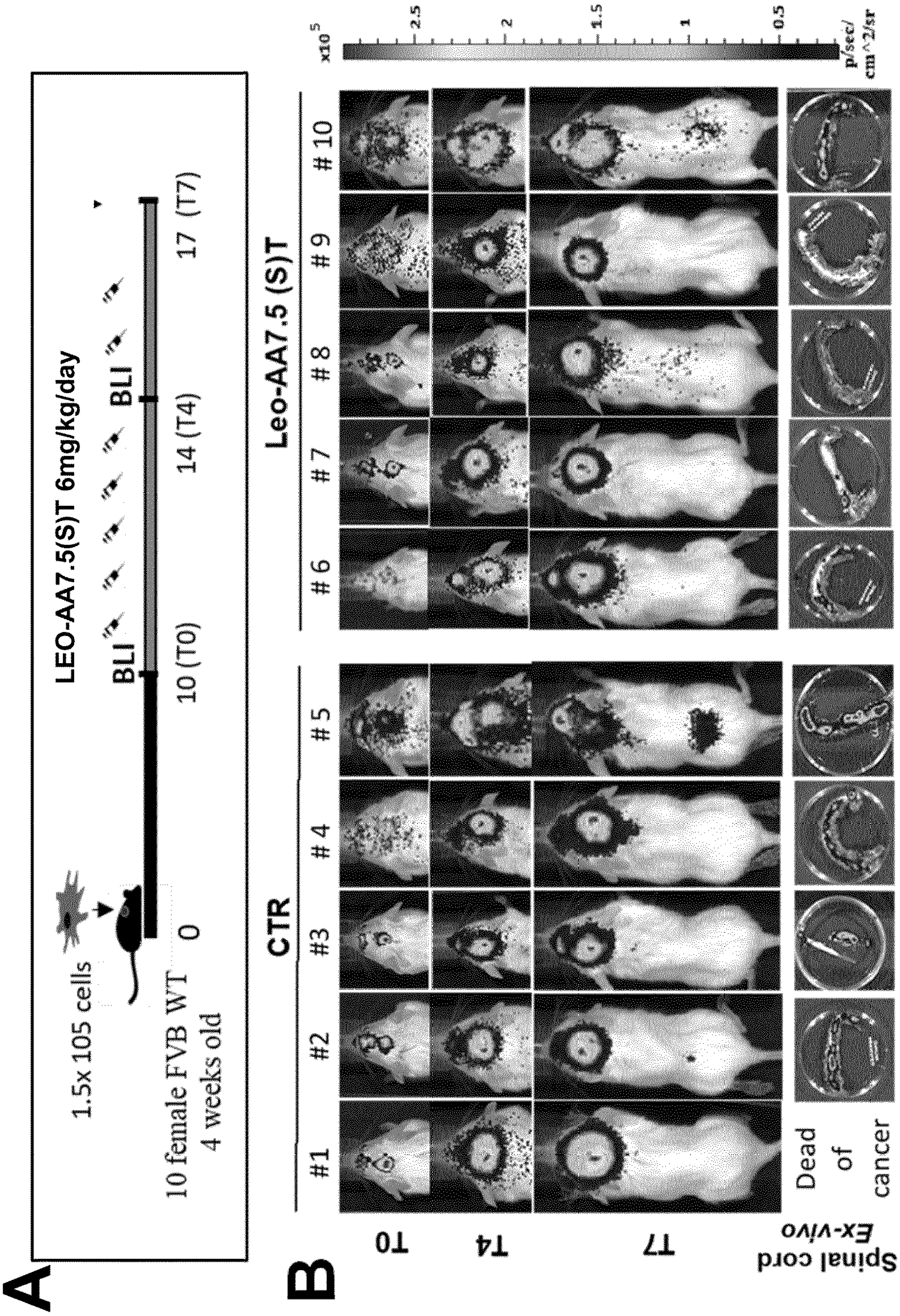
A
1.5x 105 cells
10 female FVB WT
4 weeks old
LEO-AA7.5(S)T 6mg/kg/day
BLI
BLI
0
10 (T0)
14 (T4)
17 (T7)
B
CTR
Leo-AA7.5 (S)T
#1
#2
#3
#4
#5
# 6
# 7
# 8
# 9
# 10
T0
T4
T7
Spinal cord
Ex-vivo
Dead of cancer
2.52E+05
2.06E+05
2.09E+05
6.04E+05
1.01E+05
9.46E+04
6.55E+04
9.69E+04
1.03E+04
x10^5
2.5
2
1.5
1
0.5
p/sec/cm^2/sr

Figure 7

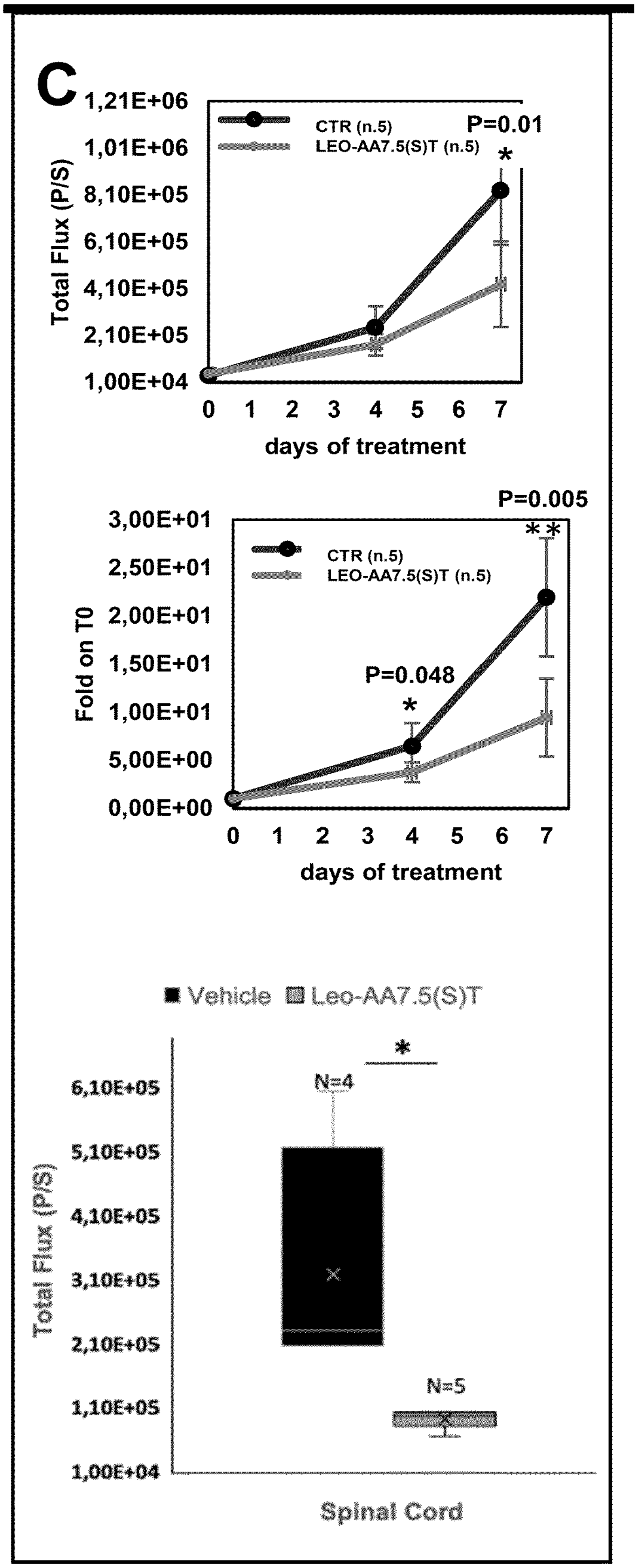


Figure 7 (continued)

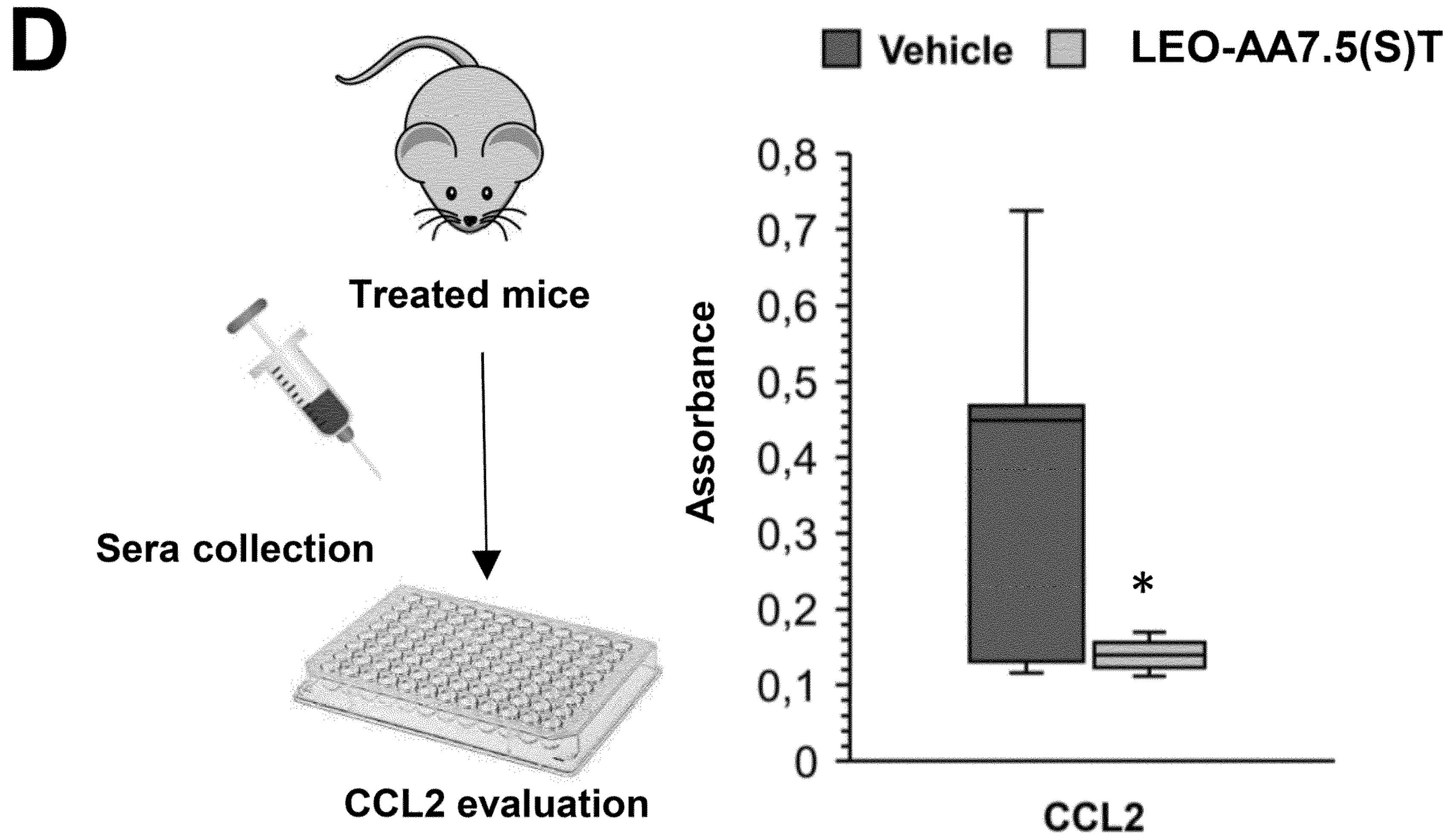


Figure 7 (continued)

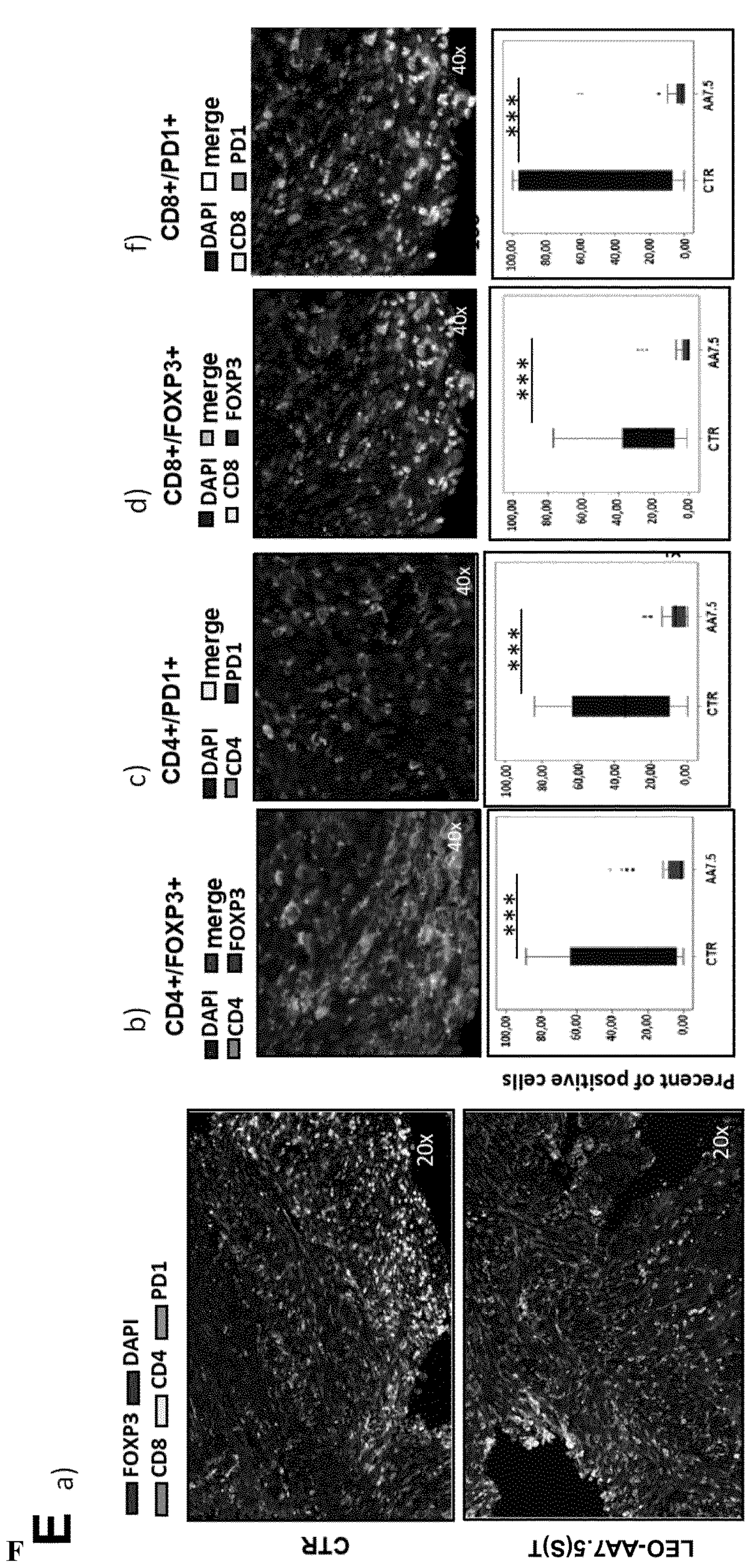


Figure 7 (continued)

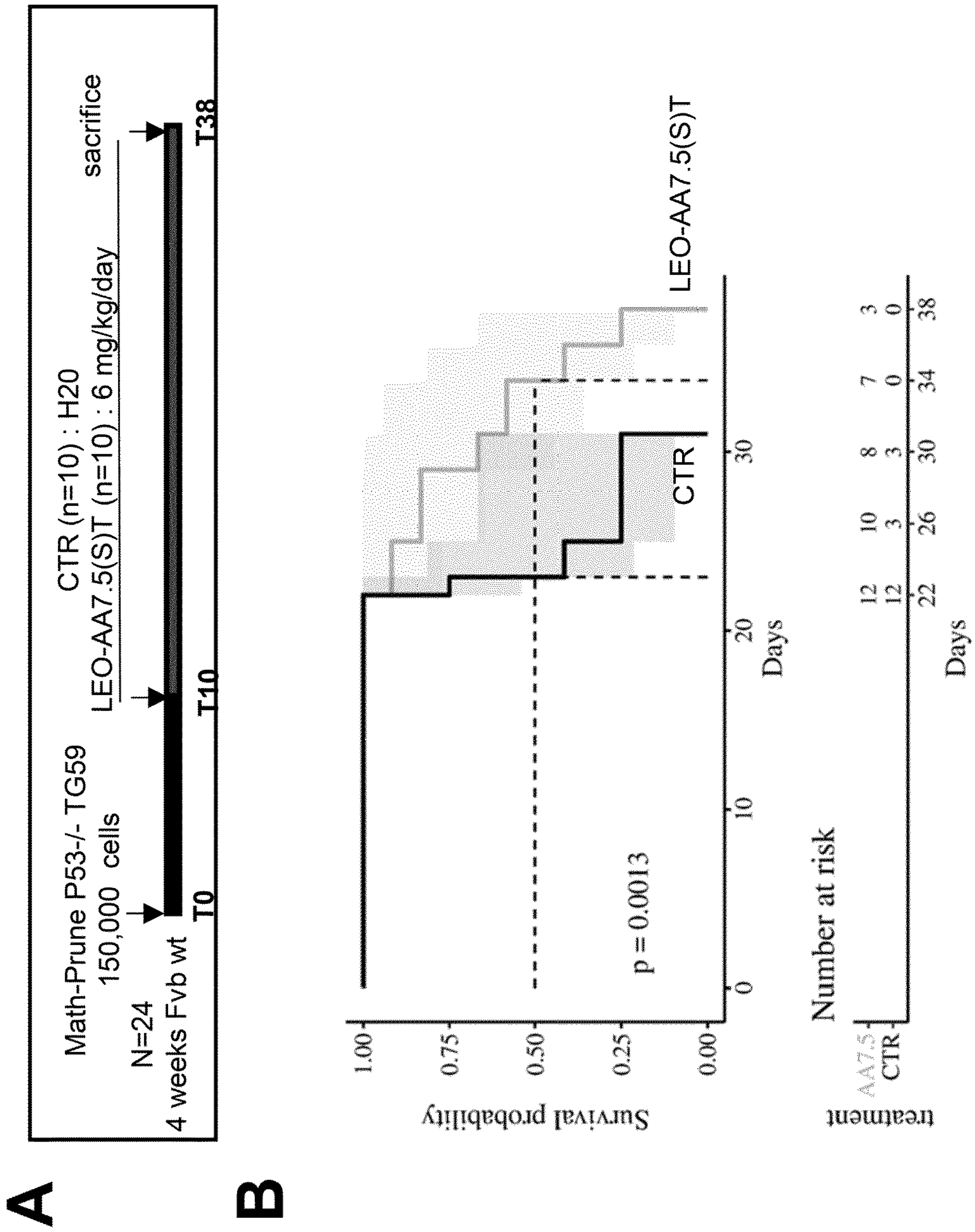
A
Math-Prune P53-/- TG59
150,000 cells
N=24
4 weeks Fvb wt
CTR (n=10) : H20
LEO-AA7.5(S)T (n=10) : 6 mg/kg/day
sacrifice
T0
T10
T38
B
Survival probability
1.00
0.75
0.50
0.25
0.00
p = 0.0013
CTR
LEO-AA7.5(S)T
0
10
20
30
Days
Number at risk
treatment
AA7.5
CTR
12 10 8 7 3
12 3 3 0 0
22 26 30 34 38
Days

Figure 8

C

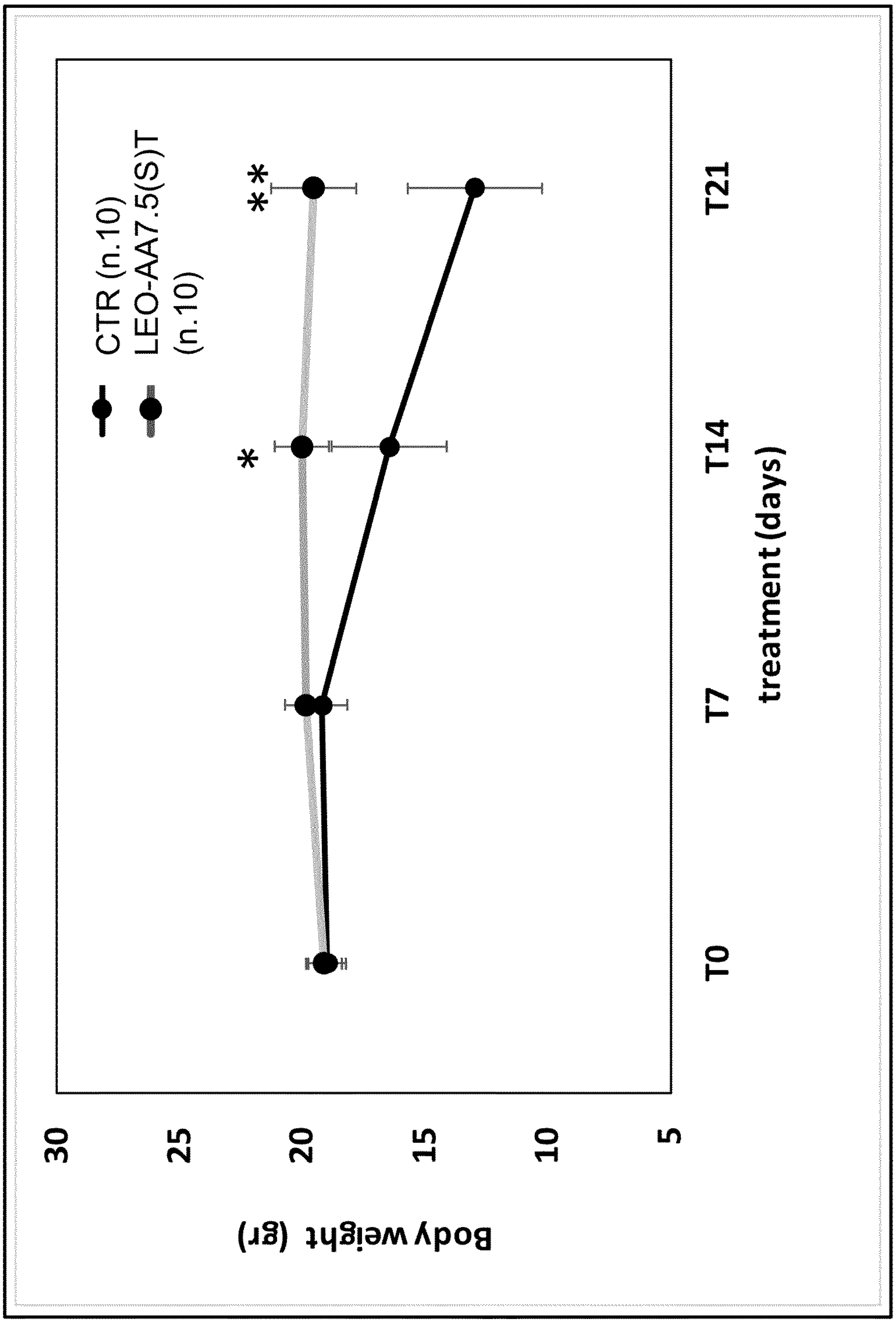


Figure 8 (continued)

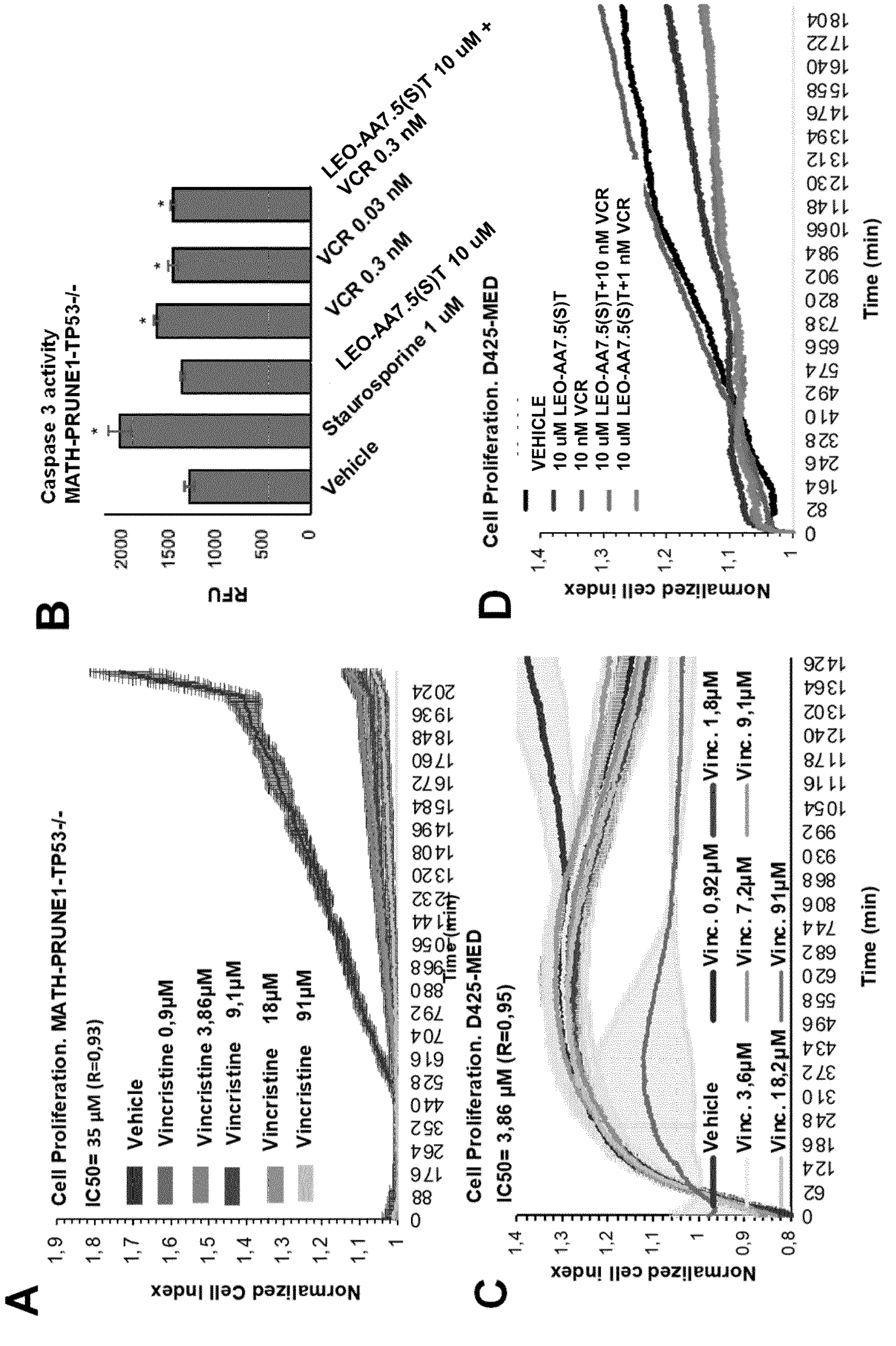
A
Cell Proliferation. MATH-PRUNE1-TP53-/-
IC50= 35 µM (R=0,93)
Vehicle
Vincristine 0,9µM
Vincristine 3,86µM
Vincristine 9,1µM
Vincristine 18µM
Vincristine 91µM
Normalized Cell Index
Time (min)
B
Caspase 3 activity
MATH-PRUNE1-TP53-/-
RFU
Vehicle
Staurosporine 1 uM
LEO-AA7.5(S)T 10 uM
VCR 0.3 nM
VCR 0.03 nM
LEO-AA7.5(S)T 10 uM + VCR 0.3 nM
C
Cell Proliferation. D425-MED
IC50= 3,86 µM (R=0,95)
Vehicle
Vinc. 0,92µM
Vinc. 1,8µM
Vinc. 3,6µM
Vinc. 7,2µM
Vinc. 9,1µM
Vinc. 18,2µM
Vinc. 91µM
Normalized cell index
Time (min)
D
Cell Proliferation. D425-MED
VEHICLE
10 uM LEO-AA7.5(S)T
10 nM VCR
10 uM LEO-AA7.5(S)T+10 nM VCR
10 uM LEO-AA7.5(S)T+1 nM VCR
Normalized cell index
Time (min)

Figure 9

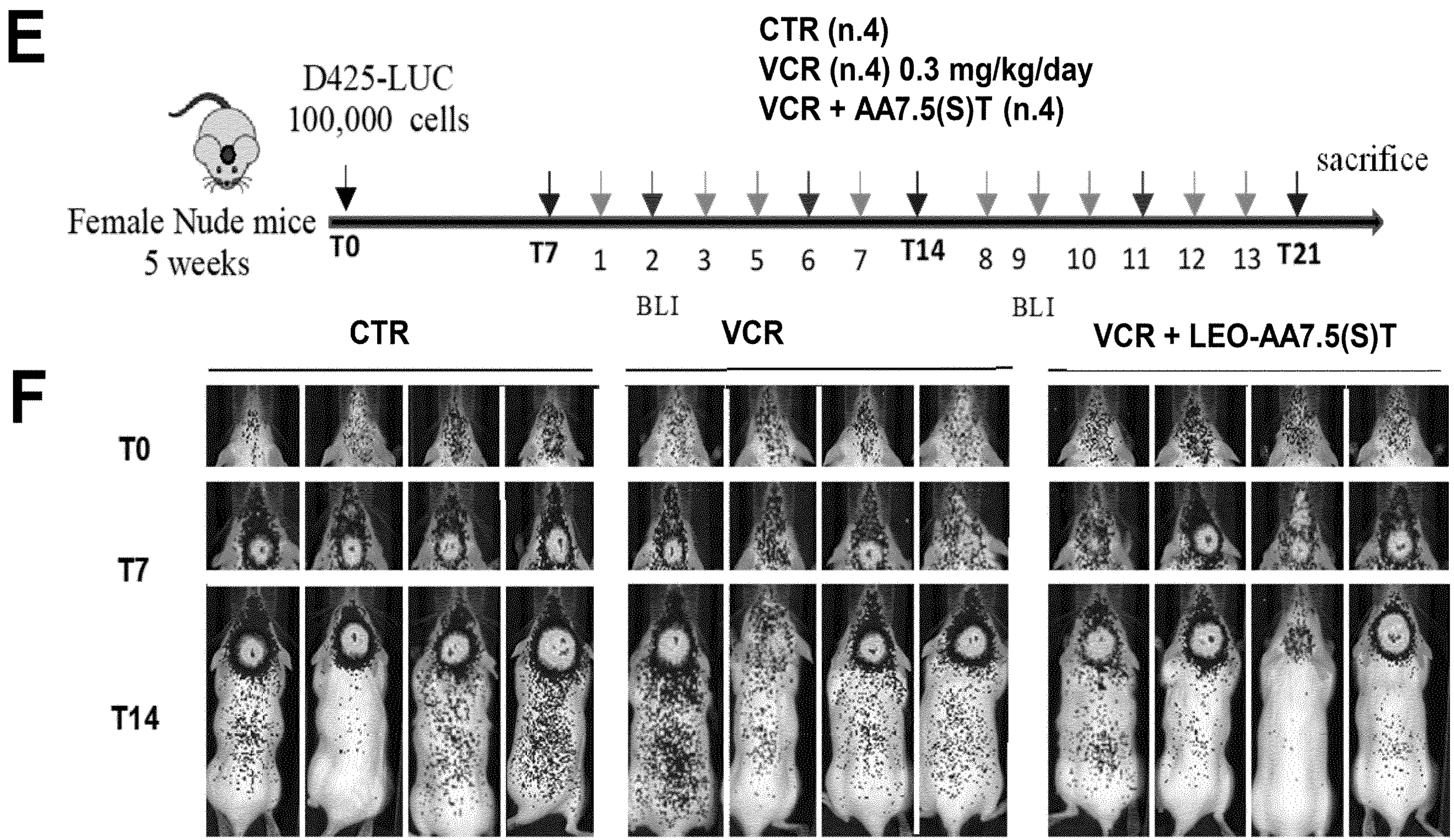


Figure 9 (continued)

G

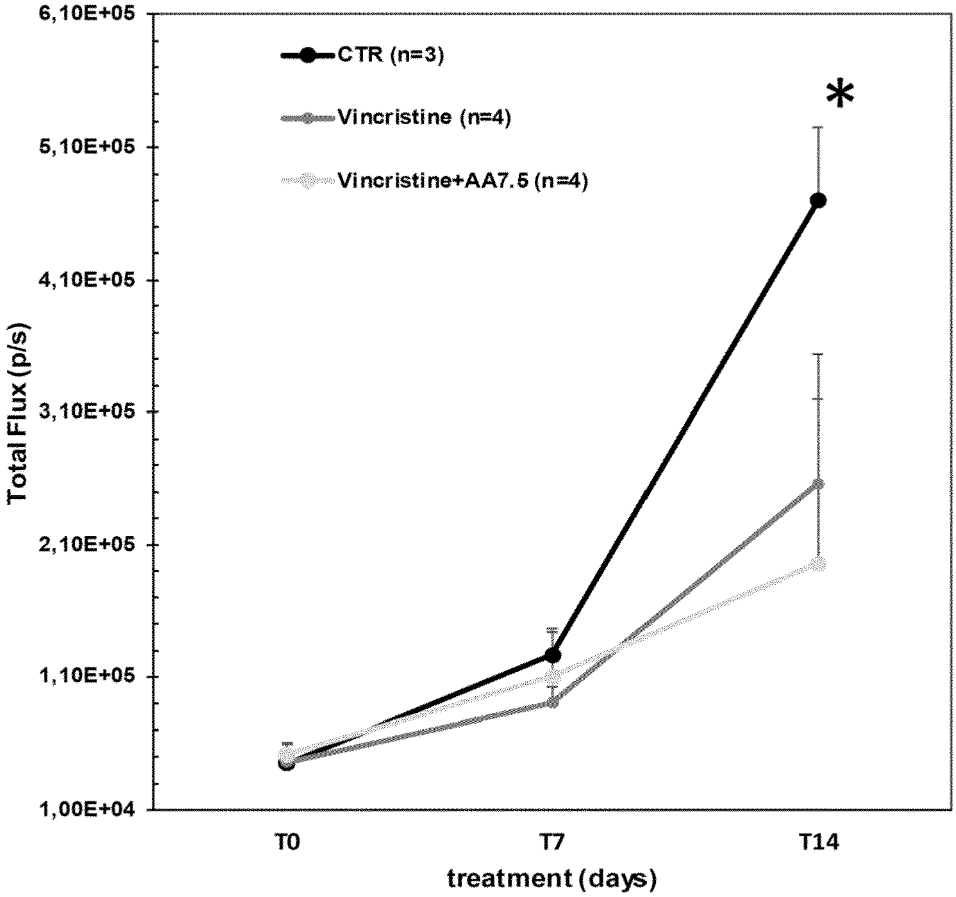


Figure 9 (continued)

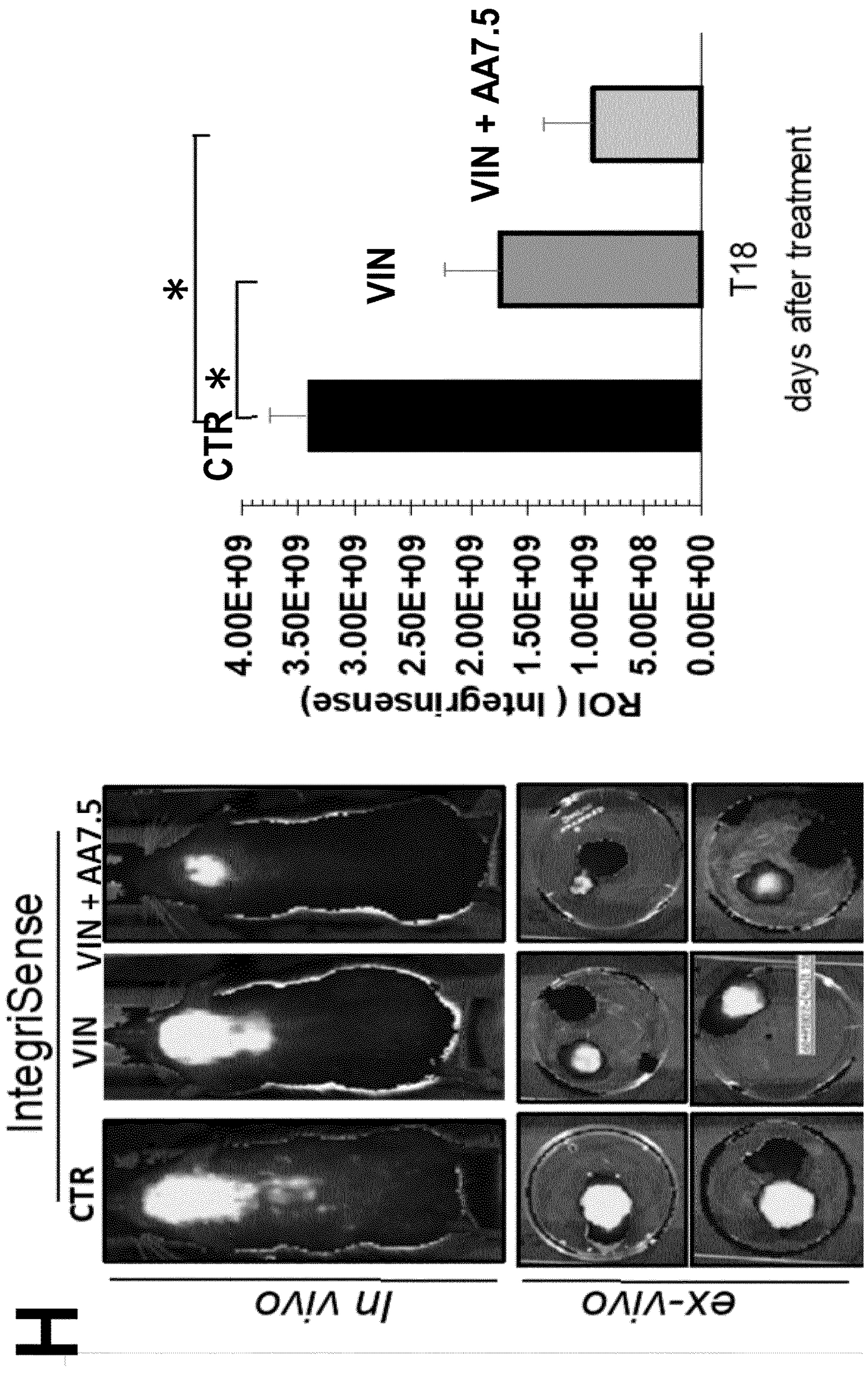


Figure 9 (continued)

A

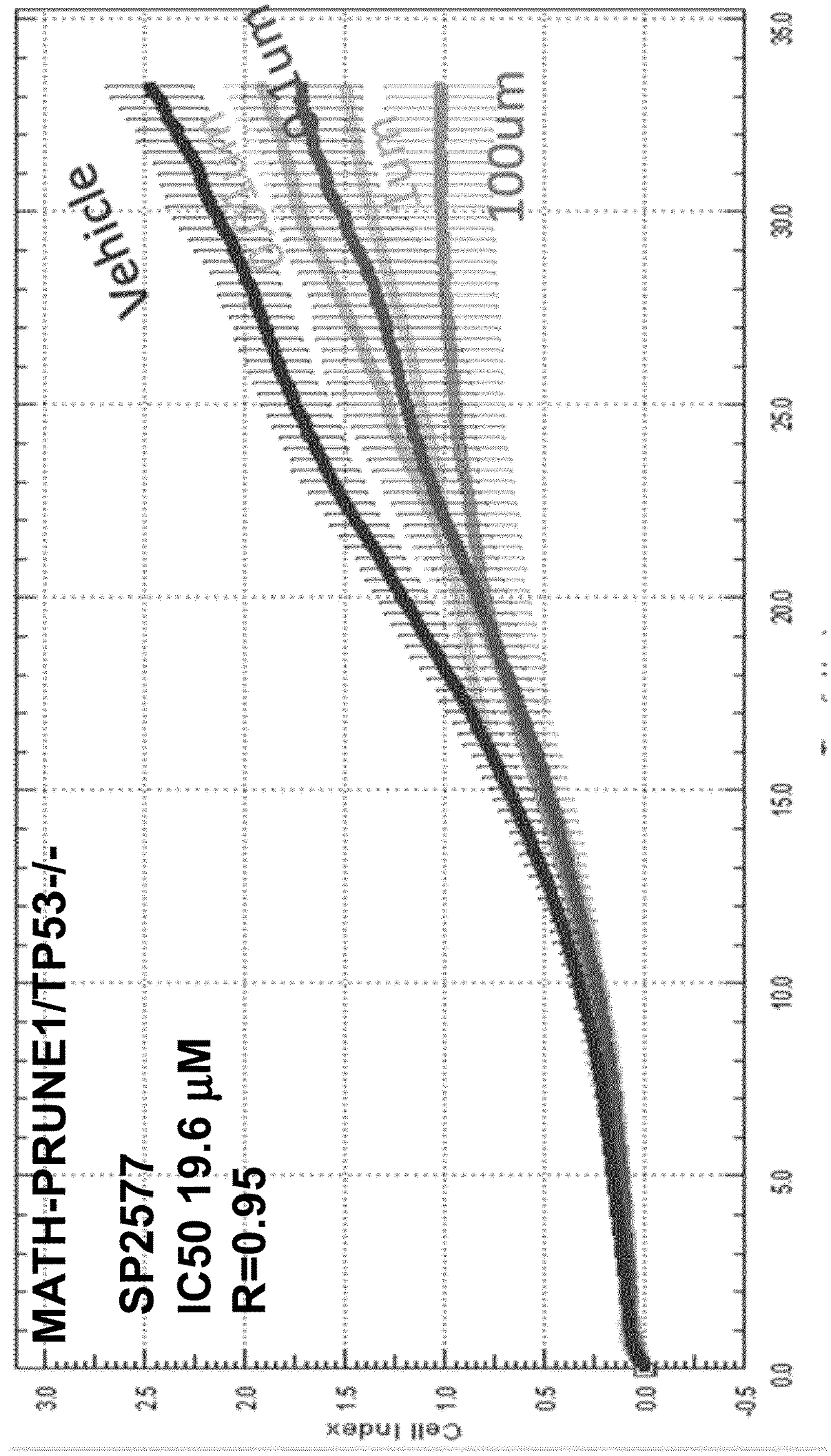
MATH-PRUNE1/TP53-/-
SP2577
IC50 19.6 µM
R=0.95
Vehicle
100um
Cell Index
3.0
2.5
2.0
1.5
1.0
0.5
0.0
-0.5
0.0
5.0
10.0
15.0
20.0
25.0
30.0
35.0

Figure 10

B

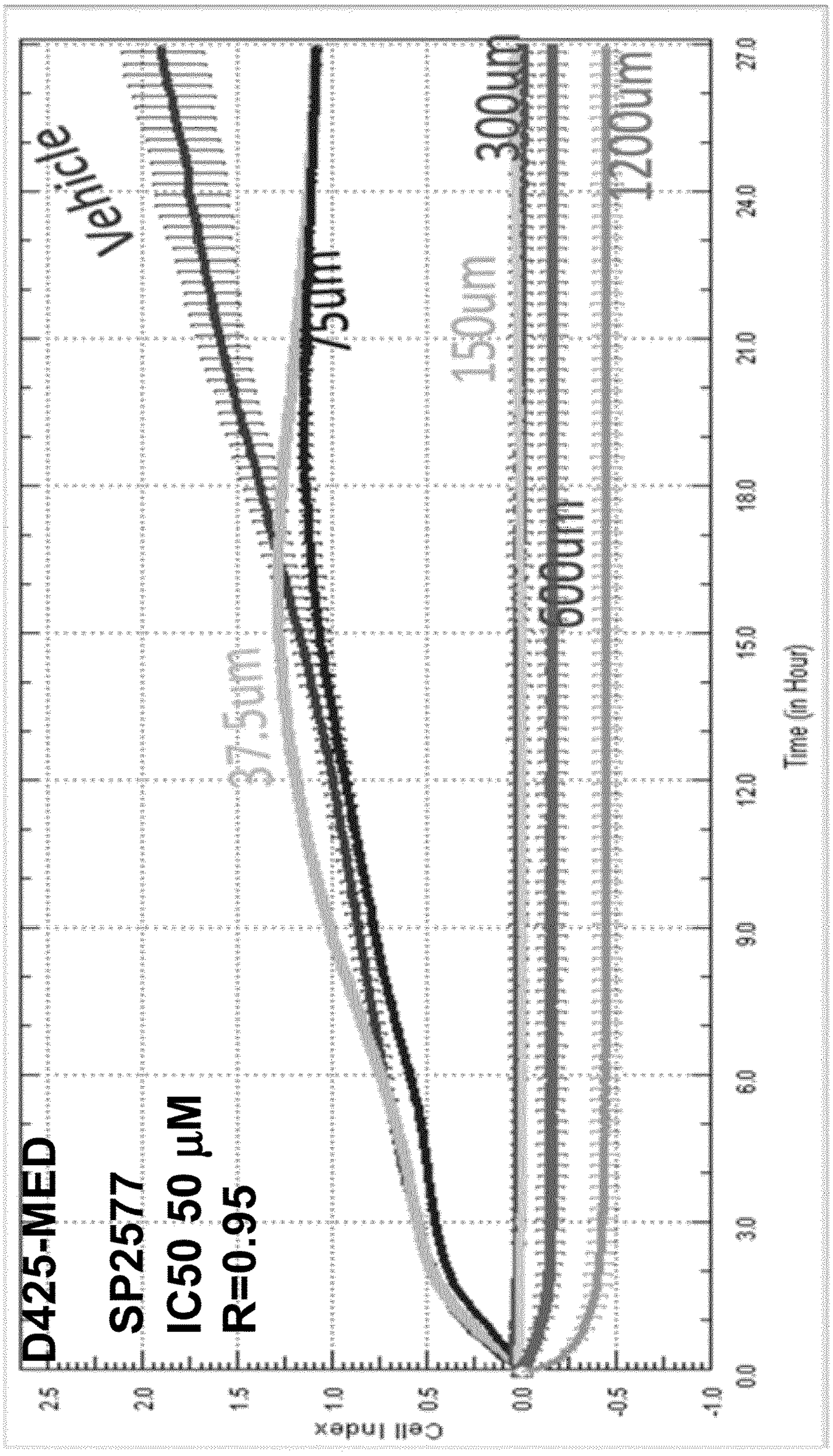


Figure 10 (continued)

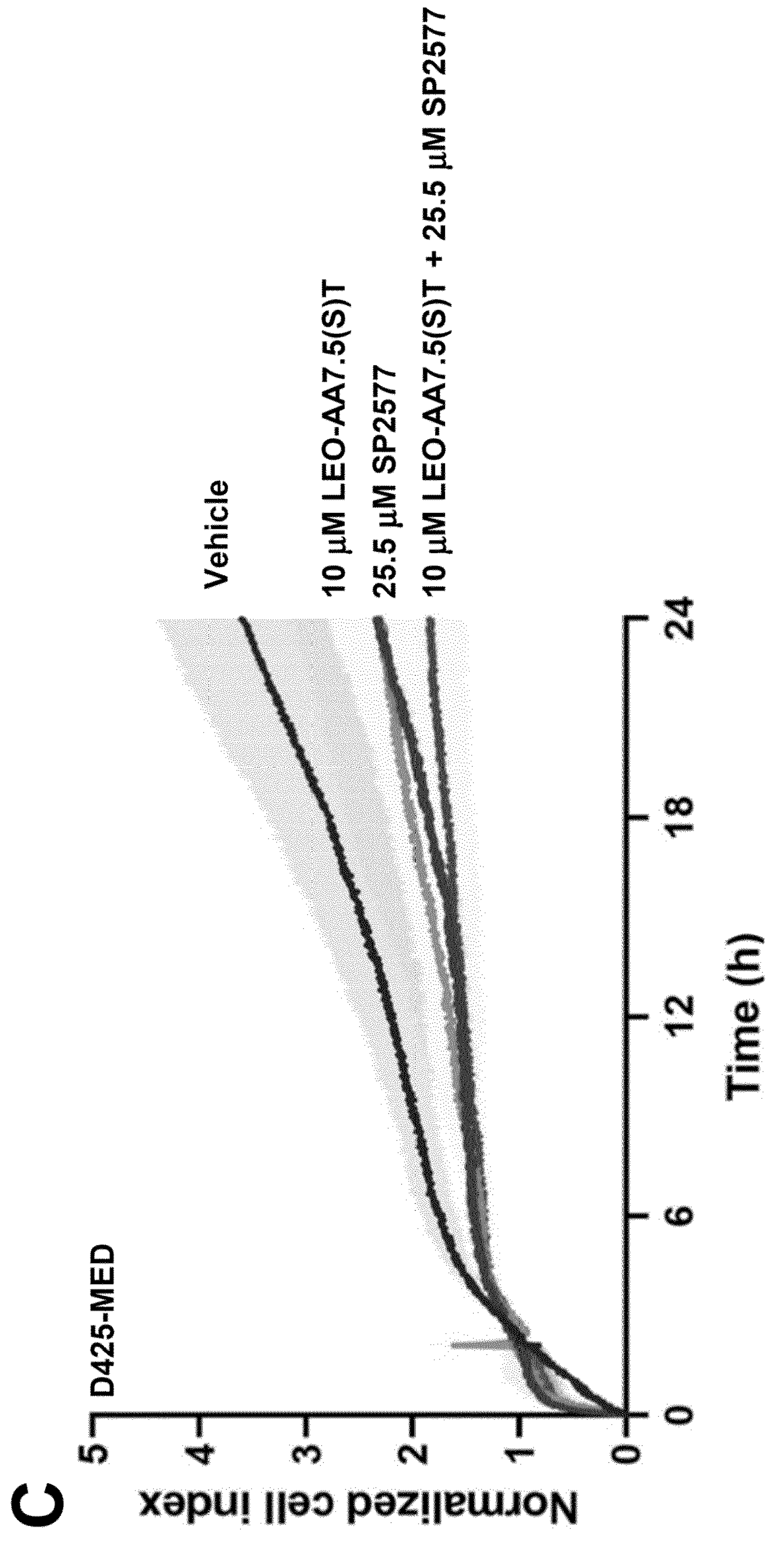


Figure 10 (continued)

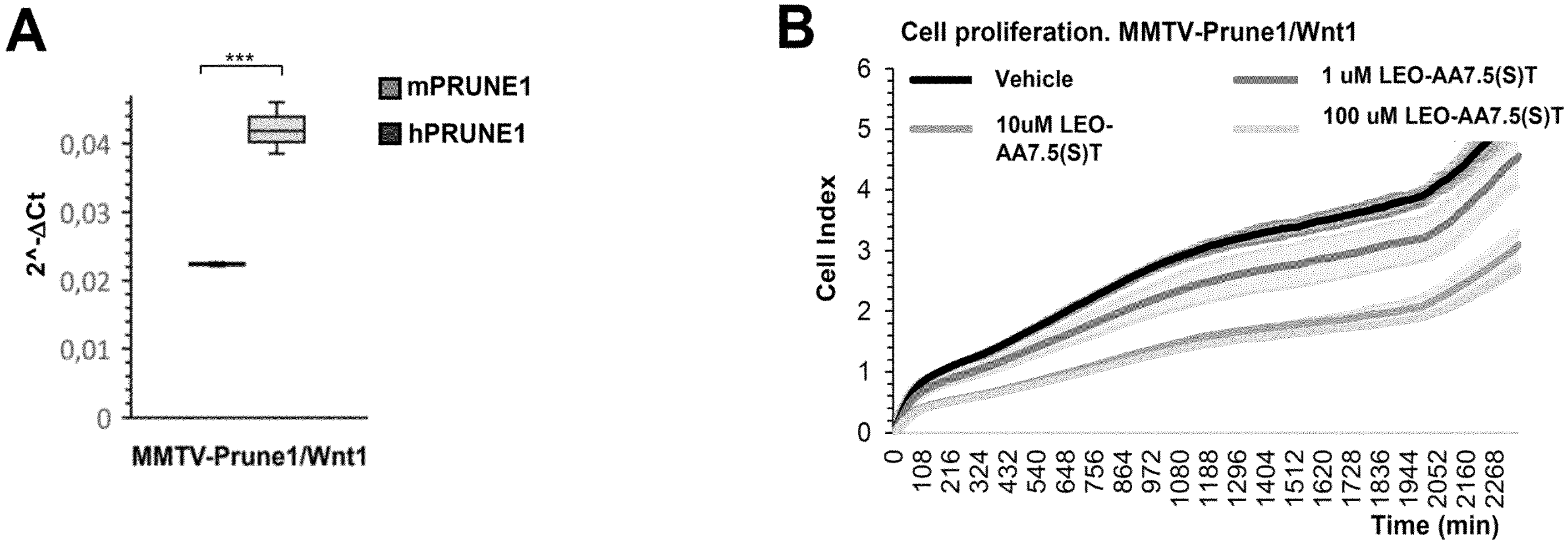
A
***
mPRUNE1
hPRUNE1
2^-ΔCt
0,04
0,03
0,02
0,01
0
MMTV-Prune1/Wnt1
B
Cell proliferation. MMTV-Prune1/Wnt1
Vehicle
10uM LEO-AA7.5(S)T
1 uM LEO-AA7.5(S)T
100 uM LEO-AA7.5(S)T
Cell Index
6
5
4
3
2
1
0
0
108
216
324
432
540
648
756
864
972
1080
1188
1296
1404
1512
1620
1728
1836
1944
2052
2160
2268
Time (min)

Figure 11

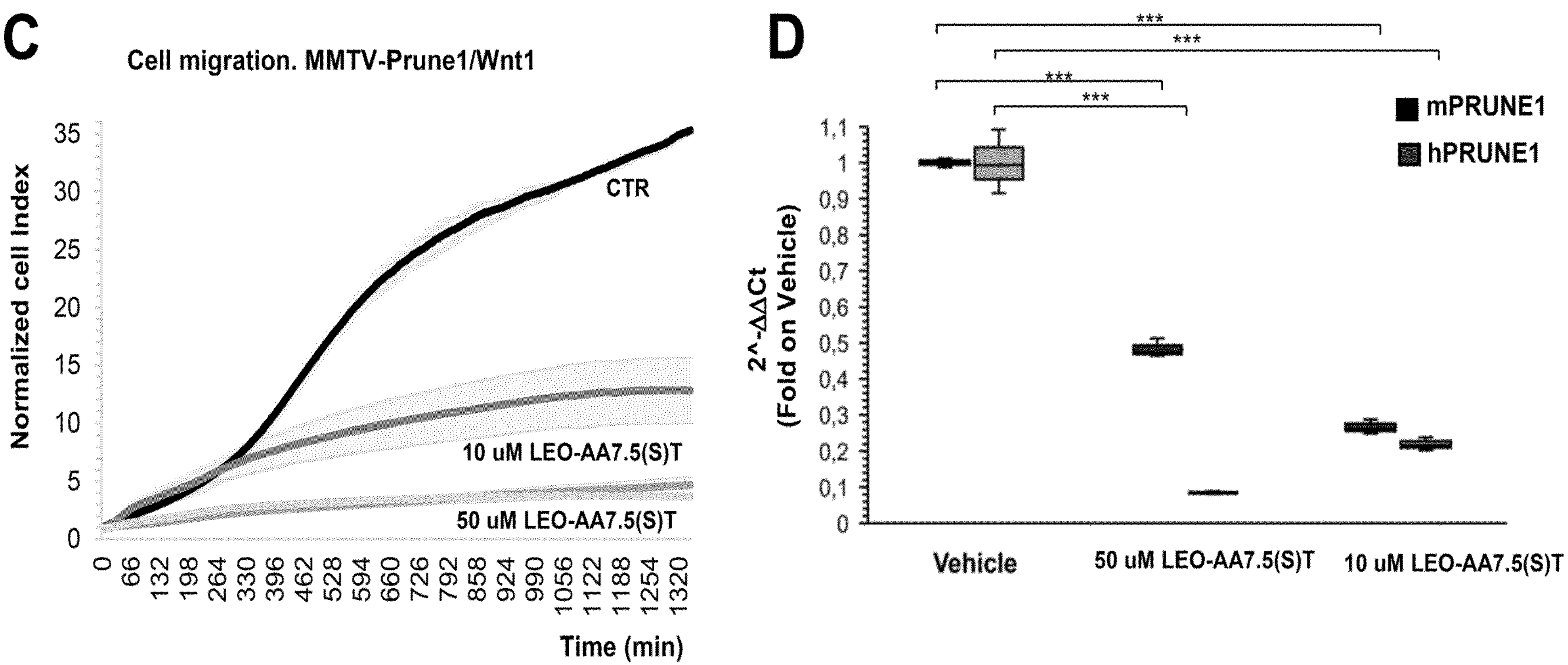


Figure 11 (continued)

E

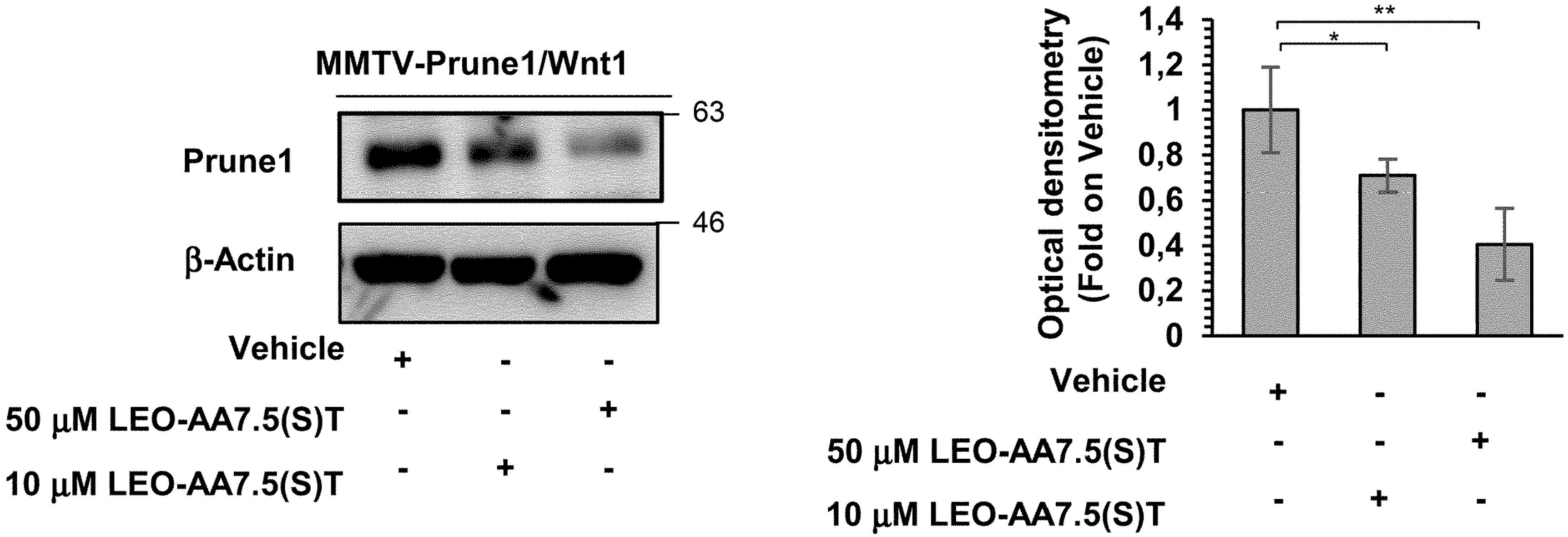


F

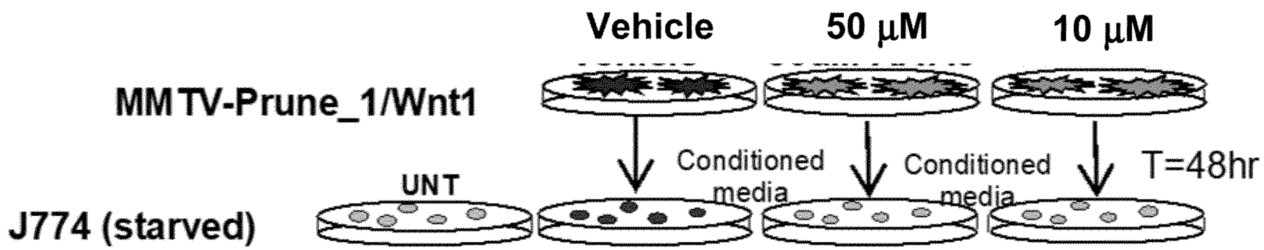


Figure 11 (continued)

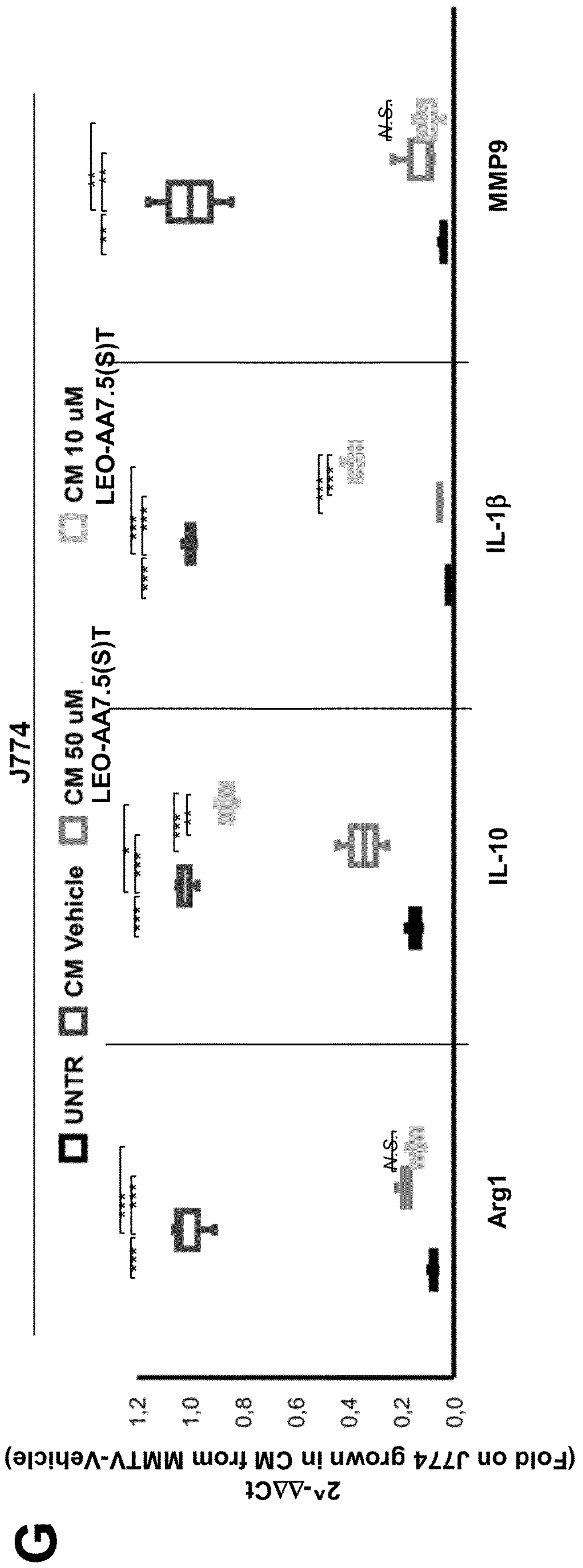


Figure 11 (continued)

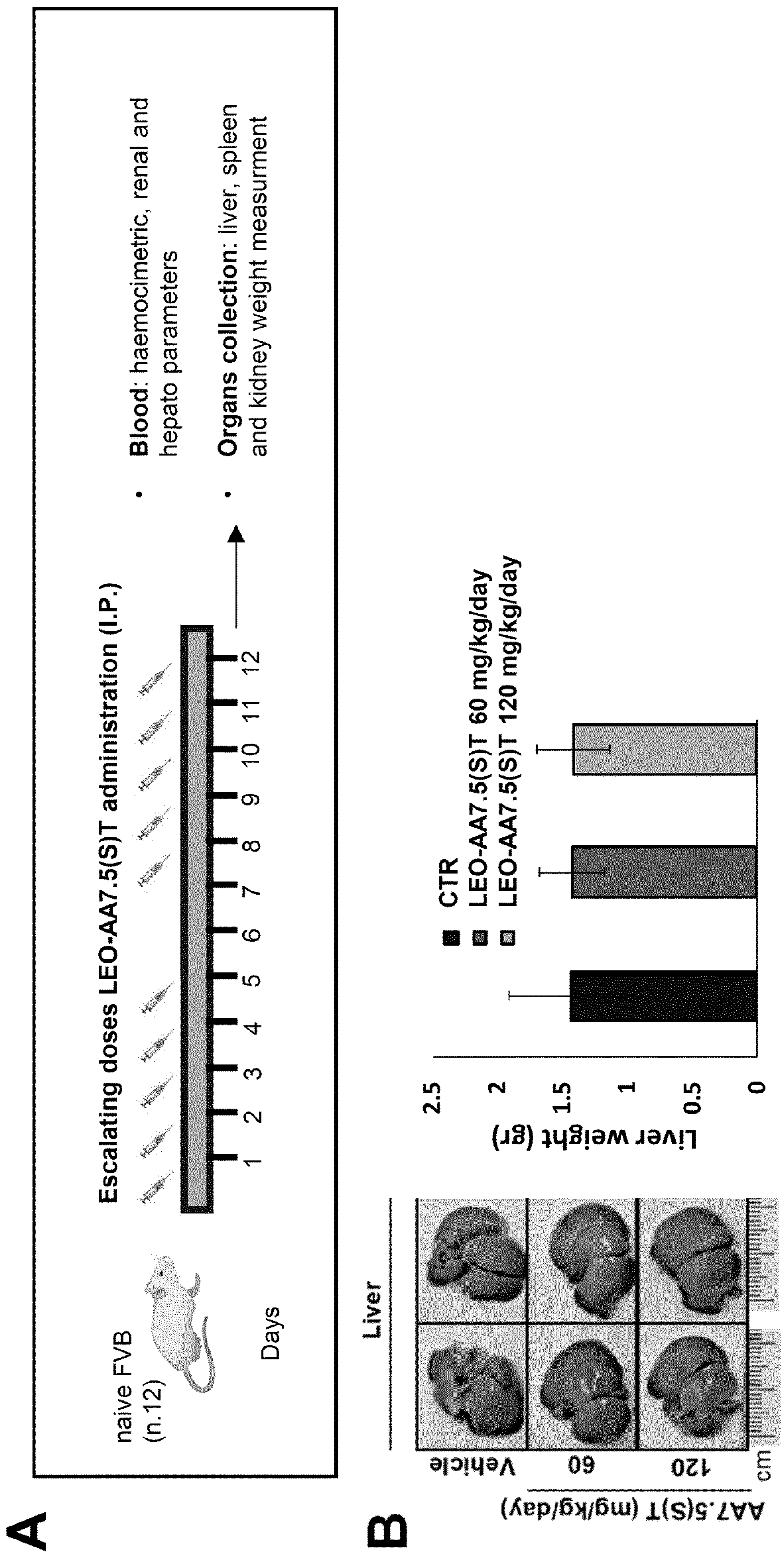
A
naive FVB
(n.12)
Escalating doses LEO-AA7.5(S)T administration (I.P.)
Days
1 2 3 4 5 6 7 8 9 10 11 12
Blood: haemocimetric, renal and hepato parameters
Organs collection: liver, spleen and kidney weight measurment
B
Liver
AA7.5(S)T (mg/kg/day)
Vehicle
60
120
cm
Liver weight (gr)
0
0.5
1
1.5
2
2.5
CTR
LEO-AA7.5(S)T 60 mg/kg/day
LEO-AA7.5(S)T 120 mg/kg/day

Figure 12

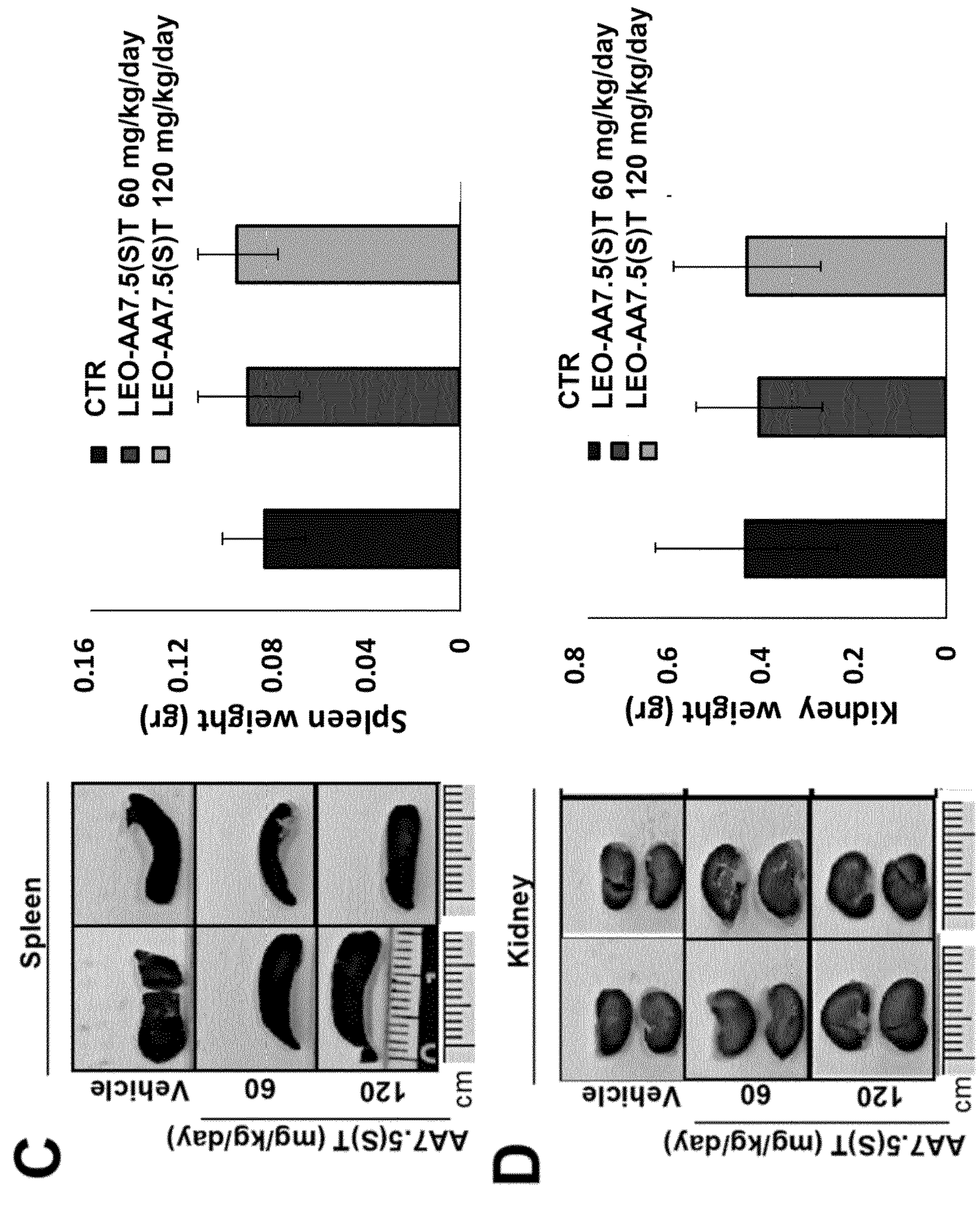


Figure 12 (continued)

**E**

| | Reference values | | (FVB naïve mice) 9 days treatment | | |
|---|---|---|---|---|---|
| | | | CTR | LEO-AA7.5(S)T (mg/Kg/day | |
| | **Unit** | **Values** | **PBS** | **60** | **120** |
| **WBC** | x10³/µL | 4 - 12 | **1.97** | **3.04** | **3.07** |
| **LYM** | % | 30 - 90 | 80 | 85.2 | 85.6 |
| **MONO** | % | 0 - 10 | 4.7 | 3.9 | 2.7 |
| **GRAN** | % | 0 - 5 | 3.2 | 2.3 | 2 |
| **HGB** | g/dl | 10 - 20 | 11.5 | 11.8 | 9.5 |
| **HCT** | % | 35 - 45 | 27.9 | 25.9 | 26.8 |
| **RBC** | x106/µL | 7 - 11 | 6 | 5.55 | 5.76 |
| **MCV** | fl | ND | 46.5 | 46.7 | 46.6 |
| **MCH** | pg | ND | 19.2 | 21.3 | 16.5 |
| **MCHC** | g/dl | ND | 41.2 | 45.5 | 35.4 |
| **PLT** | x10³/µL | ND | 814 | 1337 | 768 |
| **GPT** | U/l | 26 - 77 | 128 | 89 | 81 |
| **CRE** | mg /dl | 0.3 - 1 | 0.09 | 0.25 | 0.72 |
| **BUN** | mg/dl | 17-28 | **37** | **48** | **46** |

Figure 12 (continued)

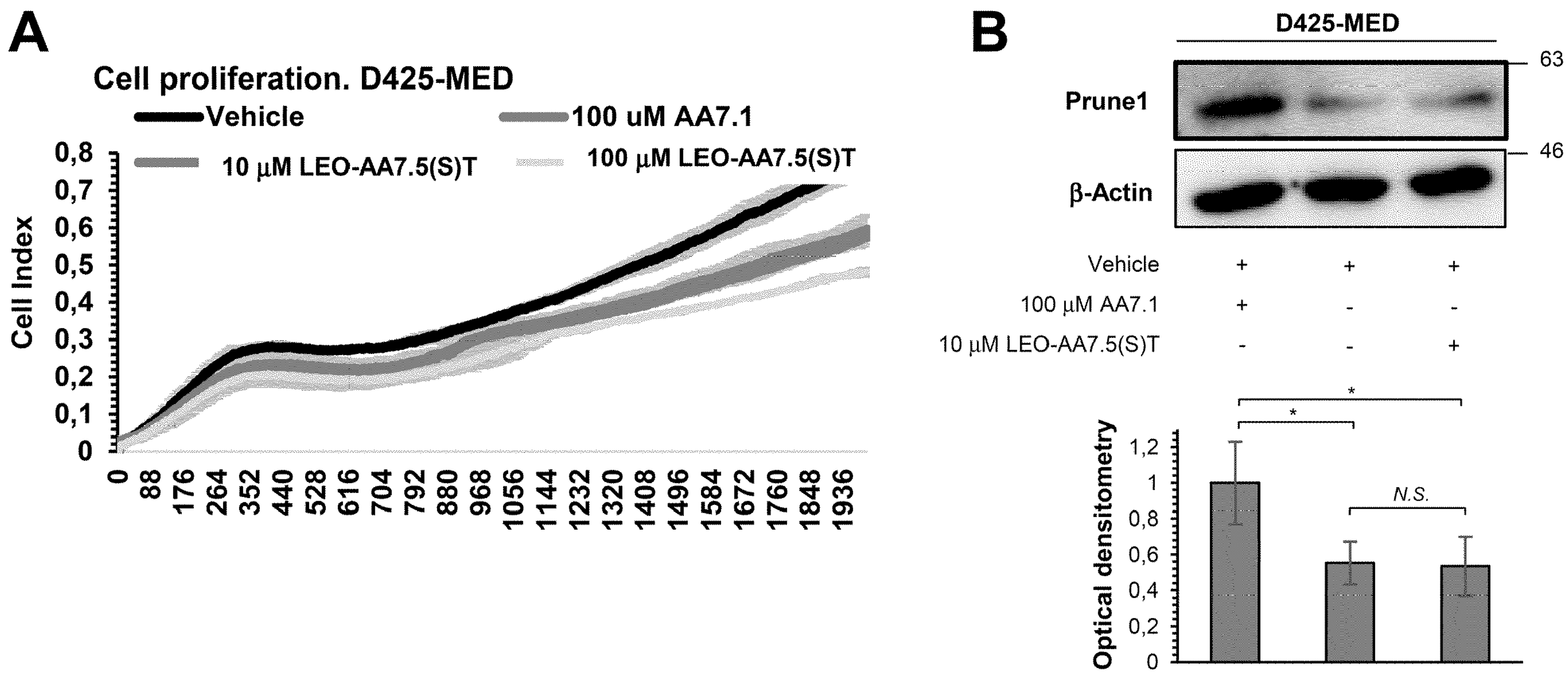
A
Cell proliferation. D425-MED
Vehicle
100 uM AA7.1
10 μM LEO-AA7.5(S)T
100 μM LEO-AA7.5(S)T
Cell Index
0,8
0,7
0,6
0,5
0,4
0,3
0,2
0,1
0
0
88
176
264
352
440
528
616
704
792
880
968
1056
1144
1232
1320
1408
1496
1584
1672
1760
1848
1936
B
D425-MED
63
46
Prune1
β-Actin
Vehicle + + +
100 μM AA7.1 + - -
10 μM LEO-AA7.5(S)T - - +
*
*
N.S.
Optical densitometry
1,2
1
0,8
0,6
0,4
0,2
0

Figure 13

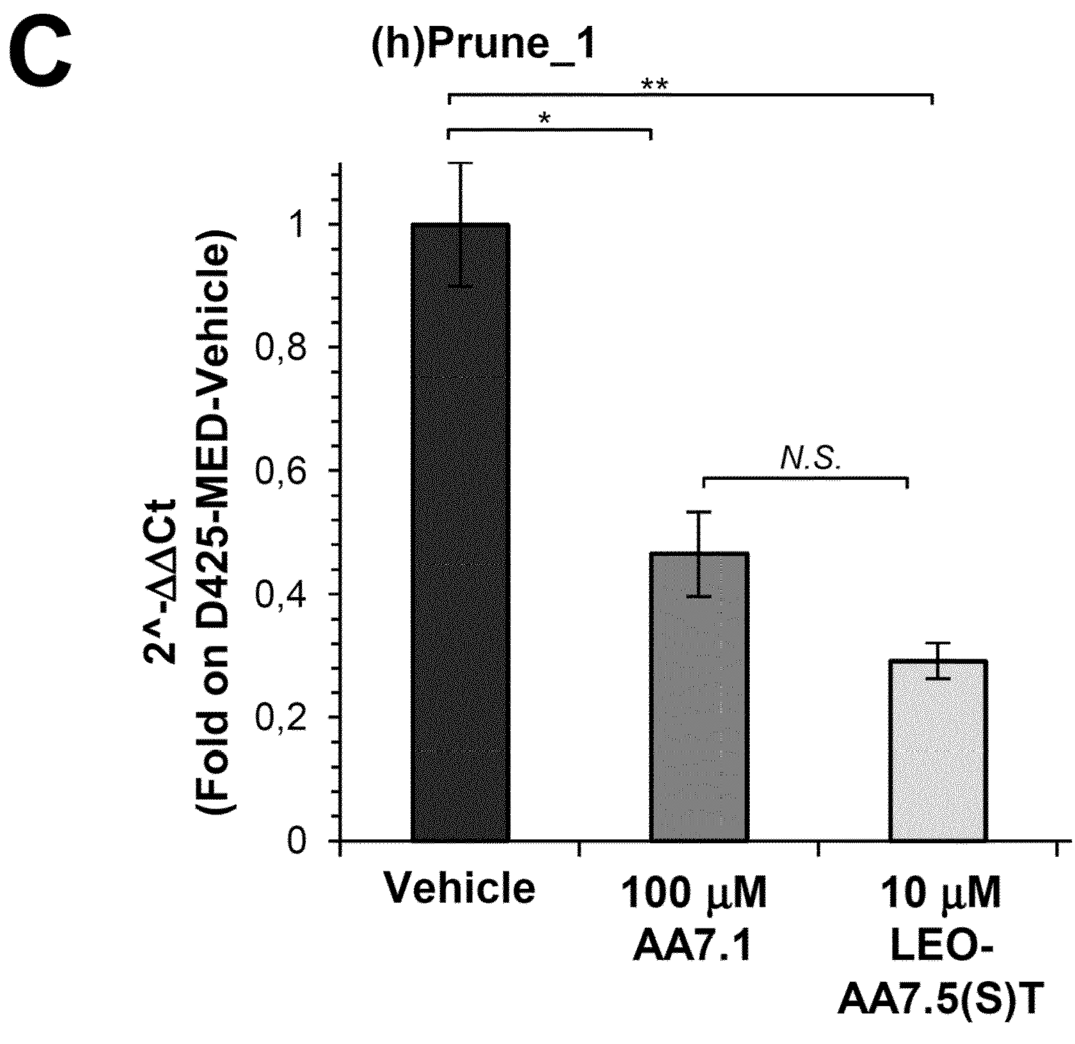


Figure 13 (continued)

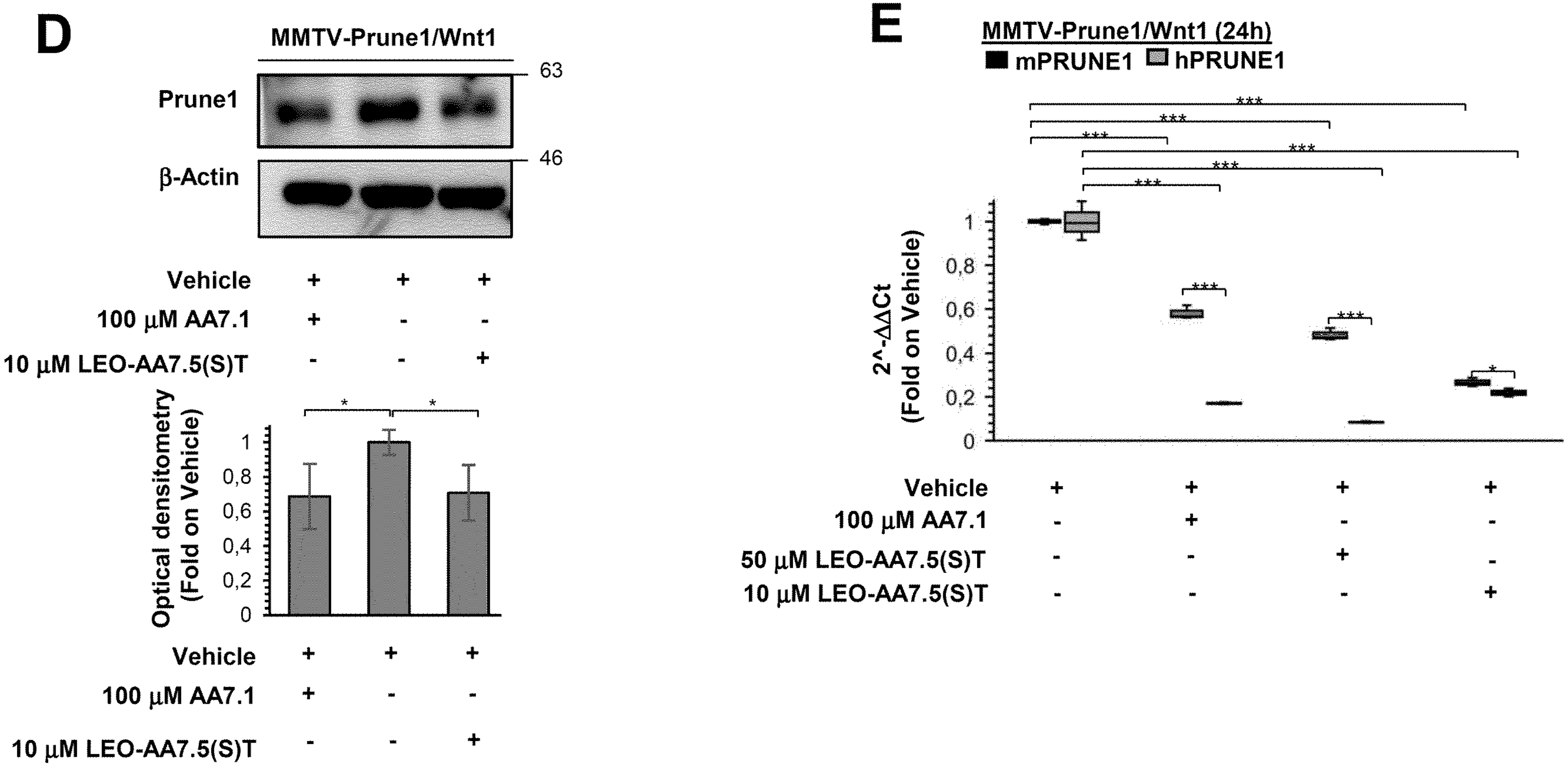


Figure 13 (continued)

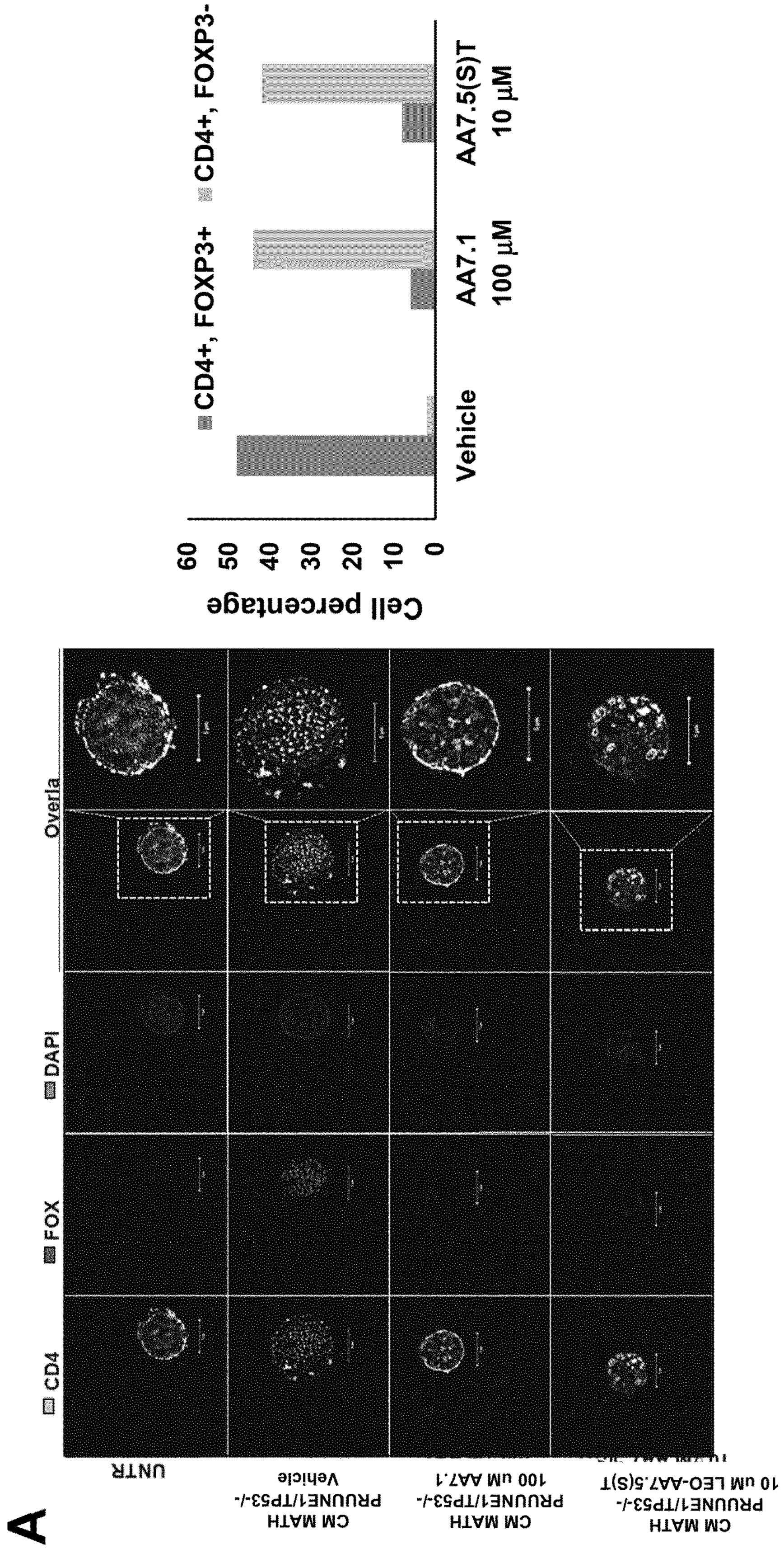

A
CD4
FOX
DAPI
Overla
UNTR
CM MATH PRUUNE1/TP53-/- Vehicle
CM MATH PRUUNE1/TP53-/- 100 uM AA7.1
CM MATH PRUUNE1/TP53-/- 10 uM LEO-AA7.5(S)T
CD4+, FOXP3+
CD4+, FOXP3-
Cell percentage
0
10
20
30
40
50
60
Vehicle
AA7.1 100 µM
AA7.5(S)T 10 µM


Figure 14

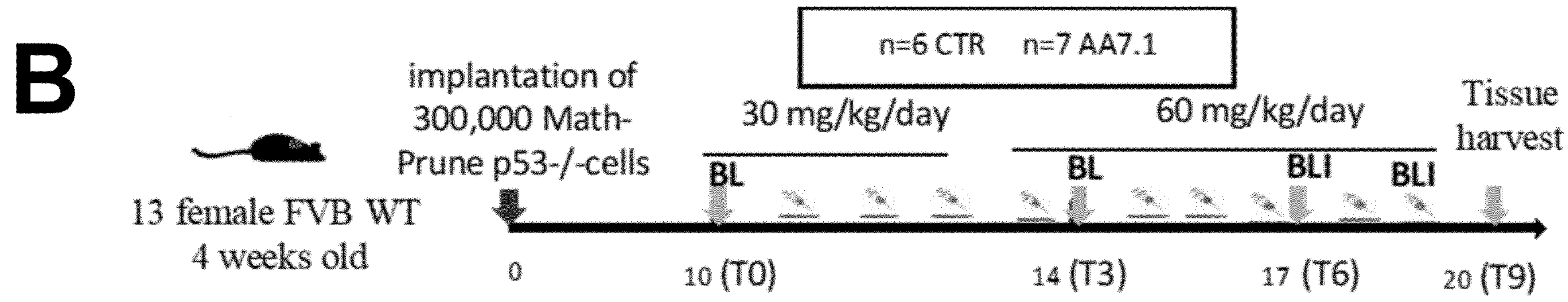


Figure 14 (continued)

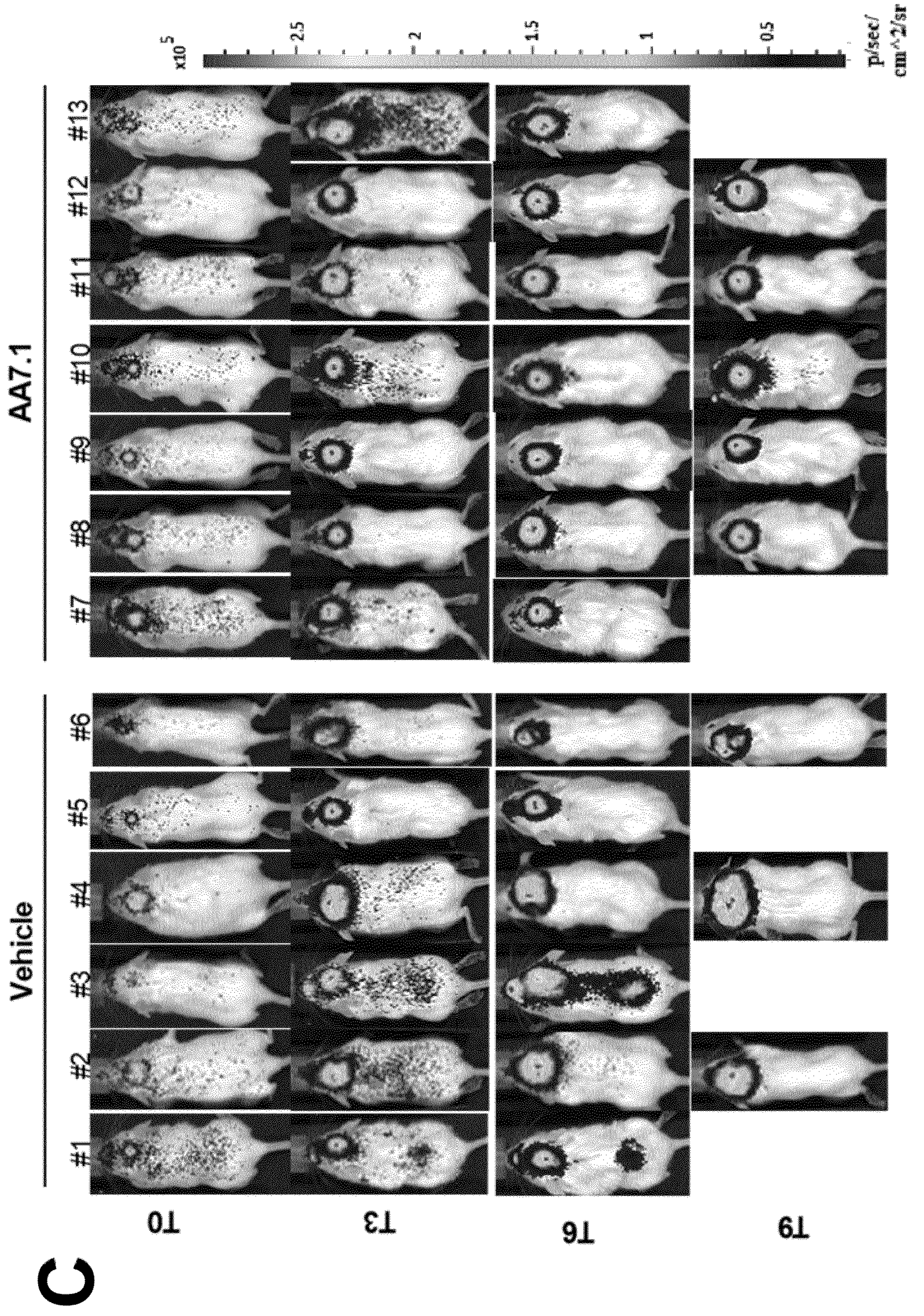

C
T0
T3
T6
T9
Vehicle
#1
#2
#3
#4
#5
#6
AA7.1
#7
#8
#9
#10
#11
#12
#13
x10^5
2.5
2
1.5
1
0.5
p/sec/
cm^2/sr

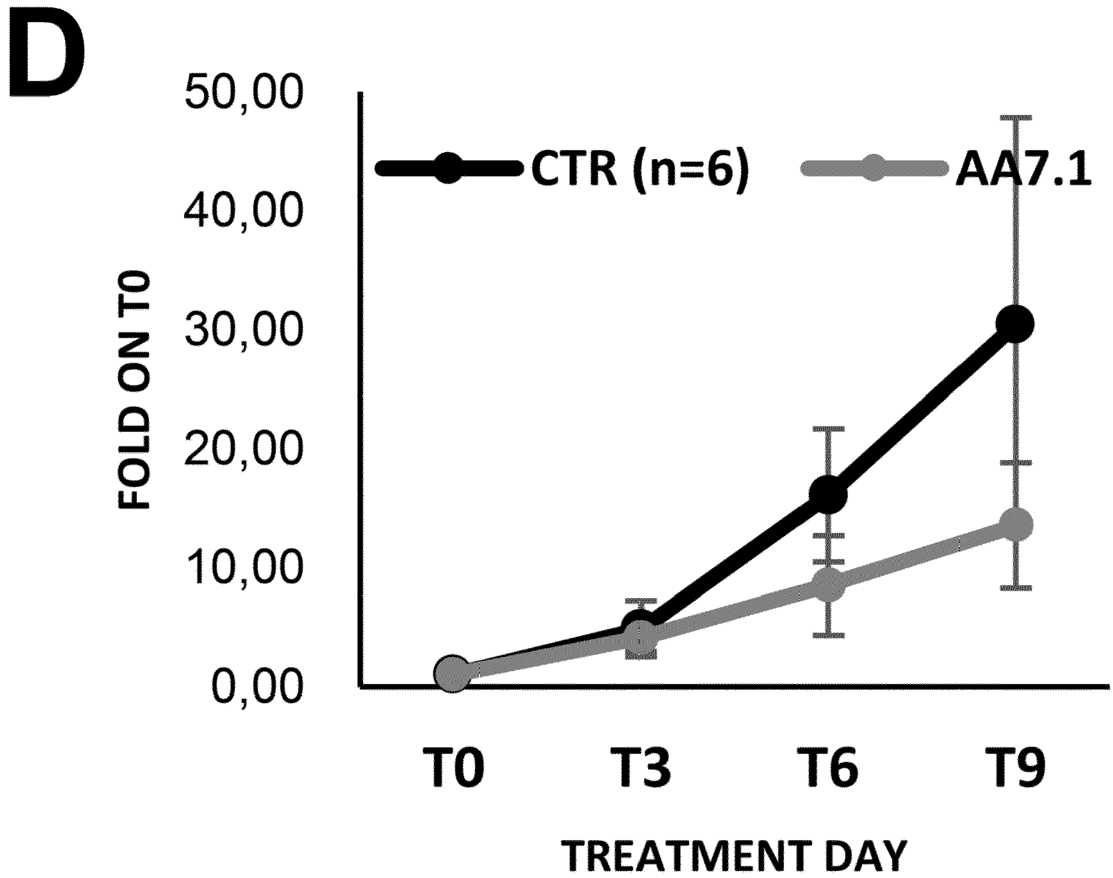


Figure 14 (continued)

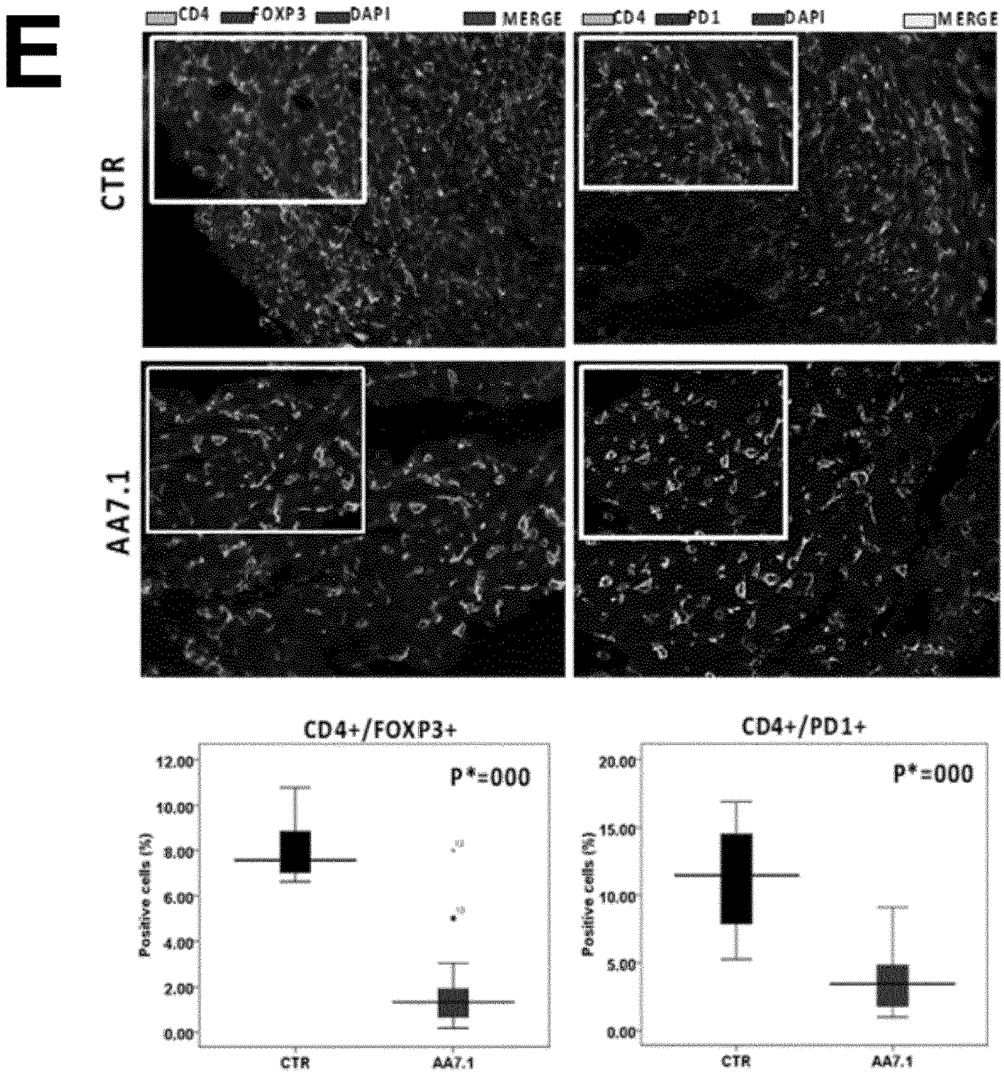


**Figure 14 (continued)**

Europäisches Patentamt
European Patent Office
Office européen des brevets

# EUROPEAN SEARCH REPORT

Application Number
EP 22 20 4452

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | FERRUCCI VERONICA ET AL: "Metastatic group 3 medulloblastoma is driven by PRUNE1 targeting NME1-TGF-[beta]-OTX2-SNAIL via PTEN inhibition", BRAIN, vol. 141, no. 5, 27 February 2018 (2018-02-27), pages 1300-1319, XP093031471, GB ISSN: 0006-8950, DOI: 10.1093/brain/awy039 * abstract * | 1-10 | INV. C07D401/04 A61P35/00 A61K31/53 |
| A,D | Ferrucci Veronica ET AL: "Supplementary Information Metastatic recurrence of Group 3 Medulloblastoma is driven by PRUNE-1 through targeting NME1-TGF-[beta]-OTX2-SNAIL signaling via PTEN inhibition", Brain, 27 February 2018 (2018-02-27), pages 1-31, XP093031505, Retrieved from the Internet: URL:https://academic.oup.com/brain/article/141/5/1300/4911167#supplementary-data [retrieved on 2023-03-14] * figures 5 - supplementary information * * Chemical Synthesis of AA7.1; page 20 * | 1-10 | |
| | | | TECHNICAL FIELDS SEARCHED (IPC) |
| | | | C07D A61P |

The present search report has been drawn up for all claims

| Place of search | Date of completion of the search | Examiner |
|---|---|---|
| Munich | 14 March 2023 | Fanni, Stefano |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014045310 A


### Non-patent literature cited in the description


- **BIBBÒ F ; SORICE C ; FERRUCCI V ; ZOLLO M.** Functional Genomics of PRUNE1 in Neurodevelopmental Disorders (NDDs) Tied to Medulloblastoma (MB) and Other Tumors. *Front Oncol.,* 22 October 2021, vol. 11, 758146
- **D'ANGELO A ; GARZIA L ; ANDRE A ; CAROTENUTO P ; AGLIO V ; GUARDIOLA O et al.** Prune cAMP Phosphodiesterase Binds Nm23-H1 and Promotes Cancer Metastasis. *Cancer Cell,* 2004, vol. 5 (2), 137-49
- **TAMMENKOSKI M ; KOIVULA K ; CUSANELLI E ; ZOLLO M ; STEEGBORN C ; BAYKOV AA et al.** Human Metastasis Regulator Protein H-Prune Is a Short-Chain Exopolyphosphatase. *Biochemistry,* 2008, vol. 47 (36), 9707-13
- **CAROTENUTO M ; DE ANTONELLIS P ; LIGUORI L ; BENVENUTO G ; MAGLIULO D ; ALONZI A et al.** H-Prune Through GSK-3beta Interaction Sustains Canonical WNT/beta-Catenin Signaling Enhancing Cancer Progression in NSCLC. *Oncotarget,* 2014, vol. 5 (14), 5736-49
- **DIANA D ; SMALDONE G ; DE ANTONELLIS P ; PIRONE L ; CAROTENUTO M ; ALONZI A et al.** Mapping Functional Interaction Sites of Human Prune C-Terminal Domain by NMR Spectroscopy in Human Cell Lysates. *Chemistry,* 2013, vol. 19 (37), 12217-20
- **ZOLLO M ; AHMED M ; FERRUCCI V ; SALPIETRO V ; ASADZADEH F ; CAROTENUTO M et al.** PRUNE Is Crucial for Normal Brain Development and Mutated in Microcephaly With Neurodevelopmental Impairment. *Brain,* 2017, vol. 140 (4), 940-52
- **FERRUCCI V ; DE ANTONELLIS P ; PENNINO FP ; ASADZADEH F ; VIRGILIO A ; MONTANARO D et al.** Metastatic Group 3 Medulloblastoma Is Driven by PRUNE1 Targeting NME1-TGF-Beta-0TX2-SNAIL via PTEN Inhibition. *Brain,* 2018, vol. 141 (5), 1300-19
- **CAROTENUTO, M. ; PEDONE, E. ; DIANA, D. et al.** Neuroblastoma tumorigenesis is regulated through the Nm23-H1/h-Prune C-terminal interaction. *Sci Rep,* 2013, vol. 3, 1351
- **SMALDONE G ; PIRONE L ; CAPOLUPO A ; VITAGLIANO L ; MONTI MC ; DI GAETANO S ; PEDONE E.** The essential player in adipogenesis GRP78 is a novel KCTD15 interactor. *Int J Biol Macromol.,* August 2018, vol. 115, 469-475
- **FERRUCCI V ; ASADZADEH F ; COLLINA F ; SICILIANO R ; BOCCIA A ; MARRONE L ; SPANO D ; CAROTENUTO M ; CHIAROLLA CM ; DE MARTINO D.** Prune-1 drives polarization of tumor-associated macrophages (TAMs) within the lung metastatic niche in triple-negative breast cancer. *iScience.,* 13 December 2020, vol. 24 (1), 101938
- **ASADZADEH F ; FERRUCCI V ; DE ANTONELLIS P ; ZOLLO M.** In vivo bioluminescence imaging using orthotopic xenografts towards patient's derived-xenograft Medulloblastoma models. *Q J Nucl Med Mol Imaging.,* March 2017, vol. 61 (1), 95-101
- **OUE N ; YOSHIDA K ; NOGUCHI T ; SENTANI K ; KIKUCHI A ; YASUI W.** Increased Expression of H-Prune Is Associated With Tumor Progression and Poor Survival in Gastric Cancer. *Cancer Sci,* 2007, vol. 98 (8), 1198-205
- **NOGUCHI T ; OUE N ; WADA S ; SENTANI K ; SAKAMOTO N ; KIKUCHI A et al.** H-Prune Is an Independent Prognostic Marker for Survival in Esophageal Squamous Cell Carcinoma. *Ann Surg Oncol,* 2009, vol. 16 (5), 1390-6
- **NAMBU J ; KOBAYASHI T ; HASHIMOTO M ; TASHIRO H ; SUGINO K ; SHIMAMOTO F et al.** H-Prune Affects Anaplastic Thyroid Cancer Invasion and Metastasis. *Oncol Rep,* 2016, vol. 35 (6), 3445-52
- **HASHIMOTO M ; KOBAYASHI T ; TASHIRO H ; ARIHIRO K ; KIKUCHI A ; OHDAN H. H.** Prune Is Associated With Poor Prognosis and Epithelial-Mesenchymal Transition in Patients With Colorectal Liver Metastases. *Int J Cancer,* 2016, vol. 139 (4), 812-23
- **ZOLLO M ; ANDRE A ; COSSU A ; SINI MC ; D ANGELO A ; MARINO N et al.** Overexpression of H-Prune in Breast Cancer Is Correlated With Advanced Disease Status. *Clin Cancer Res,* 2005, vol. 11 (1), 199-205

- **CAVALLI FMG ; REMKE M ; RAMPASEK L ; PEACOCK J ; SHIH DJH ; LUU B et al.** Intertumoral Heterogeneity Within Medulloblastoma Subgroups. *Cancer Cell,* 2017, vol. 31 (6), 737-754
- **GOH JY ; FENG M ; WANG W ; OGUZ G ; YATIM S ; LEE PL et al.** Chromosome 1q21.3 Amplification Is a Trackable Biomarker and Actionable Target for Breast Cancer Recurrence. *NatMed,* 2017, vol. 23 (11), 1319-30
- **SILVA GO ; HE X ; PARKER JS ; GATZA ML ; CAREY LA ; HOU JP et al.** Cross-Species DNA Copy Number Analyses Identifies Multiple 1q21-Q23 Subtype-Specific Driver Genes for Breast Cancer. *Breast Cancer Res Treat,* 2015, vol. 152 (2), 347-56
- **GALASSO A ; ZOLLO M.** The Nm23-H1-H-Prune Complex in Cellular Physiology: A 'Tip of the Iceberg' Protein Network Perspective. *Mol Cell Biochem,* 2009, vol. 329 (1-2), 149-59
- **GAJJAR AJ ; ROBINSON GW.** Medulloblastoma-Translating Discoveries From the Bench to the Bedside. *Nat Rev Clin Oncol,* 2014, vol. 11 (12), 714-22
- **HILL RM ; KUIJPER S ; LINDSEY JC ; PETRIE K ; SCHWALBE EC ; BARKER K et al.** Combined MYC and P53 Defects Emerge at Medulloblastoma Relapse and Define Rapidly Progressive, Therapeutically Targetable Disease. *Cancer Cell,* 2015
- **VIRGILIO A ; SPANO D ; ESPOSITO V ; DI DATO V ; CITARELLA G ; MARINO N et al.** Novel Pyrimidopyrimidine Derivatives for Inhibition of Cellular Proliferation and Motility Induced by H-Prune in Breast Cancer. *Eur J Med Chem,* 2012, vol. 57, 41-50
- **BELOW J ; M DAS J. VINCRISTINE.** StatPearls [Internet]. Treasure Island (FL). StatPearls Publishing, January 2022
- **LUMPKIN EA ; COLLISSON T ; PARAB P ; OMER-ABDALLA A ; HAEBERLE H ; CHEN P ; DOETZLHOFER A ; WHITE P ; GROVES A ; SEGIL N.** Math1-driven GFP expression in the developing nervous system of transgenic mice. *Gene Expr Patterns,* August 2003, vol. 3 (4), 389-95
- **TING WU‡§ ; XIULI ZHANG§ ; XIAOHUA HUANG§ ; YUQING YANG ; XIANXIN HUA.** Regulation of Cyclin B2 Expression and Cell Cycle G2/M Transition by Menin. *THEJOURNALOFBIOLOGICALCHEMISTRY,* vol. 285 (24), 18291-18300
- **NORTHCOTT PA ; DUBUC AM ; PFISTER S ; TAYLOR MD.** Molecular subgroups of medulloblastoma. *Expert Rev Neurother.,* July 2012, vol. 12 (7), 871-84
- **TAYLOR MD ; NORTHCOTT PA ; KORSHUNOV A ; REMKE M ; CHO YJ ; CLIFFORD SC ; EBERHART CG ; PARSONS DW ; RUTKOWSKI S ; GAJJAR A.** Molecular subgroups of medulloblastoma: the current consensus. *Acta Neuropathol.,* April 2012, vol. 123 (4), 465-72
- **FANGUSARO J ; CEFALO MG ; GARRÉ ML ; MARSHALL LV ; MASSIMINO M ; BENETTAIB B ; BISERNA N ; POON J ; QUAN J ; CONLIN E.** Phase 2 Study of Pomalidomide (CC-4047) Monotherapy for Children and Young Adults With Recurrent or Progressive Primary Brain Tumors. *Front Oncol.,* 08 June 2021, vol. 11, 660892
- **GARZIA L ; KIJIMA N ; MORRISSY AS ; DE ANTONELLIS P ; GUERREIRO-STUCKLIN A ; HOLGADO BL ; WU X ; WANG X ; PARSONS M ; ZAYNE K.** A Hematogenous Route for Medulloblastoma Leptomeningeal Metastases. *Cell,* 22 February 2018, vol. 172 (5), 1050-1062
- *Cell.,* 31 May 2018, vol. 173 (6), 1549
- **LEE, C. ; RUDNEVA, V.A. ; ERKEK, S. et al.** Lsdl as a therapeutic target in Gfil-activated medulloblastoma. *Nat Commun,* 2019, vol. 10, 332